(19) Europäisches Patentamt
European Patent Office
Office européen des brevets

(11) EP 4 703 364 A1

(12) **EUROPEAN PATENT APPLICATION**
published in accordance with Art. 153(4) EPC

(43) Date of publication:
**04.03.2026 Bulletin 2026/10**

(21) Application number: **24795535.4**

(22) Date of filing: **07.02.2024**

(51) International Patent Classification (IPC):
***C07D 513/14*** *(2006.01)* ***A61K 31/4353*** *(2006.01)*
***A61K 31/55*** *(2006.01)* ***A61P 35/00*** *(2006.01)*
***A61P 37/06*** *(2006.01)*

(86) International application number:
**PCT/CN2024/076636**

(87) International publication number:
**WO 2024/222147 (31.10.2024 Gazette 2024/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **23.04.2023 CN 202310438329**

(71) Applicant: **Horizon Omics Biotech Limited**
**Beijing 100084 (CN)**

(72) Inventor: **XU, Hong**
**Beijing 100084 (CN)**

(74) Representative: **Müller-Boré & Partner Patentanwälte PartG mbB**
**Friedenheimer Brücke 21**
**80639 München (DE)**

# (54) QUINOLONE ANALOG AND USE THEREOF

(57) Provided are a quinolone analog represented by formula (I) or a pharmaceutically acceptable salt, a stereoisomer, a racemate, a deuterated compound, or a solvent thereof, a pharmaceutical composition thereof, and the use thereof in the preparation of a drug for treating tumors, autoimmune diseases, or diseases caused by bacteria, fungi or viruses.

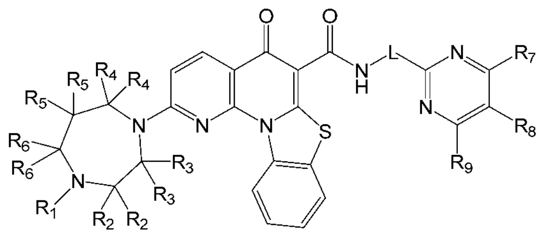

(I)

EP 4 703 364 A1

## Description

### REFERENCE TO RELATED APPLICATION

The present application claims the priority to and the benefits of Chinese Patent Application No. 202310438329.5, filed with the China National Intellectual Property Administration on April 23, 2023, the entire contents of which are incorporated herein by reference.

### TECHNICAL FIELD

The present application relates to a class of quinolone analogs and preparation methods and uses thereof, and belongs to the field of medicinal chemistry.

### BACKGROUND

Tetracycloquinolone compounds interact with quadruplex forming regions of nucleic acids and modulate the transcription of ribosomal RNAs. Specifically, tetracycloquinolone compounds can stabilize DNA G-quadruplexes (G4s) in cancer cells, thereby inducing synthetic lethality in these cells. Additionally, tetracycloquinolone compounds selectively inhibit rRNA synthesis mediated by Pol I within the nucleolus.

DNA typically exists in a double-stranded form but in certain guanine tandem repeat regions, guanines are connected via Hoogsteen hydrogen bonds, where four guanines (Gs) form a cyclic plane, and two or more layers of these quartets stack through $\pi$-$\pi$ interactions to form a special higher-order structure known as a quadruplex structure. Quadruplex DNAs are enriched at the ends of chromosomes and in the promoter regions of genes. Quadruplex DNAs located at the ends of chromosomes have the function to inhibit telomerase activity; therefore, stabilizing quadruplex DNAs at the ends of chromosomes can suppress cancer growth. Many oncogenes, such as c-Myc, KRAS, and c-Kit, have promoter regions rich in quadruplex DNA structures. Evidence suggests that stabilizing quadruplex DNAs in these regions can reduce oncogene expression, thereby inhibiting cancer progression.

The quadruplex DNA may be generally a transient structure that typically forms during DNA replication and transcription, when the double-stranded DNA unwinds. Cells also possess a variety of helicases that can resolve the higher-order structure of quadruplex DNAs. However, when the quadruplex DNA binds to a stabilizer drug, rendering it difficult for helicases to unwind the quadruplex structure, this stabilized structure inevitably hinders DNA replication and transcription, and may even cause DNA breaks. The homologous recombination (HR) DNA repair pathway mediated by BRCA1 and BRCA2, as well as the non-homologous end joining (NHEJ) repair pathway, are critical for repairing broken DNAs. These pathways may also be responsible for bypassing quadruplex structures during DNA replication and transcription, ensuring the smooth progression of these processes. Multiple literatures have reported that cancers with BRCA1/2 deficiencies exhibit high sensitivity to quadruplex DNA stabilizers. Additionally, cancer cells harbor more quadruplex DNA structures compared to normal cells. Consequently, quadruplex DNA stabilizers hold promise as potential clinical drugs for treating BRCA1/2-deficient tumors, HR-deficient tumors, and other tumors with DNA damage repair deficiencies.

CX-5461 was previously considered an RNA polymerase I inhibitor that suppresses rDNA translation. Some literatures have also suggested that CX-5461 has a topoisomerase inhibitory activity and stabilizes quadruplex DNA.

Although CX-5461 has demonstrated preliminary efficacy in HR-deficient solid tumors, it requires high dosages in clinical trials. In the NCT02719977 clinical trial for treating solid tumors in humans, the recommended dosage of CX-5461 was 475 mg/$m^2$, and adverse effects such as phototoxicity, neutropenia, acral erythema, and nausea were observed. Additionally, the current clinical administration of CX-5461 involves multiple intravenous infusions, which need to be performed in hospitals. This consumes substantial medical resources, and is less convenient and cost-effective compared to oral administration. Furthermore, intravenous infusion introduces risks such as intravenous infusion-related infections, phlebitis, and thrombosis. Considering the underlying health conditions of cancer patients receiving CX-5461 treatment, as well as the outbreaks of epidemic infectious diseases (e.g., COVID-19, SARS) in recent years, it is highly necessary to develop oral drugs of CX-5461 that do not occupy valuable medical resources.

Currently, there is an urgent clinical need to enhance the tumor killing activity of compounds, improve their metabolic stability, reduce their toxic side effects, and increase their bioavailability, thereby developing the compounds into oral formulations that are more convenient for patients to use.

### SUMMARY OF INVENTION

In one aspect, the present application provides a quinolone analog represented by Formula (I), or a pharmaceutically acceptable salt, stereoisomer, racemate, deuterated compound, or solvate thereof:

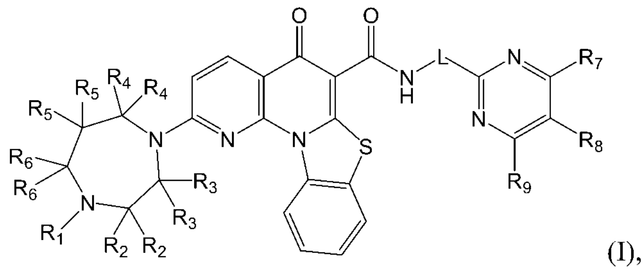

(I),

wherein:

- $R_1$, $R_2$, $R_3$, $R_4$, $R_5$, and $R_6$ are selected from H, deuterium, halogen, C1-C6 alkyl, or C1-C4 alkyl substituted with one or more deuterium atoms;
- $R_7$, $R_8$, and $R_9$ are selected from H, deuterium, or C1-C4 alkyl substituted with one or more deuterium atoms; alternatively, $R_7$, $R_8$, and $R_9$ may simultaneously or separately be D, halogen, azido, -CN, $-CF_3$, $-CONR_{10}R_{11}$, $-NR_{10}R_{11}$, $-SR_{11}$, $-OR_{11}$, $-R_{12}$, -W, -M-W, -V, or $-L-N(R_{13})-V$;
- L is $-(CH_2)_n-$, where n = 0-6;
- $R_{10}$ is selected from H or C1-C6 alkyl, optionally substituted with one or more halogen atoms or =O;
- $R_{11}$ is selected from H, C1-C10 alkyl, C1-C10 heteroalkyl, C2-C10 alkenyl, or C2-C10 heteroalkenyl, each of which may optionally be substituted with one or more halogen atoms, =O, or optionally substituted 3- to 7-membered carbocycle or heterocycle;
- $R_{12}$ is selected from optionally substituted C1-C10 alkyl, C2-C10 alkenyl, C5-C10 aryl, or C6-C12 arylalkyl or heterocycle, each of which may optionally be substituted with one or more halogen atoms, =O, or optionally substituted 3- to 6-membered carbocycle or heterocycle;
- $R_{13}$ is selected from H, C1-C10 alkyl, C1-C10 heteroalkyl, C2-C10 alkenyl, or C2-C10 heteroalkenyl, each of which may optionally be substituted with one or more halogen atoms, =O, or optionally substituted 3- to 7-membered carbocycle or heterocycle;
- M is a linker selected from C1-C10 alkylene, C1-C10 heteroalkylene, C2-C10 alkenylene, or C2-C10 heteroalkenylene, each of which may optionally be substituted with one or more substituents selected from halogen, =O, or C1-C6 alkyl;
- W is selected from optionally substituted 4- to 7-membered nitrogen-containing heterocycle, which optionally contains a heteroatom selected from N, O, or S as a ring-forming member; and
- V is selected from optionally substituted 3- to 4-membered carbocycle, or C1-C6 alkyl substituted with 1 to 4 fluorine atoms.

In some embodiments, in Formula (I):

- $R_1$, $R_2$, $R_3$, $R_4$, $R_5$, and $R_6$ are selected from H, deuterium, halogen, C1-C6 alkyl, or C1-C4 alkyl substituted with one or more deuterium atoms;
- $R_7$, $R_8$, and $R_9$ are selected from H, deuterium, or C1-C4 alkyl substituted with one or more deuterium atoms; alternatively, $R_7$, $R_8$, and $R_9$ may simultaneously or separately be D, halogen, azido, -CN, $-CF_3$, $-CONR_{10}R_{11}$, $-NR_{10}R_{11}$, $-SR_{11}$, $-OR_{11}$, $-R_{12}$, -W, -M-W, or -V;
- L is $-(CH_2)_n-$, where n = 0-6;
- $R_{10}$ is selected from H or C1-C6 alkyl, optionally substituted with one or more halogen atoms or =O;
- $R_{11}$ is selected from H, C1-C10 alkyl, C1-C10 heteroalkyl, C2-C10 alkenyl, or C2-C10 heteroalkenyl, each of which may optionally be substituted with one or more halogen atoms, =O, or optionally substituted 3- to 7-membered carbocycle or heterocycle;
- $R_{12}$ is selected from optionally substituted C1-C10 alkyl, C2-C10 alkenyl, C5-C10 aryl, or C6-C12 arylalkyl or heterocycle, each of which may optionally be substituted with one or more halogen atoms, =O, or optionally substituted 3- to 6-membered carbocycle or heterocycle;
- M is a linker selected from C1-C10 alkylene, C1-C10 heteroalkylene, C2-C10 alkenylene, or C2-C10 heteroalkenylene, each of which may optionally be substituted with one or more substituents selected from halogen, =O, or C1-C6 alkyl;
- W is selected from optionally substituted 4- to 7-membered nitrogen-containing heterocycle, which optionally contains a heteroatom selected from N, O, or S as a ring-forming member;
- V is selected from optionally substituted 3- to 4-membered carbocycle, or C1-C6 alkyl substituted with 1 to 4 fluorine atoms.

In some embodiments, in Formula (I):

$R_1$, $R_2$, $R_3$, $R_4$, $R_5$, and $R_6$ are each independently selected from H, deuterium, halogen, C1-C6 alkyl, or C1-C4 alkyl substituted with one or more deuterium atoms;
$R_7$, $R_8$, and $R_9$ are each independently selected from H, deuterium, halogen, azido, -CN, C1-C6 alkyl, C1-C6 haloalkyl, -O(C1-C6 alkyl), -S(C1-C6 alkyl), $-CONR_{10}R_{11}$, $-NR_{10}R_{11}$, 3- to 4-membered cycloalkyl, 4- to 7-membered nitrogen-containing heterocycle, or $-(CH_2)_m$-(4- to 7-membered nitrogen-containing heterocycle), wherein $R_{10}$ and $R_{11}$ are each independently selected from H or C1-C6 alkyl; the 4- to 7-membered nitrogen-containing heterocycle contains 1, 2, 3, or 4 nitrogen atoms; and m = 1-3;
L is $-(CH_2)_n-$, where n = 1-6;
$R_{10}$ is selected from H or C1-C6 alkyl, wherein the C1-C6 alkyl is optionally substituted with one or more halogen atoms;
$R_{11}$ is selected from H or C1-C6 alkyl, wherein the C1-C6 alkyl is optionally substituted with one or more halogen atoms.

In some embodiments, $R_1$, $R_2$, $R_3$, $R_4$, $R_5$, and $R_6$ are each independently selected from H, deuterium, C1-C6 alkyl, or C1-C4 alkyl substituted with one or more deuterium atoms.
In some embodiments, $R_1$ is C1-C6 alkyl, or C1-C4 alkyl substituted with one or more deuterium atoms, and $R_2$ to $R_6$ are each independently H or deuterium.
In some embodiments, $R_1$ is $-CH_3$ or $-CD_3$, and $R_2$ to $R_6$ are all H or all deuterium.
In some embodiments, $R_1$ is $-CH_3$ or $-CD_3$, and $R_2$ to $R_6$ are all H; or, $R_4$ is H and $R_2$, $R_3$, $R_5$, and $R_6$ are all deuterium.
In some embodiments, $R_1$ is $-CH_3$, and $R_2$ to $R_6$ are all H. In some embodiments, $R_1$ is $-CD_3$, and $R_2$ to $R_6$ are all H.
In some embodiments, $R_7$, $R_8$, and $R_9$ are each independently selected from H, deuterium, halogen, azido, -CN, C1-C6 alkyl, C1-C6 haloalkyl, -O(C1-C6 alkyl), -S(C1-C6 alkyl), $-CONR_{10}R_{11}$, $-NR_{10}R_{11}$, 3- to 4-membered cycloalkyl, 5- to 6-membered nitrogen-containing heterocycle, or $-CH_2$-(5- to 6-membered nitrogen-containing heterocycle), wherein $R_{10}$ and $R_{11}$ are each independently selected from H or C1-6 alkyl, and the 5- to 6-membered nitrogen-containing heterocycle contains 1, 2, 3, or 4 nitrogen atoms.
In some embodiments, one of $R_7$, $R_8$, and $R_9$ is selected from H, halogen, azido, -CN, C1-C6 alkyl, C1-C6 haloalkyl, -O(C1-C6 alkyl), -S(C1-C6 alkyl), $-CONR_{10}R_{11}$, $-NR_{10}R_{11}$, 3- to 4-membered cycloalkyl, 5- to 6-membered nitrogen-containing heterocycle, or $-CH_2$-(5- to 6-membered nitrogen-containing heterocycle), wherein $R_{10}$ and $R_{11}$ are each independently selected from H or C1-6 alkyl, and the 5- to 6-membered nitrogen-containing heterocycle contains 1, 2, 3, or 4 nitrogen atoms; and the other two of $R_7$, $R_8$, and $R_9$ are H. In some embodiments, $R_7$, $R_8$, and $R_9$ are all deuterium.
In some embodiments, $R_8$ is selected from H, halogen, azido, -CN, C1-C6 alkyl, C1-C6 haloalkyl, -O(C1-C6 alkyl), -S(C1-C6 alkyl), $-CONR_{10}R_{11}$, $-NR_{10}R_{11}$, 3- to 4-membered cycloalkyl, 5- to 6-membered nitrogen-containing heterocycle, or $-CH_2$-(5- to 6-membered nitrogen-containing heterocycle), wherein $R_{10}$ and $R_{11}$ are each independently selected from H or C1-6 alkyl, and the 5- to 6-membered nitrogen-containing heterocycle contains 1, 2, 3, or 4 nitrogen atoms; and $R_7$ and $R_9$ are H.
In some embodiments, $R_7$ is selected from H, halogen, azido, -CN, C1-C6 alkyl, C1-C6 haloalkyl, -O(C1-C6 alkyl), -S(C1-C6 alkyl), $-CONR_{10}R_{11}$, $-NR_{10}R_{11}$, 3- to 4-membered cycloalkyl, 5- to 6-membered nitrogen-containing heterocycle, or $-CH_2$-(5- to 6-membered nitrogen-containing heterocycle), wherein $R_{10}$ and $R_{11}$ are each independently selected from H or C1-6 alkyl, and the 5- to 6-membered nitrogen-containing heterocycle contains 1, 2, 3, or 4 nitrogen atoms; and $R_8$ and $R_9$ are H.
In some embodiments, $R_8$ is H, F, Cl, $-CF_3$, -CN, $-N_3$, $-CONHCH_3$, $-CON(CH_3)_2$, $-OCH_3$, $-SCH_3$, $-NHCH_3$, $-N(CH_3)_2$, $-C_2H_5$, $-CH_2$-1-pyrrolyl, $-CH_2$-4-pyridyl, 1-pyrrolyl, 2H-tetrazol-5-yl, cyclopropyl, or $-CH_2CF_3$; and $R_7$ and $R_9$ are H.
In some embodiments, $R_7$ is H, F, Cl, $-CF_3$, -CN, $-N_3$, $-CONHCH_3$, $-CON(CH_3)_2$, $-OCH_3$, $-SCH_3$, $-NHCH_3$, $-N(CH_3)_2$, $-C_2H_5$, $-CH_2$-1-pyrrolyl, $-CH_2$-4-pyridyl, 1-pyrrolyl, 2H-tetrazol-5-yl, cyclopropyl, or $-CH_2CF_3$; and $R_8$ and $R_9$ are H. In some embodiments, $R_7$ is H, F, Cl, $-CF_3$, -CN, $-N_3$, $-CONHCH_3$, $-CON(CH_3)_2$, $-OCH_3$, $-SCH_3$, $-NHCH_3$, $-N(CH_3)_2$, $-C_2H_5$, cyclopropyl, or $-CH_2CF_3$; and $R_8$ and $R_9$ are H. In some embodiments, $R_7$ is H, F, -CN, $-CONHCH_3$, $-OCH_3$, $-C_2H_5$, cyclopropyl, or $-CH_2CF_3$; and $R_8$ and $R_9$ are H.
In some embodiments, L is $-(CH_2)_n-$, where n = 1-3. In some embodiments, L is $-CH_2-$.
In some embodiments, $R_{10}$ and $R_{11}$ are each independently H or $-CH_3$.
In some embodiments, the compound of Formula (I) is selected from the following compounds:

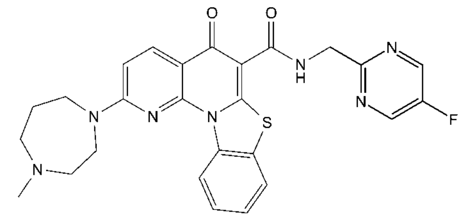
H-1,

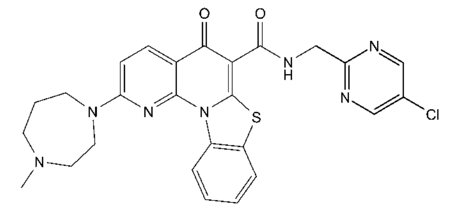
H-2,

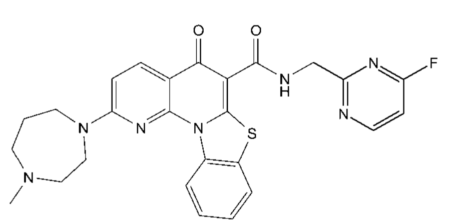
H-3,

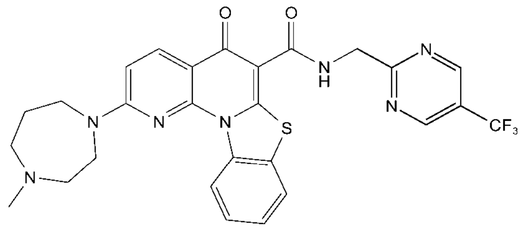
H-4,

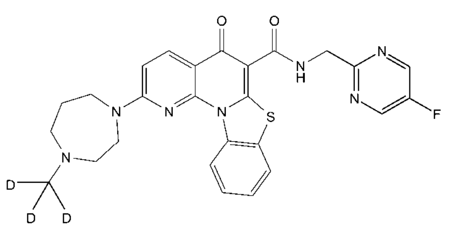
H-5,

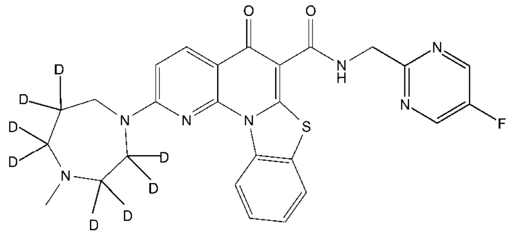
H-6,

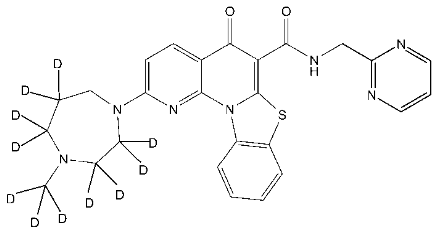
H-7,

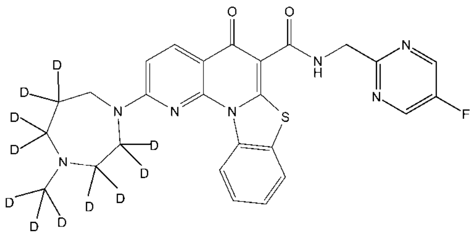
H-8,

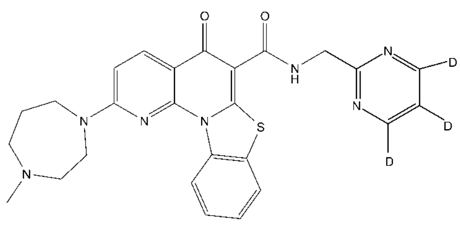
H-9,

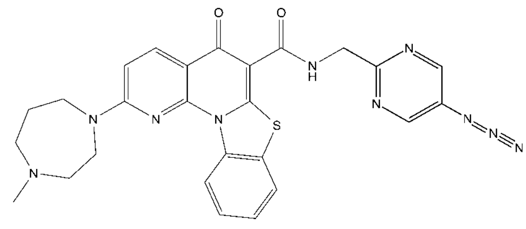
H-10,

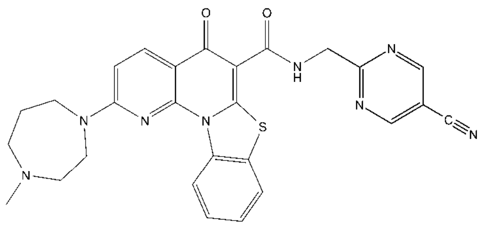
H-11,

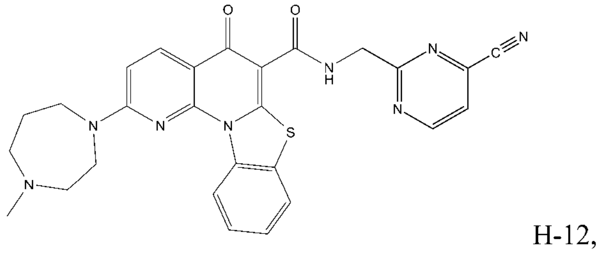
H-12,

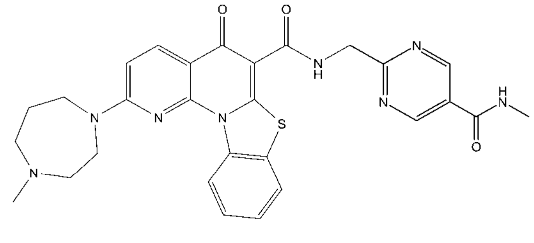
H-13,

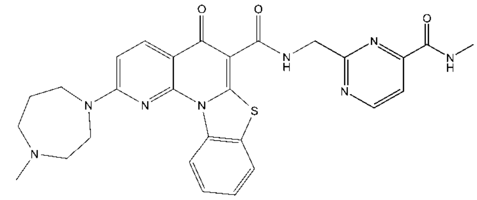
H-14,

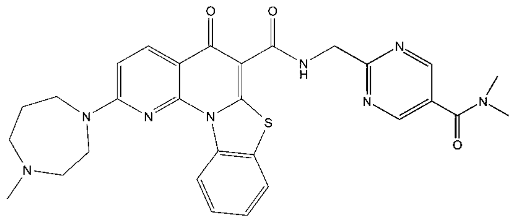
H-15,

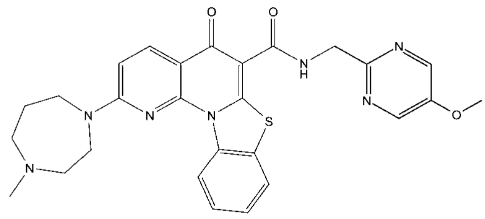
H-16,

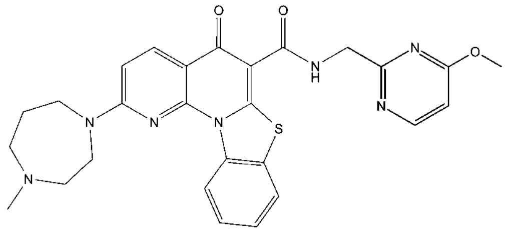
H-17,

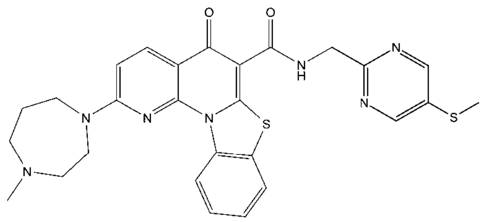
H-18,

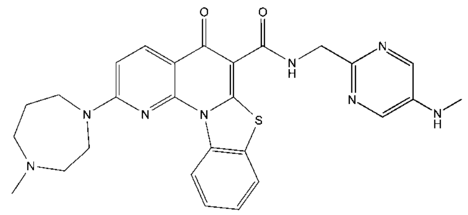
H-19,

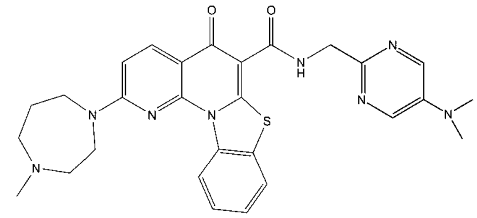
H-20,

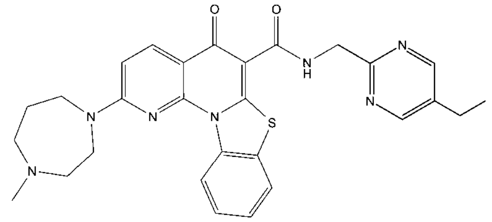
H-21,

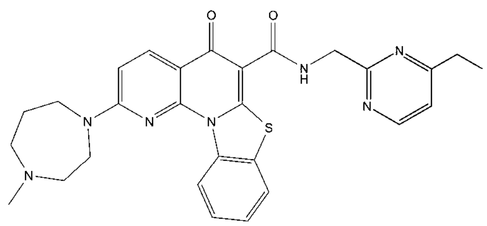
H-22,

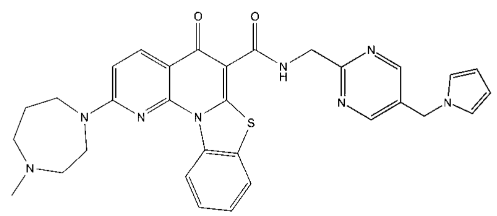
H-23,

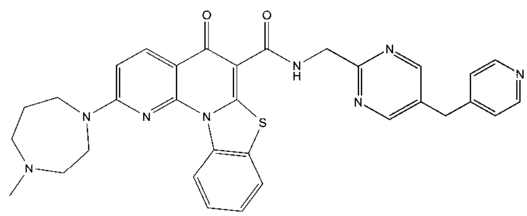
H-24,

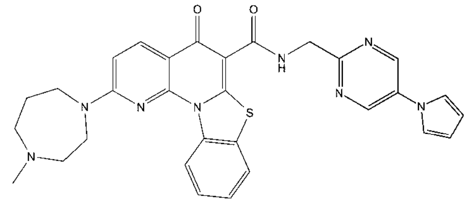

H-25,

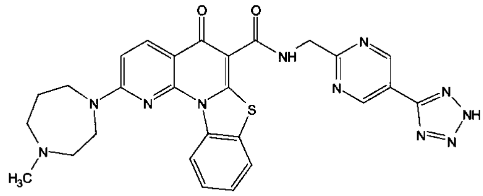

H-26,

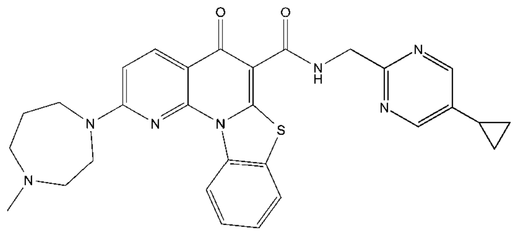

H-27,

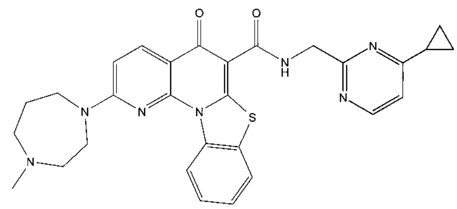

H-28,

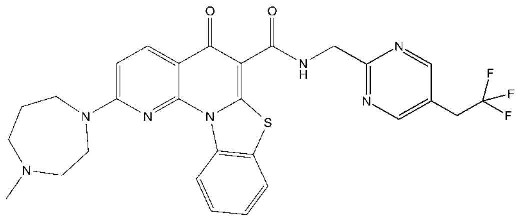

H-29,

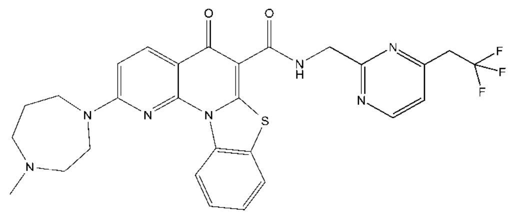

H-30,

or

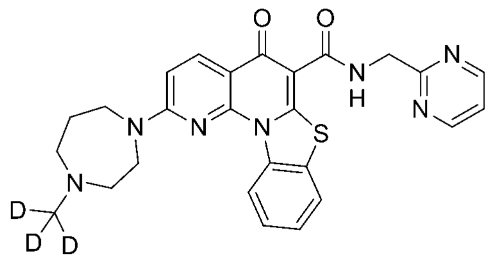

H-31.

Further, the compound also comprises a pharmaceutically acceptable salt, stereoisomer, racemate, deuterated compound, or solvate thereof.

The "pharmaceutically acceptable salt" of the compound represented by Formula (I) in the present application usually exists in the form of a free acid. The acid addition salt of a free amino compound of the present application can be prepared by methods known in the art. Suitable acids include, but are not limited to, phosphoric acid, hydrochloric acid, hydrobromic acid, hydrofluoric acid, hydroiodic acid, sulfuric acid, nitric acid, maleic acid, fumaric acid, benzoic acid, ascorbic acid, succinic acid, methanesulfonic acid, formic acid, acetic acid, trifluoroacetic acid, oxalic acid, propionic acid, tartaric acid, salicylic acid, citric acid, gluconic acid, lactic acid, mandelic acid, phenylacetic acid, aspartic acid, stearic acid, palmitic acid, glycolic acid, glutamic acid, p-toluenesulfonic acid, and benzenesulfonic acid. In addition, groups containing a basic nitrogen atom may be quaternized with the following reagents: lower alkyl halides, e.g., chlorides, bromides, and iodides of methyl, ethyl, and butyl; dialkyl sulfates, e.g., dimethyl sulfate, diethyl sulfate, dibutyl sulfate, and dipentyl sulfate; long-chain halides, e.g., chlorides, bromides, and iodides of decyl, dodecyl, tetradecyl, and octadecyl; arylalkyl halides, e.g., benzyl bromide and phenylethyl bromide, and others. The resulting products are water-soluble, oil-soluble, or dispersible. Therefore, the "pharmaceutically acceptable salt" shall include all acceptable salt forms.

The "stereoisomer" and "racemate" of the compound represented by Formula (I) in the present application generally refer to the compound of Formula (I) having a chiral center and existing in the forms of a racemate, a racemic mixture, and individual enantiomers or diastereomers. All isomeric forms, including their mixtures, are included in the present application.

In addition, some compounds represented by Formula (I) may also form solvates with water or other organic solvents, such as hydrates and tert-butanol solvates. Such solvates are also similarly included within the scope of the

present application.

Those skilled in the art will understand that any compound may contain non-natural proportions of atomic isotopes at one or more atoms constituting the compound. In the context of the present application, the term "deuterated" refers to the presence of deuterium atoms at relevant sites of the compound in proportions exceeding the natural proportion (i.e., exceeding the natural abundance of deuterium). Therefore, any compound represented by Formula (I) that contains deuterium atoms at relevant positions in proportions higher than the natural abundance of deuterium falls within the protection scope of the present application. For example, it should be understood that compounds represented by Formula (I) with corresponding deuteration ratios or deuterium contents, obtained by introducing deuterium atoms using commercially available deuteration reagents through chemical synthesis methods that are the same as or similar to those shown in the examples of the present application, fall within the protection scope of the present application. The chemical synthesis methods and deuteration reagents herein are not limited to those exemplified in the examples, but shall be understood to encompass all synthesis methods or routes available in the field for obtaining the compounds of the present application, and all deuteration reagents that can be used in conjunction with the aforementioned synthesis methods or routes to introduce deuterium atoms into the target molecules.

In another aspect, the present application provides a pharmaceutical composition comprising the compound or the pharmaceutically acceptable salt, stereoisomer, racemate, deuterated compound, or solvate thereof described in the present application, as an active ingredient.

In another aspect, the present application provides a pharmaceutical composition comprising a therapeutically effective amount of the compound or the pharmaceutically acceptable salt, stereoisomer, racemate, deuterated compound, or solvate thereof described in the present application, and one or more pharmaceutically acceptable carriers or excipients.

In another aspect, the present application provides the use of the compound or the pharmaceutically acceptable salt, stereoisomer, racemate, deuterated compound, or solvate thereof described in the present application, in drugs.

In another aspect, the present application provides the use of the compound or the pharmaceutically acceptable salt, stereoisomer, racemate, deuterated compound, or solvate thereof described in the present application or the pharmaceutical composition described in the present application in the manufacture of a medicament for the treatment of tumors, autoimmune diseases, or diseases caused by bacteria, fungi, or viruses.

In another aspect, the present application provides the compound or the pharmaceutically acceptable salt, stereoisomer, racemate, deuterated compound, or solvate thereof described in the present application or the pharmaceutical composition described in the present application for use in the treatment of tumors, autoimmune diseases, or diseases caused by bacteria, fungi, or viruses.

In a further aspect, the present application provides a method for treating tumors, autoimmune diseases, or diseases caused by bacteria, fungi, or viruses, the method comprising administering to a patient a therapeutically effective amount of the compound or the pharmaceutically acceptable salt, stereoisomer, racemate, deuterated compound, or solvate thereof described in the present application or the pharmaceutical composition described in the present application.

In a furthermore aspect, the present application provides the use of the compound or the pharmaceutically acceptable salt, stereoisomer, racemate, deuterated compound, or solvate thereof described in the present application or the pharmaceutical composition described in the present application in the treatment of tumors, autoimmune diseases, or diseases caused by bacteria, fungi, or viruses.

Method of Use

The present application provides the use of a quinolone analog represented by Formula (I) or a pharmaceutically acceptable salt, stereoisomer, racemate, deuterated compound, or solvate thereof in the manufacture of a medicament.

The present application provides uses of the aforementioned compound or the pharmaceutically acceptable salt thereof in the manufacture of a medicament for treating cancers with BRCA-1 or BRCA-2 mutations (homozygous or heterozygous mutations), homologous recombination repair-deficient cancers, non-homologous end joining (NHEJ) repair-deficient cancers, or other DNA damage repair-deficient cancers; and for treating cancers with overexpression of c-Myc, N-Myc, or L-Myc. Use alone and in combination therapy is included. Combination therapy may be performed by simultaneous or sequential administration.

The present application provides the use of the aforementioned compound or the pharmaceutically acceptable salt thereof in the manufacture of a medicament for treating diseases that are ameliorated or rendered resistant by the use of a PARP inhibitor, or in combination with a PARP inhibitor drug. The PARP inhibitor drug used in combination is selected from Olaparib, Niraparib, Rucaparib, Talazoparib, Fluzoparib, Pamiparib, or other PARP inhibitors.

The present application provides the use of the aforementioned compound or the pharmaceutically acceptable salt thereof in combination with a topoisomerase I and topoisomerase II inhibitor, an ATM inhibitor, an ATR inhibitor, or a DNA-pK inhibitor.

Further, the cancer is breast cancer, ovarian cancer, endometrial cancer, cervical cancer, oral cancer, pancreatic cancer, prostate cancer, brain cancer, lung cancer, liver cancer /hepatocellular carcinoma (HCC), leukemia, lymphoma, myeloma, multiple myeloma, skin cancer, peritoneal cancer, colorectal cancer, glioblastoma, melanoma, osteosarcoma, cervical cancer, Ewing's sarcoma, lymph node cancer, gastrointestinal malignancies, head and neck cancer, kidney cancer, cardiac cancer, or other cancers.

The present application provides the use of the aforementioned compound or the pharmaceutically acceptable salt thereof, either alone or in combination therapy, for treating autoimmune deficiency diseases such as multiple sclerosis. The combination therapy can be performed by simultaneous or sequential administration.

The present application provides the use of the aforementioned compound or the pharmaceutically acceptable salt thereof in the manufacture of a medicament for treating diseases caused by bacteria, fungi, or viruses. Viral infections include, but are not limited to, hepatitis B virus, hepatitis C virus, rhinovirus, varicella-zoster virus, herpes simplex virus, cytomegalovirus, poxvirus, encephalitis virus, hantavirus, arbovirus, human papillomavirus (HPV), Sironi virus, human immunodeficiency virus (HIV), influenza virus, Epstein-Barr (EB) virus, respiratory syncytial virus, coronaviruses (SARS-CoV, SARS-CoV-2, MERS-CoV), and so on. Use alone and in combination therapy are included. The combination therapy can be performed by simultaneous or sequential administration.

Formulations

The formulations of the present application are prepared by using the compound represented by Formula (I) or the pharmaceutically acceptable salt, stereoisomer, racemate, deuterated compound, or solvate thereof as the active ingredient, together with pharmaceutically acceptable excipients or auxiliary ingredients. The pharmaceutically acceptable auxiliary ingredients have certain physiological activities, but their addition does not alter the dominant role of the pharmaceutical composition in disease treatment and only exerts auxiliary effects. These auxiliary effects are merely the utilization of the known activities of the ingredients and are conventional adjuvant therapeutic methods in the medical field. The combined use of the auxiliary ingredients with the pharmaceutical composition of the present application shall still fall within the protection scope of the present application.

The term "pharmaceutically acceptable carrier" refers to one or more compatible solid or liquid fillers or gel substances that are suitable for human use, and must have sufficient purity and sufficiently low toxicity. Examples of the pharmaceutically acceptable carrier include, but are not limited to, cellulose and its derivatives (e.g., sodium carboxymethyl cellulose, sodium ethyl cellulose, cellulose acetate, etc.), gelatin, talc, solid lubricants (e.g., stearic acid, magnesium stearate), calcium sulfate, vegetable oils (e.g., soybean oil, sesame oil, peanut oil, olive oil, etc.), polyols (e.g., propylene glycol, glycerol, mannitol, sorbitol, etc.), emulsifiers, wetting agents, colorants, flavoring agents, stabilizers, antioxidants, preservatives, pyrogen-free water, and any other types of non-toxic solid, semi-solid, and liquid fillers, diluents, encapsulating materials, and formulation adjuvants.

Further, the present application provides a pharmaceutical composition comprising a therapeutically effective amount of the quinolone compound or the pharmaceutically acceptable salt, stereoisomer, racemate, deuterated compound, or solvate thereof as an active ingredient, and a pharmaceutically acceptable carrier.

Pharmaceutical Composition

The pharmaceutical composition of the present application may be in a solid or liquid form. On one hand, the carrier is in the form of microparticles, so that the composition is, for example, in the form of tablets or powders. The carrier may be a liquid, and the composition is, for example, an oral syrup, an injectable liquid, or an aerosol suitable for administration by inhalation, etc. When intended for oral administration, the pharmaceutical composition is preferably in a solid or liquid form. The semi-solid, semi-liquid, suspension, and gel forms are included herein as the solid or liquid forms. For oral solid compositions, the pharmaceutical composition may be formulated into powders, granules, compressed tablets, pills, capsules, chewable tablets, powder tablets, and the like. Such solid compositions usually contain one or more inert diluents or edible carriers. In addition, one or more of the following substances may also be present: binders, e.g., carboxymethyl cellulose, ethyl cellulose, microcrystalline cellulose, xanthan gum/tragacanth, or gelatin; excipients, e.g., starch, lactose, or dextrin; disintegrants, e.g., alginic acid, sodium alginate, corn starch, etc.; lubricants, e.g., magnesium stearate or hydrogenated vegetable oil; glidants, e.g., colloidal silicon dioxide; sweeteners, e.g., sucrose or saccharin; flavoring agents, e.g., peppermint, methyl salicylate, or sweet orange flavor; and colorants.

The pharmaceutical composition may be administered parenterally, intracisternally, vaginally, intraperitoneally, topically (e.g., powders, ointments, drops, and transdermal patches), rectally, or buccally. As used herein, the term "parenteral" refers to an administration mode that includes intravenous, intramuscular, intraperitoneal, intrasternal, subcutaneous, and intra-articular injections and infusions. The pharmaceutical composition for parenteral injection includes pharmaceutically acceptable sterile aqueous or non-aqueous solutions, dispersions, suspensions, and emulsions, as well as sterile powders for reconstitution into sterile injection solutions or dispersions immediately before use.

Suitable aqueous and non-aqueous carriers, diluents, solvents, or excipients include water, ethanol, polyols (e.g., glycerol, propylene glycol, polyethylene glycol, etc.), carboxymethyl cellulose, and suitable mixtures thereof, vegetable oils (e.g., olive oil), and injectable organic esters (e.g., ethyl oleate). In the case of dispersions, the required particle size may be maintained by using coating materials such as lecithin, and the suitable fluidity may be maintained by using surfactants.

When the pharmaceutical composition is in a capsule form (e.g., gelatin capsules), in addition to the above-mentioned types of substances, it may also contain a liquid carrier such as polyethylene glycol or oil. The pharmaceutical composition may be in a liquid form, such as tinctures, syrups, solutions, emulsions, or suspensions. When administered orally, the composition preferably contains, in addition to the compound of the present application, one or more of sweeteners, preservatives, dyes/colorants, and flavor enhancers. For compositions intended for injection, one or more of surfactants, preservatives, wetting agents, dispersants, suspending agents, buffers, stabilizers, and isotonic agents may be included.

The pharmaceutical composition of the present application may include various substances that modify the physical form of solid or liquid dosage units.

The pharmaceutical composition of the present application in a solid or liquid form may include an agent that binds to the compound of the present application and thereby aids in the delivery of the compound. Suitable agents with this capability include monoclonal or polyclonal antibodies, proteins, or liposomes.

The pharmaceutical composition of the present application may be prepared using methods well-known in the pharmaceutical field. For example, the pharmaceutical composition intended for administration by injection may be prepared by combining the compound of the present application with sterile distilled water to form a solution. A surfactant may be added to form a homogeneous solution or suspension. The surfactant is a compound that interacts non-covalently with the compound of the present application, thereby promoting the dissolution or uniform suspension of the compound of the present application in an aqueous delivery system.

The compound represented by Formula (I) or the pharmaceutically acceptable salt, stereoisomer, racemate, deuterated compound, or solvate thereof of the present application is administered in a therapeutically effective amount, which will vary depending on a variety of factors, including the activity of the specific compound used; the metabolic stability and duration of action of the compound; the age, weight, general health status, gender, diet, mode and timing of administration, excretion rate, drug combination, severity of the specific condition or disorder of the patient; and the individual receiving the therapy.

The compound represented by Formula (I) or the pharmaceutically acceptable salt, stereoisomer, racemate, deuterated compound, or solvate thereof of the present application may also be administered simultaneously with, before, or after the administration of one or more other therapeutic agents. This combination therapy includes the administration of a single pharmaceutical dosage formulation containing the compound of the present application and one or more additional active agents, as well as the administration of the compound represented by Formula (I) of the present application with separate pharmaceutical dosage formulations of each active agent.

A medical composition consists of the compound represented by Formula (I) of the present application or the pharmaceutically acceptable salt, stereoisomer, racemate, deuterated compound, or solvate thereof, combined with an immunomodulator, an antibiotic (e.g., penicillins, aminoglycosides, quinolones, macrolides, etc.), and an antiviral drug (e.g., neuraminidase inhibitors, cap-dependent endonuclease inhibitors, RNA-dependent RNA polymerase inhibitors, M2 protein inhibitors, etc.).

Definitions and Explanations

The definitions of groups and terms described in the present application, including those defined as examples, exemplary definitions, preferred definitions, definitions of specific compounds in the examples, etc., may be arbitrarily combined with each other.

A specific term shall not be considered uncertain or unclear if it is not specifically defined, but shall be understood in accordance with its ordinary meaning in the art. When a trade name appears herein, it is intended to refer to the corresponding commodity or its active ingredient.

Unless otherwise defined, the following terms used in the description and claims have the meanings set forth below.

The term "stereoisomer" refers to isomers resulting from different spatial arrangements of atoms in a molecule, including cis-trans isomers, enantiomers, and diastereomers.

The term "pharmaceutical composition" refers to a mixture of one or more of the compounds of the present application with additional chemical components, such as a pharmaceutically acceptable carrier. The pharmaceutical composition is intended to facilitate the process of administering the compound to animals.

The term "pharmaceutical carrier" refers to an inactive ingredient in the pharmaceutical composition that does not cause significant irritation to the organism and does not interfere with the biological activity and properties of the

administered compound, such as, but not limited to, calcium carbonate, various sugars (e.g., lactose, mannitol, etc.), starch, cyclodextrins, magnesium stearate, cellulose, gelatin, water, polyethylene glycol, propylene glycol, ethylene glycol, castor oil or hydrogenated castor oil or polyethoxylated hydrogenated castor oil, sesame oil, corn oil, peanut oil, and the like.

The term "substituted" means that any one or more hydrogen atoms on a specific atom are replaced by substituents, provided that the valence state of the specific atom is normal and the substituted compound is stable. When the substituent is oxo (i.e., =O), it means that two hydrogen atoms are replaced, and oxo does not occur on aromatic groups. Unless otherwise specified, the term "substituted" refers to any level of substitution, such as monosubstituted, di-substituted, tri-substituted, tetra-substituted, or penta-substituted, as long as such substitution is permitted. The substituents are selected independently, and substitution may occur at any chemically accessible position. It should be understood that substitution on a given atom is limited by valence. It should also be understood that the substitution on a given atom results in a chemically stable molecule.

The term "optional" or "optionally" means that the subsequent described event or situation may or may not occur, and the description includes both the occurrence and non-occurrence of the event or situation. For example, ethyl being "optionally" substituted with halogen, means that ethyl may be unsubstituted ($CH_2CH_3$), monosubstituted (e.g., $CH_2CH_2F$, $CH_2CH_2Cl$), poly-substituted (e.g., $CHFCH_2F$, $CH_2CHF_2$, $CHFCH_2Cl$, $CH_2CHCl_2$), or fully substituted (e.g., $CF_2CF_3$, $CF_2CCl_3$, $CCl_2CCl_3$). Those skilled in the art will understand that for any group containing one or more substituents, no substitution or substitution pattern that is sterically impossible and/or cannot be synthesized will be introduced.

When any variable (e.g., B) appears more than once in the composition or structure of a compound, its definition in each case is independent. For example, if a group is substituted with 2 $R^b$, each $R^b$ has independent options.

When a bond of a substituent is cross-connected to two atoms on a ring, the substituent may be bonded to any atom on the ring.

The term "pharmaceutically acceptable" refers to those compounds, materials, compositions, and/or dosage forms that, within the scope of sound medical judgment, are suitable for use in contact with human and animal tissues without excessive toxicity, irritation, allergic reactions, or other problems or complications, and are commensurate with a reasonable benefit-risk ratio.

The term "alkyl", used alone or in combination with other terms, refers to a saturated hydrocarbon group that may be linear or branched. The term "Cn-Cm alkyl" refers to an alkyl group having n to m carbon atoms. In some embodiments, the alkyl group contains 1 to 10 carbon atoms, 1 to 6 carbon atoms, 1 to 4 carbon atoms, 1 to 3 carbon atoms, or 1 to 2 carbon atoms. Examples of an alkyl include, but are not limited to, methyl, ethyl, n-propyl, isopropyl, n-butyl, tert-butyl, isobutyl, sec-butyl, 2-methyl-1-butyl, n-pentyl, 3-pentyl, n-hexyl, 1,2,2-trimethylpropyl, and the like.

The term "alkenyl", used alone or in combination with other terms, refers to a linear or branched hydrocarbon group with one or more carbon-carbon double bonds. The term "Cn-Cm alkenyl" refers to an alkenyl group having n to m carbon atoms. In some embodiments, the alkenyl group contains 2 to 10, 2 to 6, 2 to 4, or 2 to 3 carbon atoms. Examples of the alkenyl group include, but are not limited to, vinyl, n-propenyl, isopropenyl, n-butenyl, sec-butenyl, and the like.

The term "alkylene", used alone or in combination with other terms, refers to a divalent alkyl linking group. The term "Cn-Cm alkylene" refers to an alkylene group having n to m carbon atoms. In some embodiments, the alkylene group contains 1 to 10 carbon atoms, 1 to 6 carbon atoms, 1 to 4 carbon atoms, 1 to 3 carbon atoms, or 1 to 2 carbon atoms. Examples of the alkylene group include, but are not limited to, methylene, ethylene, propylene, ethane-1,2-diyl, ethane-1,1-diyl, propane-1,3-diyl, propane-1,2-diyl, propane-1,1-diyl, butane-1,4-diyl, butane-1,3-diyl, butane-1,2-diyl, 2-methyl-propane-1,3-diyl, and the like.

The term "carbonyl", used alone or in combination with other terms, refers to the -C(=O)- group, which can also be written as C(O).

The term "azido" refers to $-N_3$.

The term "cyano" or "nitrile" refers to a group of the formula -C≡N, which can also be written as -CN.

The term "halo" or "halogen", used alone or in combination with other terms, refers to fluorine, chlorine, bromine, and iodine. In some embodiments, "halo" refers to a halogen atom selected from F or Cl. In some embodiments, the halo group is F.

As used herein, the term "haloalkyl" refers to an alkyl group in which one or more hydrogen atoms are replaced by halogen atoms. The term "Cn-Cm haloalkyl" refers to a Cn-Cm alkyl group having n to m carbon atoms and containing at least 1 to at most {2(n to m)+1} halogen atoms, where the halogen atoms may be the same or different. In some embodiments, the halogen atoms are fluorine atoms. In some embodiments, the haloalkyl group has 1 to 10, 1 to 6, or 1 to 4 carbon atoms. Examples of the haloalkyl group include $CF_3$, $C_2F_5$, $CH_2CF_3$, $CHF_2$, $CH_2F$, $CCl_3$, $CHCl_2$, $C_2Cl_5$, etc. In some embodiments, the haloalkyl group is trifluoromethyl ($CF_3$).

The term "aryl", used alone or in combination with other terms, refers to an aromatic hydrocarbon group, which may be monocyclic or polycyclic (e.g., having 2 fused rings). The term "Cn-Cm aryl" refers to an aryl group having n to m ring carbon atoms. The aryl group includes, for example, phenyl, naphthyl, and the like. In some embodiments, the aryl group has 5 to 10 carbon atoms. In some embodiments, the aryl group has 6 carbon atoms. In some embodiments, the aryl

group has 10 carbon atoms. In some embodiments, the aryl group is phenyl.

The term "heterocycle" refers to a saturated, partially unsaturated, or fully unsaturated monocyclic or polycyclic heterocyclic ring that has at least one heteroatom ring member selected from the group consisting of sulfur, oxygen, and nitrogen. In some embodiments, the heterocycle has 1, 2, 3, or 4 heteroatom ring members independently selected from the group consisting of nitrogen, sulfur, and oxygen. In some embodiments, the heterocycle has 3 to 6, 3 to 7, 5 to 12, or 6 to 12 ring atoms (including carbon atoms) and 1, 2, 3, or 4 heteroatom ring members independently selected from the group consisting of nitrogen, sulfur, and oxygen. The heterocycle includes heteroaromatic rings.

The term "heteroaryl", used alone or in combination with other terms, refers to a monocyclic or polycyclic aromatic heterocyclic ring containing at least one heteroatom ring member selected from the group consisting of sulfur, oxygen, and nitrogen. In some embodiments, the heteroaryl ring has 1, 2, 3, or 4 heterocyclic ring members independently selected from nitrogen, sulfur, and oxygen. In some embodiments, the heteroaryl has 5 to 14 or 5 to 12 ring atoms (including carbon atoms) and 1, 2, 3, or 4 heteroatom ring members independently selected from the group consisting of nitrogen, sulfur, and oxygen. In some embodiments, the heteroaryl has 5 to 10 ring atoms (including carbon atoms) and 1, 2, 3, or 4 heteroatom ring members independently selected from the group consisting of nitrogen, sulfur, and oxygen. In some embodiments, the heteroaryl has 5 to 6 ring atoms and 1 or 2 heteroatom ring members independently selected from the group consisting of nitrogen, sulfur, and oxygen. In some embodiments, the heteroaryl is a 5-membered or 6-membered heteroaryl ring. In other embodiments, the heteroaryl is an 8-membered, 9-membered, or 10-membered fused bicyclic heteroaryl ring. Examples of the heteroaryl include, but are not limited to, thienyl, furyl, pyrrolyl, oxazolyl, thiazolyl, imidazolyl, pyrazolyl, isoxazolyl, isothiazolyl, oxadiazolyl, triazolyl, tetrazolyl, thiadiazolyl, and their benzo derivatives (e.g., benzofuryl, benzothienyl, benzothiazolyl, benzoxazolyl, benzisoxazolyl, benzimidazolyl, benzotriazolyl, indazolyl, indolyl, isoindolyl, etc.); or pyridyl, pyridazinyl, pyrimidinyl, pyrazinyl, triazinyl, and their benzo derivatives (e.g., quinolyl, quinazolinyl, isoquinolyl, etc.); or azocinyl, indolizinyl, purinyl, and their benzo derivatives; or cinnolinyl, phthalazinyl, quinazolinyl, quinoxalinyl, naphthyridinyl, pteridinyl, carbazolyl, acridinyl, phenazinyl, phenothiazinyl, phenoxazinyl, etc. The term "5-6 membered heteroaryl" refers to an aromatic ring system having 5 or 6 ring atoms, which contains 1 to 3, preferably 1 or 2, heteroatoms independently selected from the group consisting of N, O, and S.

A 5-membered heteroaryl ring refers to a heteroaryl group with five ring atoms, where one or more (e.g., 1, 2, or 3) of the ring atoms are independently selected from the group consisting of N, O, and S.

A 6-membered heteroaryl ring refers to a heteroaryl group with six ring atoms, where one or more (e.g., 1, 2, or 3) of the ring atoms are independently selected from the group consisting of N, O, and S.

The term "carbocycle", used alone or in combination with other terms, refers to a non-aromatic hydrocarbon ring system (monocyclic, bicyclic, or polycyclic), including cyclized alkyl and cyclized alkenyl. The term "Cn-Cm carbocycle" refer to a cycloalkyl group having n to m ring-forming carbon atoms. The carbocycle may have 3, 4, 5, 6, or 7 ring-forming carbon atoms (C3-C7, C3-C6, or C3-C4). In some embodiments, the carbocycle has 3 to 7 ring members, 3 to 6 ring members, or 3 to 4 ring members. In some embodiments, the carbocycle is monocyclic. In some embodiments, the carbocycle is monocyclic or bicyclic. Examples of the carbocycle include, but are not limited to, cyclopropyl, cyclobutyl, cyclopentyl, cyclohexyl, cycloheptyl, cyclopentenyl, cyclohexenyl, cyclohexadienyl, and the like. In some embodiments, the carbocycle is cyclopropyl, cyclobutyl, cyclopentyl, or cyclohexyl. In some embodiments, the carbocycle is cyclopropyl.

The term "nitrogen-containing heterocycle" refers to a heterocycle in which the ring-forming atoms include at least one nitrogen heteroatom in addition to carbon atoms. In some embodiments, the nitrogen-containing heterocycle has 3 to 7 ring members, 3 to 6 ring members, or 3 to 4 ring members. Examples of the nitrogen-containing heterocycle include, but are not limited to, pyrrole, imidazole, thiazole, pyridine, triazole, tetrazole, quinoline, indole, and the like. In some embodiments, the 4- to 7-membered nitrogen-containing heterocycle is pyrrole, pyridine, or tetrazole.

The term "heteroalkyl" refers to an alkyl group in which one or more (preferably 1, 2, or 3) carbon atoms are replaced by oxygen, nitrogen, phosphorus, silicon, or sulfur atoms (preferably oxygen, sulfur, or nitrogen). Examples of the heteroalkyl include C1-C10 heteroalkyl, C1-C6 heteroalkyl, and C1-C3 heteroalkyl. The term "heteroalkenyl" refers to an alkenyl group in which one or more (preferably 1, 2, or 3) carbon atoms are replaced by oxygen, nitrogen, phosphorus, silicon, or sulfur atoms (preferably oxygen, sulfur, or nitrogen). Examples of the heteroalkenyl include C2-C10 heteroalkenyl, C2-C6 heteroalkenyl, and C2-C3 heteroalkenyl.

As used herein, the expressions "ambient temperature" and "room temperature" are understood in the art and generally refer to a temperature, such as a reaction temperature, that is approximately the temperature of the room where the reaction is conducted, for example, a temperature ranging from about 20°C to about 30°C.

The present invention also includes pharmaceutically acceptable salts of the compounds described herein. The term "pharmaceutically acceptable salt" refers to a derivative of the disclosed compound, where the parent compound is modified by converting the existing acidic or basic moiety into its salt form. The pharmaceutically acceptable salts of the present invention include, for example, non-toxic salts of the parent compound formed with non-toxic inorganic or organic acids. The pharmaceutically acceptable salts of the present invention may be synthesized from the parent compound containing a basic or acidic moiety through conventional chemical methods.

As used herein, the term "heteroatom" shall be understood to mean an atom other than carbon, such as O, N, or S.

## DESCRIPTION OF DRAWINGS

FIG. 1 shows the effect curves of repeated administration at a dose of 50 mg/kg of CX-5461, Compound H-31, and the vehicle on the body weight of mice.

FIG. 2-1 and FIG. 2-2 show the effect curves of repeated administration at a dose of 55 mg/kg of CX-5461, compounds of the H series, and the vehicle on the body weight of mice.

FIG. 3 shows the inhibitory effects of Olaparib, Compound H-31, and the vehicle on tumors in the human triple-negative breast cancer MDA-MB-436 model.

## DESCRIPTION OF EMBODIMENTS

The present application is further described below in conjunction with the examples, but these examples are not intended to limit the scope of the present application.

For the experimental methods in the examples of the present application where specific conditions are not indicated, conventional conditions are generally followed; for reagents where specific sources are not indicated, they are conventional commercially available reagents.

Unless otherwise defined, the terms used in the description and claims shall have the following meanings.

The term "pharmaceutical composition" refers to a mixture of one or more of the compounds of the present application with additional chemical components, such as a pharmaceutically acceptable carrier. The pharmaceutical composition is intended to facilitate the administration process of the compound to animals.

The term "pharmaceutical carrier" refers to an inactive ingredient in the pharmaceutical composition that does not cause significant irritation to the organism and does not interfere with the biological activity and properties of the administered compound, for example, but not limited to, calcium carbonate, calcium phosphate, various sugars (e.g., lactose, mannitol, etc.), starch, cyclodextrins, magnesium stearate, cellulose, magnesium carbonate, acrylic polymers or methacrylic polymers, gelatin, water, polyethylene glycol, propylene glycol, ethylene glycol, castor oil or hydrogenated castor oil or polyethoxylated hydrogenated castor oil, sesame oil, corn oil, peanut oil, and the like.

The present application is further illustrated below in conjunction with examples:

Example 1: Preparation of Compound H-1

1. Preparation of 2,5-dichloropyridine-3-carbonyl chloride

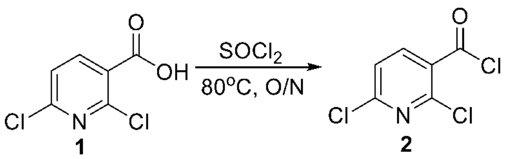


Compound 2,5-dichloropyridine-3-carboxylic acid (5 g, 26.0 mmol, 1.0 eq.) was added to thionyl chloride (40 mL) under ice-bath cooling, and then the reaction system was heated to 80°C and reacted overnight (o/n, same applies below). Thionyl chloride was removed under vacuum to obtain 2,5-dichloropyridine-3-carbonyl chloride (5.4 g, a yellow oily substance, with a yield of 98%). LC-MS (ESI): m/z 206.00 $[M+1]^+$ (the acyl chloride was quenched with methanol, and the MS shows the methyl ester of Compound **1**). $^1H$ NMR (400 MHz, $CDCl_3$) δ: 8.40-8.30 (m, 1H), 7.50-7.40 (m, 1H).

2. Preparation of Compound **4**

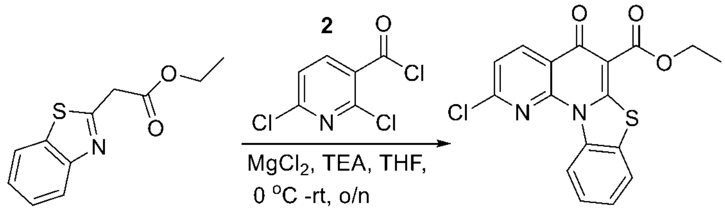


2

3 4

Compound **3** (1.0 g, 4.5 mmol, 1.0 eq.) was dissolved in tetrahydrofuran (10 mL), and then anhydrous magnesium chloride (430 mg, 4.5 mmol, 1.0 eq.) was added to the system. Under nitrogen protection, the reaction mixture was stirred at 0°C for half an hour. Then 2,5-dichloropyridine-3-carbonyl chloride (Compound 2, 2.8 g, 13.5 mmol, 3.0 eq.) and triethylamine (2.3 g, 22.6 mmol, 5.0 eq.) were slowly added to the system respectively. The mixture reacted overnight at room temperature. The reaction mixture was directly filtered, and the filter cake was washed with water and methanol, and then dried to obtain Compound **4** (1.2 g, a light yellow solid, with a yield of 68%). LC-MS (ESI): m/z 359.2 $[M+1]^+$, $^1H$ NMR (400 MHz, $CDCl_3$) δ: 9.53 (d, 1H), 8.84 (d, 1H), 7.82-7.69 (m, 1H), 7.64-7.52 (m, 2H), 7.50-7.40 (m, 1H), 4.58-4.42 (m, 2H), 1.50-1.45 (m, 3H).

3. Preparation of Compound **6**

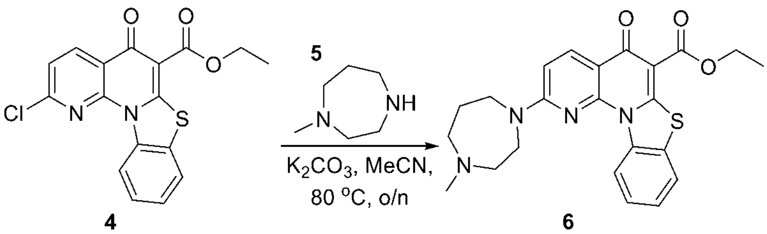


5

4 6

Compound **4** (5 g, 13.9 mmol, 1.0 eq.) and potassium carbonate (9.6 g, 69.5 mmol, 5.0 eq.) were added to acetonitrile (80 mL). Then N-methylhomopiperazine (3.2 g, 27.9 mmol, 2.0 eq.) was added to the system. Under nitrogen protection, the reaction mixture reacted overnight at 80°C. After being cooled to room temperature, the reaction mixture was directly filtered, and the filter cake was washed with water and methanol, and then dried to obtain Compound **6** (4.4 g, a light yellow solid, with a yield of 72%). LC-MS (ESI): m/z 437.2 $[M+1]^+$, $^1$HNMR(400 MHz, DMSO-$d_6$) δ: 9.23(d, 1H), 8.15 (d, 1H), 7.91-7.86 (m, 1H), 7.46-7.32 (m, 2H), 6.84 (d, 1H), 4.28 (q, 2H), 3.90-3.50 (m, 4H), 2.80-2.60 (m, 2H), 2.50-2.40 (m, 2H), 2.30-2.15 (m, 3H), 2.00-1.85 (m, 2H), 1.29 (t, 3H).

4. Preparation of Compound **7**

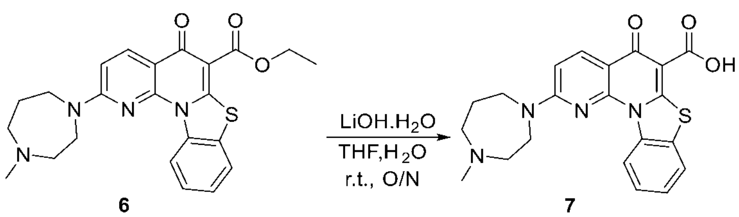


6 7

Compound **6** (4.45 g, 10.2 mmol, 1.0 eq.) was dissolved in tetrahydrofuran (30 mL) and water (30 mL), and lithium hydroxide monohydrate (1.23 g, 30.6 mmol, 3.0 eq.) was added to the system. The reaction mixture reacted overnight at room temperature, and then directly filtered. The filter cake was washed with ethyl acetate, and dried under vacuum to obtain Compound **7** (4.15 g, a light yellow solid, with a yield of 98%). LC-MS (ESI): m/z 409.1 $[M+1]^+$, $^1H$ NMR (400 MHz, DMSO-$d_6$) δ: 9.40 (d, 1H), 8.36 (d, 1H), 7.87 (d, 1H), 7.50-7.35 (m, 2H), 7.10-6.90 (m, 1H), 4.10-3.50 (m, 4H), 2.75-2.65 (m, 2H), 2.50-2.40 (m, 2H), 2.24 (s, 3H), 2.00-1.90 (m, 2H).

5. Preparation of Compound **H-1**

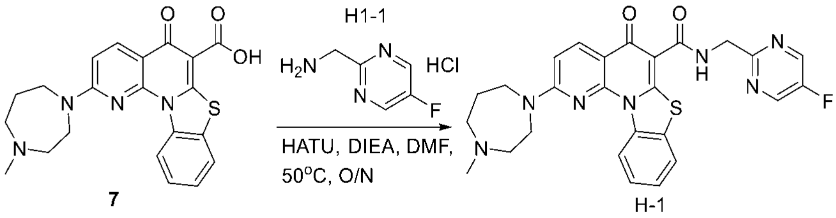


2-(7-Azabenzotriazol-1-yl)-N,N,N',N'-tetramethyluronium hexafluorophosphate (0.07 g, 0.184 mmol, 1.5 eq.) and N,N-diisopropylethylamine (0.065 g, 0.488 mol, 4.0 eq.) were added to a solution of Compound 7 (0.05 g, 0.122 mmol, 1.0 eq.) in N,N-dimethylformamide (3 mL). The mixture was stirred at 50°C for 0.5 hour under nitrogen protection. Then Compound **H1-1** (0.031 g, 0.184 mmol, 1.5 eq.) dissolved in N,N-dimethylformamide (1 mL) was added, and the resulting mixture was stirred at 50°C for 16 hours under nitrogen protection. The reaction mixture was spin dried to obtain a residue. The residue was slurried with methanol and filtered, and the filter cake was washed and dried to obtain Compound **H-1** (0.043 g, a light yellow solid, with a yield of 68%). LC-MS (ESI): m/z 518.4 $[M+1]^+$, $^1H$ NMR (400 MHz, DMSO-$d_6$) δ: 11.11 (d, 1H), 9.30-9.15 (m, 1H), 8.95-8.85 (m, 2H), 8.36 (d, 1H), 7.95 (d, 1H), 7.63-7.37 (m, 2H), 7.15-6.95 (m, 1H), 4.85-4.70 (m, 2H), 4.15-3.90 (m, 2H), 3.90-3.60 (m, 2H), 3.60-3.50 (m, 2H), 3.30-3.20 (m, 2H), 2.80-2.60 (m, 3H), 2.30-2.10 (m, 2H).

Example 2: Preparation of Compound H-2

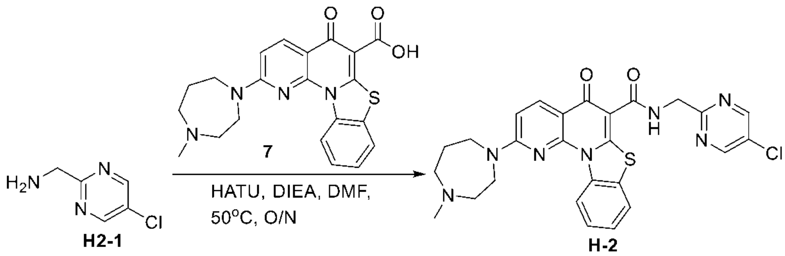


2-(7-Azabenzotriazol-1-yl)-N,N,N',N'-tetramethyluronium hexafluorophosphate (0.16 g, 0.42 mmol, 1.5 eq.) and N,N-diisopropylethylamine (0.15 mL, 0.84 mmol, 3.0 eq.) were added to a solution of Compound 7 (0.114 g, 0.28 mmol, 1.0 eq.) in N,N-dimethylformamide (3 mL). The mixture was stirred at 50°C for 0.5 hour under nitrogen protection. Then Compound **H2-1** (0.06 g, 0.42 mmol, 1.5 eq.) dissolved in N,N-dimethylformamide (2 mL) was added, and the resulting mixture was stirred at 50°C for 16 hours under nitrogen protection. The reaction mixture was spin dried to obtain a residue. The residue was purified by preparative HPLC, and the eluate was lyophilized to obtain Compound **H-2** (0.0245 g, a light yellow solid, with a yield of 17%). LC-MS (ESI): m/z 534.2 $[M+1]^+$, $^1H$ NMR (400 MHz, DMSO-$d_6$) δ: 11.16 (s, 1H), 9.50-9.35 (m, 1H), 8.95-8.85 (m, 2H), 8.45-8.30 (m, 1H), 8.30-8.20 (m, 1H), 8.05-7.90 (m, 1H), 7.60-7.40 (m, 2H), 7.10-6.95 (m, 1H), 4.85-4.70 (m, 2H), 4.05-3.60 (m, 6H), 2.85-2.65 (m, 2H), 2.26 (s, 3H), 2.05-1.90 (m, 2H).

Example 3: Preparation of Compound H-4

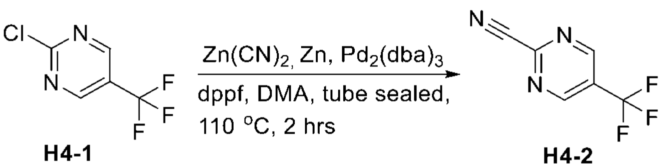


H4-1 H4-2

Zinc cyanide (0.43 g, 3.62 mmol, 0.66 eq.), zinc (0.13 g, 1.97 mmol, 0.36 eq.), tris(dibenzylideneacetone) dipalladium (0.15 g, 0.16 mmol, 0.03 eq.) and 1,1'-bis(diphenylphosphino)ferrocene (0.19 g, 0.33 mmol, 0.06 eq.) were

added to a solution of Compound **H4-1** (1.0 g, 5.48 mmol, 1.0 eq.) in DMA (40 mL). The mixture reacted in a sealed tube at 110°C for 2 hours under nitrogen protection. The reaction mixture was extracted with ethyl acetate and saturated brine, and the organic phases were combined, and then spin dried to obtain a residue. The residue was purified by silica gel column chromatography to obtain Compound **H4-2** (0.3 g, a yellow oil, with a yield of 30%). $^1$H NMR (400 MHz, $CDCl_3$) δ: 9.11 (t, 2H).

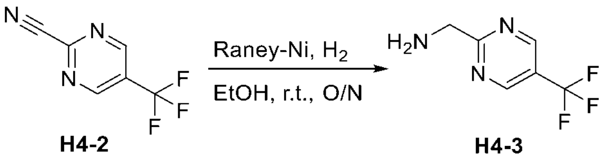


**H4-2** **H4-3**

Raney-Ni (0.08 g) and $NH_3.H_2O$ (0.2 mL) were added to a solution of Compound **H4-2** (0.4 g, 2.31 mmol) in EtOH (20 mL). The mixture was stirred at room temperature for 2 hours under a hydrogen atmosphere (3.5 MPa). After filtration, the filtrate was spin dried to obtain a residue, which was directly used in the next step without further purification. LC-MS (ESI): m/z 178.0 $[M+1]^+$.

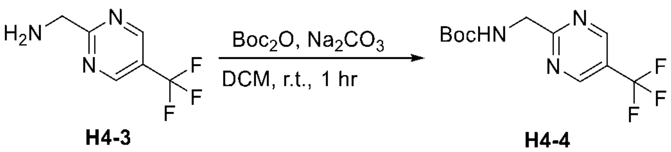


**H4-3** **H4-4**

Di-tert-butyl dicarbonate (0.33 g, 1.49 mmol, 1.2 eq.) and an aqueous solution of saturated sodium bicarbonate (4 mL) were added to a solution of crude Compound **H4-3** (0.22 g, 1.24 mmol, 1.0 eq.) in dichloromethane (6 mL). The mixture was stirred at room temperature for 1 hour under nitrogen protection. The reaction mixture was extracted with dichloromethane and water, and the organic phases were combined and dried, and then spin dried to obtain a residue. The residue was purified by silica gel column chromatography to obtain Compound **H4-4** (0.07 g, a yellow oil, with a yield of 36%). LC-MS (ESI): m/z 279.2 $[M+1]^+$, $^1$H NMR (400 MHz, $CDCl_3$) δ: 8.93 (s, 2H), 5.57 (s, 1H), 4.66 (d, 2H), 1.46 (s, 9H).

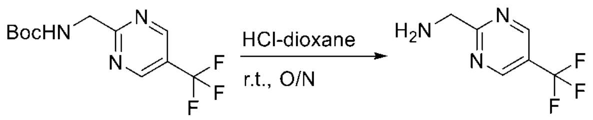


**H4-4** **H4-5**

HCl-dioxane (4M, 3 mL) was added to Compound **H4-4** (0.07 g, 0.25 mmol). The mixture was stirred at room temperature for 16 hours under nitrogen protection. The solvent was removed by spin drying to obtain Compound **H4-5** (65 mg, a gray solid, with a yield of 100%). LC-MS (ESI): m/z 178.0 $[M+1]^+$, $^1$H NMR (400 MHz, DMSO-$d_6$) δ: 9.36 (s, 2H), 8.58 (s, 3H), 4.41 (s, 2H).

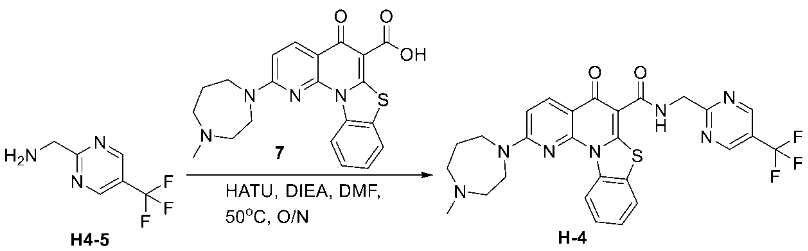


**H4-5** **H-4**

2-(7-Azabenzotriazol-1-yl)-N,N,N',N'-tetramethyluronium hexafluorophosphate (0.14 g, 0.37 mmol, 1.5 eq.) and N,N-diisopropylethylamine (0.13 g, 1.0 mmol, 4.0 eq.) were added to a solution of Compound 7 (0.1 g, 0.25 mmol, 1.0 eq.) in N,N-dimethylformamide (4 mL). The mixture was stirred at 50°C for 0.5 hour under nitrogen protection. Then Compound **H4-5** (0.08 g, 0.37 mmol, 1.5 eq.) dissolved in N,N-dimethylformamide (2 mL) was added, and the resulting mixture was stirred at 50°C for 16 hours under nitrogen protection. The reaction mixture was spin dried to obtain a residue. The residue was purified by preparative thin-layer chromatography to obtain Compound **H-4** (0.06 g, a light yellow solid, with a yield of 44%). LC-MS (ESI): m/z 568.8 $[M+1]^+$, $^1$H NMR (400 MHz, DMSO-$d_6$) δ: 11.25-11.15 (m, 1H), 9.45-9.30 (m, 1H), 9.26 (s, 2H), 8.32 (d, 1H), 7.94 (d, 1H), 7.55-7.45 (m, 1H), 7.45- 7.35 (m, 1H), 7.05-6.95 (m, 1H), 4.90 (d, 2H), 4.05-3.55 (m, 4H),

2.80-2.65 (m, 2H), 2.55-2.50 (m, 2H), 2.26 (s, 3H), 2.00-1.90 (m, 2H).

Example 4: Preparation of Compound H-5

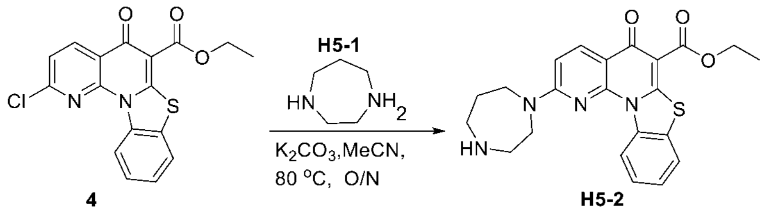


4 H5-2

Potassium carbonate (616 mg, 4.459 mmol, 4.0 eq.) and Compound **H5-1** (167 mg, 1.672 mmol, 1.5 eq.) were added to a solution of Compound 4 (400 mg, 1.114 mmol, 1.0 eq.) in acetonitrile (8 mL). The reaction mixture was stirred at 80°C for 16 hours under nitrogen protection. The resulting solution was directly spin dried, then water was added, and the mixture was washed and extracted with dichloromethane/methanol (10/1, v/v). The organic phases were combined and spin dried. The crude product was slurried with ethyl acetate overnight and filtered to obtain the filter residue as Product **H5-2** (320 mg, a white solid, with a yield of 68%). LC-MS (ESI): m/z 423.5 $[M+1]^+$.

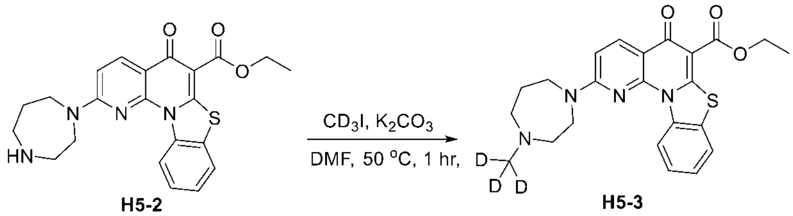


H5-2 H5-3

Potassium carbonate (265 mg, 1.917 mmol, 3.0 eq.) was added to a solution of Compound **H5-2** (270 mg, 0.639 mmol, 1.0 eq.) in N,N-dimethylformamide (7 mL). After stirring at 50°C for 10 minutes, iodomethane-$d_3$ (70 mg, 0.479 mmol, 0.75 eq.) was added. The reaction mixture reacted at 50°C for 0.5 hour under a nitrogen atmosphere. The reaction was quenched by adding water, and extracted with ethyl acetate. The organic phases were combined, concentrated, and purified by column chromatography to finally obtain Product **H5-3** (180 mg, a white solid, with a yield of 60%). LC-MS (ESI): m/z 440.6 $[M+1]^+$.

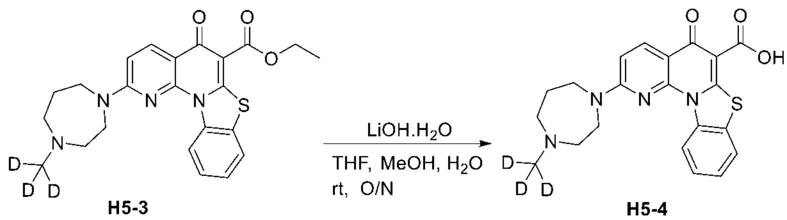


H5-3 H5-4

$LiOH.H_2O$ (26 mg, 0.614 mmol, 3.0 eq.) was added to a solution of Compound **H5-3** (90 mg, 0.205 mmol, 1.0 eq.) in tetrahydrofuran/methanol/water (3 mL/1 mL/3 mL). The mixture was stirred overnight at room temperature under nitrogen protection. The solvent was removed under reduced pressure, and lyophilization was performed to obtain Compound **H5-4** (91 mg, a white solid, crude product containing salt). LC-MS (ESI): m/z 412.2 $[M+1]^+$, $^1H$ NMR (400 MHz, DMSO-$d_6$) δ: 9.50-9.35 (m, 1H), 8.37 (d, 1H), 7.95-7.85 (m, 1H), 7.55-7.35 (m, 2H), 7.00 (d, 1H), 4.10-3.60 (m, 4H), 2.80-2.70 (m, 2H), 2.55-2.50 (m, 2H), 2.05-1.90 (m, 2H).

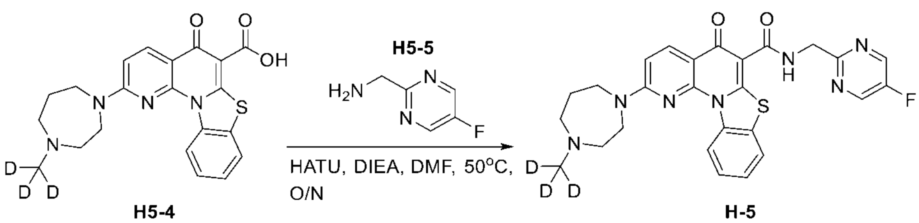


H5-4 H-5

2-(7-Azabenzotriazol-1-yl)-N,N,N',N'-tetramethyluronium hexafluorophosphate (125 mg, 0.328 mmol, 1.5 eq.) and N,N-diisopropylethylamine (142 mg, 1.094 mmol, 5.0 eq.) were added to a solution of Compound **H5-4** (91 mg, 0.219 mmol, 1.0 eq.) in N,N-dimethylformamide (3 mL). The mixture was stirred at 50°C for 0.5 hour under nitrogen protection. Then Compound **H5-5** (72 mg, 0.437 mmol, 2.0 eq.) dissolved in N,N-dimethylformamide (1 mL) was added, and the resulting mixture was stirred at 50°C for 16 hours under nitrogen protection. The reaction mixture was spin dried to obtain a residue. The crude product was directly slurried with methanol (5 mL) for 30 minutes, and filtered to obtain the filter residue, which was purified by preparative HPLC to finally obtain Compound **H-5** (12.8 mg, a light yellow solid, with a two-step yield of 11%). LC-MS (ESI): m/z 521.4 $[M+1]^+$, $^1$H NMR (400 MHz, DMSO-$d_6$) δ: 11.25-11.15 (m, 1H), 9.50-9.40 (m, 1H), 8.88 (s, 2H), 8.45-8.30 (m, 1H), 8.15-8.10 (m, 1H), 7.65-7.40 (m, 2H), 7.10-6.95 (m, 1H), 4.85-4.75 (m, 2H), 4.05-3.60 (m, 4H), 3.10-2.75 (m, 2H), 2.70-2.55 (m, 2H), 2.10-1.95 (m, 2H).

Example 5: Preparation of Compound H-6

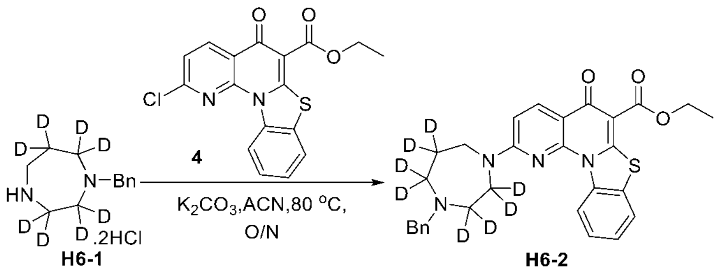


H6-1 H6-2

Potassium carbonate (4.3 g, 31.3 mmol, 5.0 eq.) was added to a solution of Compound 4 (1.7 g, 6.27 mmol, 1.0 eq.) in acetonitrile (50 mL). Then Compound **H6-1** (2.5 g, 6.89 mmol, 1.1 eq.) was added, and the mixture was stirred at 80°C for 16 hours under nitrogen protection. The reaction mixture was then cooled to room temperature, washed and extracted with dichloromethane/methanol (10/1, v/v), dried, and spin dried to remove the solvent to obtain a residue. The residue was purified by silica gel column chromatography to obtain Compound **H6-2** (2.8 g, a light yellow solid, with a yield of 72%). LC-MS (ESI): m/z 521.2 $[M+1]^+$, $^1$H NMR (400 MHz, DMSO-$d_6$) δ: 9.40-9.20 (m, 1H), 8.23 (d, 1H), 8.05-7.85 (m, 1H), 7.55- 7.35 (m, 2H), 7.30-7.10 (m, 5H), 6.94 (d, 1H), 4.29 (q, 2H), 3.90-3.65 (m, 2H), 3.59 (s, 2H), 1.29 (t, 3H).

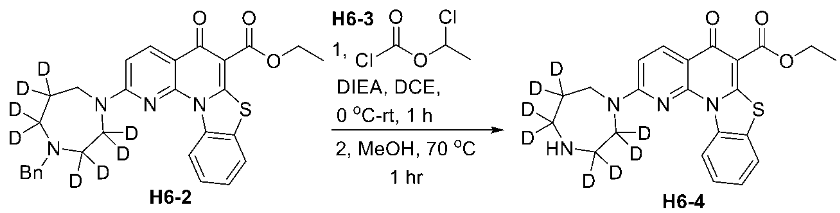


H6-2 H6-4

N,N-diisopropylethylamine (1.1 g, 8.64 mmol, 2.5 eq.) was added to a solution of Compound **H6-2** (1.8 g, 3.46 mmol, 1.0 eq.) in 1,2-dichloroethane (30 mL). Then Compound **H6-3** (0.99 g, 6.91 mmol, 2.0 eq.) was added at 0°C, and the mixture was stirred at room temperature for 1 hour under nitrogen protection. The reaction mixture was then spin dried and dissolved in methanol (30 mL). The resulting mixture was stirred at 70°C for 1 hour under nitrogen protection. The reaction mixture was spin dried, dichloromethane/ethyl acetate (10 mL/10 mL) was added, and the resulting mixture was stirred overnight at room temperature. Filtration was then performed, and the filter cake was washed with dichloromethane, and dried to obtain the crude product of Compound **H6-4** (1.64 g, a yellow solid). LC-MS (ESI): m/z 431.0 $[M+1]^+$, $^1$H NMR (400 MHz, DMSO-$d_6$) δ: 9.30-9.15 (m, 2H), 8.23 (d, 1H), 8.00-7.90 (m, 1H), 7.60-7.50 (m, 1H), 7.50-7.40 (m, 1H), 7.00 (d, 1H), 4.29 (q, 2H), 3.90-3.75 (m, 2H), 1.30 (t, 3H).

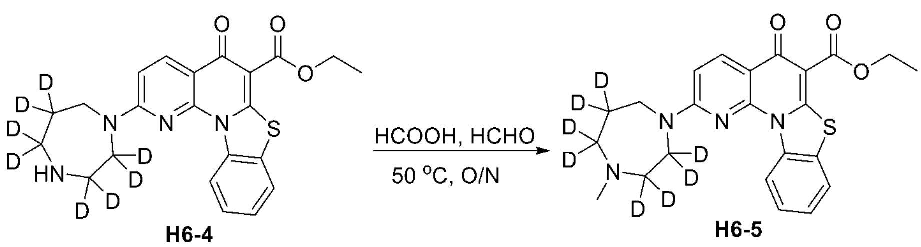


H6-4 H6-5

HCHO (12 mL) was added to a solution of Compound **H6-4** (1.13 g) in HCOOH (12 mL). The mixture was stirred overnight at 50°C under nitrogen protection. The pH of the solution was adjusted to be weakly alkaline with a saturated aqueous solution of sodium bicarbonate, and the aqueous phase was extracted with dichloromethane/methanol (10:1, v/v, 300 mL × 2). The combined organic layers were concentrated to obtain a residue. The residue was purified by silica gel column chromatography to obtain Compound **H6-5** (0.85 g, a light yellow solid, with a yield of 72%). LC-MS (ESI): m/z 445.0 [M+1]$^+$, $^1$H NMR (400 MHz, DMSO-$d_6$) δ: 9.40-9.20 (m, 1H), 8.19 (d, 1H), 8.00-7.90 (m, 1H), 7.55-7.30 (m, 2H), 7.00-6.80 (m, 1H), 4.28 (q, 2H), 3.80-3.50 (m, 2H), 2.27 (s, 3H), 1.30 (t, 3H).

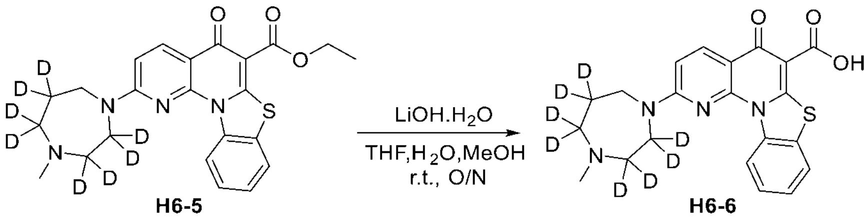


**H6-5** **H6-6**

LiOH•$H_2O$ (0.24 g, 5.74 mmol, 3.0 eq.) was added to a mixed solution of Compound **H6-5** (0.85 g, 1.91 mmol, 1.0 eq.) in tetrahydrofuran (10 mL), methanol (5 mL) and water (10 mL). The reaction mixture was stirred at room temperature for 16 hours under nitrogen protection. The reaction mixture was concentrated to obtain a residue. The residue was washed with water and filtered. The filter cake was dried to obtain Compound **H6-6** (0.9 g, a light yellow solid), which was used in the next step.

LC-MS (ESI): m/z 417.0 [M+1]$^+$, $^1$H NMR (400 MHz, DMSO-$d_6$) δ: 9.50-9.35 (m, 1H), 8.37 (d, 1H), 7.88 (d, 1H), 7.55-7.30 (m, 2H), 6.99 (d, 1H), 4.00-3.50 (m, 2H), 2.25 (s, 3H).

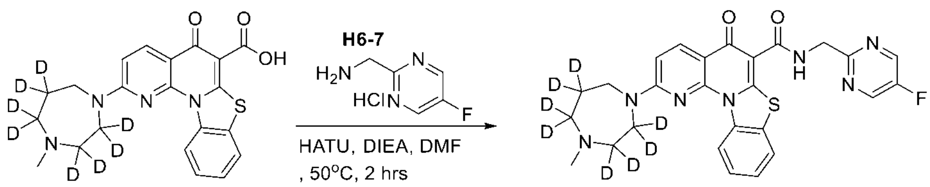


**H6-6** **H-6**

2-(7-Azabenzotriazol-1-yl)-N,N,N',N'-tetramethyluronium hexafluorophosphate (69 mg, 0.18 mmol, 1.5 eq.) and N,N-diisopropylethylamine (0.08 mL, 0.48 mmol, 4.0 eq.) were added to a solution of Compound **H6-6** (50 mg, 0.12 mmol, 1.0 eq.) in N,N-dimethylformamide (2 mL). The mixture was stirred at 50°C for 0.5 hour under nitrogen protection. Then Compound **H6-7** (0.03 g, 0.18 mmol, 1.5 eq.) dissolved in N,N-dimethylformamide (1 mL) was added, and the resulting mixture was stirred at 50°C for 16 hours under nitrogen protection. The reaction mixture was spin dried to obtain a residue, which was purified by preparative HPLC, and the eluate was lyophilized to obtain Compound **H-6** (6.7 mg, a light yellow solid, with a two-step yield of 10%). LC-MS (ESI): m/z 526.2 [M+1]$^+$, $^1$H NMR (400 MHz, DMSO-$d_6$) δ: 11.25-11.15 (m, 1H), 9.00-8.90 (m, 1H), 8.95-8.85 (m, 2H), 8.40-8.30 (md, 1H), 8.25-8.15 (m, 1H), 8.05-7.95 (m, 1H), 7.60-7.40 (m, 2H), 7.10-6.95 (m, 1H), 4.85-4.75 (m, 2H), 3.85-3.65 (m, 2H), 2.26 (s, 3H).

Example 6: Preparation of Compound H-7

1. Preparation of Compound 9

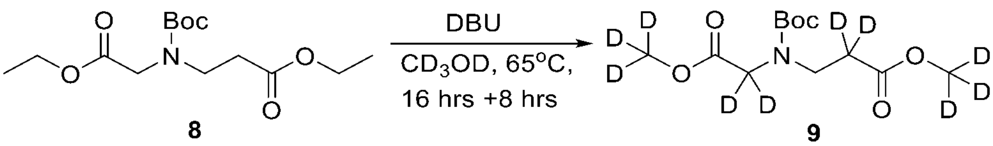


**8** **9**

Ethyl 3-(tert-butoxycarbonyl)(2-ethoxy-2-oxoethyl)amino)propanoate (1.0 g, 3.3 mmol, 1.0 eq.) was dissolved in deuterated methanol (15 mL), and 1,8-diazabicyclo[5.4.0]undec-7-ene (DBU) (8.3 g, 33.0 mmol, 10.0 eq.) was added to the solution. The mixture was stirred at 65°C for 16 hours. The reaction mixture was extracted with water and dichloromethane, and concentrated for the next step. MS-ESI: m/z 286 [M+1]$^+$.

2. Preparation of Compound **10**

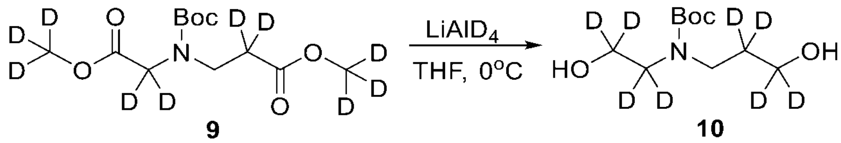


9 10

Compound **9** (500.0 mg, 1.8 mmol, 1.0 eq.) was dissolved in tetrahydrofuran (300 mL), and lithium aluminum deuteride (83 mg, 2.0 mmol, 1.1 eq.) was added to the solution. The mixture was stirred at 0°C for 2 hours. The reaction mixture was extracted with water and dichloromethane, and concentrated to obtain Compound **10** (400 mg, a yellow oil, with a yield of 97.5%). MS-ESI: m/z 228 $[M+1]^+$.

3. Preparation of Compound **11**

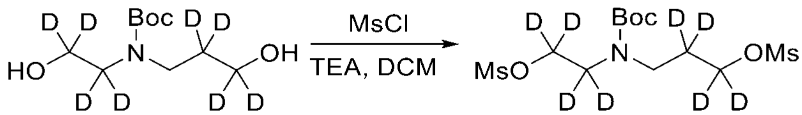


10 11

Compound **10** (400.0 mg, 1.8 mmol, 1.0 eq.) was dissolved in dichloromethane (200 mL), and triethylamine (545.0 mg, 5.4 mmol, 3.0 eq.) and methanesulfonyl chloride (311.0 mg, 2.7 mmol, 1.5 eq.) were added to the solution. The mixture was stirred at room temperature for 3 hours. The reaction mixture was extracted with water and dichloromethane, and concentrated to obtain Compound **11** (400 mg, a yellow oil, with a yield of 58.1%). MS-ESI: m/z 384 $[M+1]^+$.

4. Preparation of Compound **12**

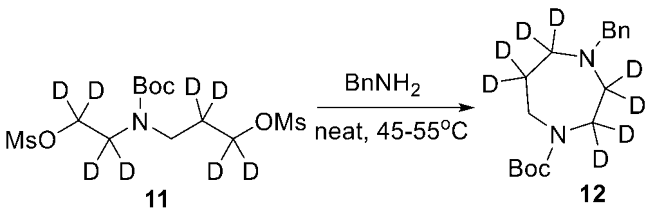


11 12

Compound **11** (400.0 mg, 1.0 mmol, 1.0 eq.) was dissolved in benzylamine (200 mL). The mixture was stirred at room temperature for 16 hours. The reaction mixture was extracted with water and dichloromethane, concentrated, and purified by column chromatography to obtain Compound **12** (200 mg, a yellow solid, with a yield of 64.7%). MS-ESI: m/z 299 $[M+1]^+$.

5. Preparation of Compound **13**

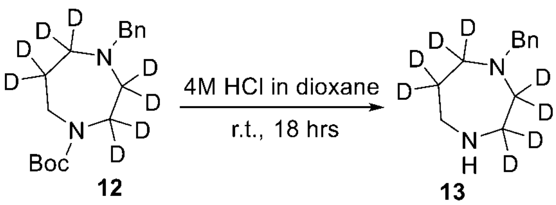


12 13

Compound **12** (200 mg, 0.7 mmol, 1.0 eq.) was dissolved in a solution of hydrogen chloride (4M) in 1,4-dioxane. The mixture was stirred at room temperature for 18 hours. The reaction mixture was filtered and concentrated to obtain Compound **13** (150 mg, a yellow oil, with a yield of 100%). MS-ESI: m/z 199 [M+1]$^+$.

6. Preparation of Compound **H7-1**

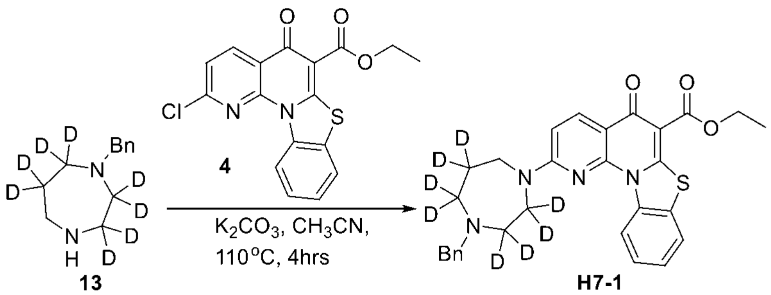


13 H7-1

Compound **13** (150.0 mg, 0.6 mmol, 1.0 eq.) from the previous step and Compound **4** (215.0 mg, 0.6 mmol, 1.0 eq.) were dissolved in acetonitrile (15 mL), and potassium carbonate (248.0 mg, 1.8 mmol, 3.0 eq.) was added to the solution. The mixture was stirred at 110°C for 4 hours. The reaction mixture was extracted with water and dichloromethane, and concentrated to obtain Compound **H7-1** (300 mg, a yellow solid, with a yield of 94.0%). MS-ESI: m/z 521 [M+1]$^+$, $^1$H NMR (400 MHz, DMSO-$d_6$) δ: 9.40-9.20 (m, 1H), 8.23 (d, 1H), 8.00-7.90 (m, 1H), 7.55-7.35 (m, 2H), 7.35-7.15 (m, 5H), 7.00-6.85 (m, 1H), 4.29 (q, 2H), 3.85-3.60 (m, 2H), 3.59 (s, 2H), 1.30 (t, 3H).

7. Preparation of Compound **H7-3**

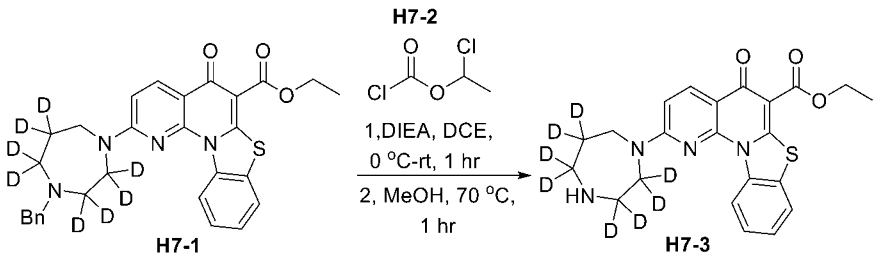


H7-1 H7-3

N,N-diisopropylethylamine (1.1 g, 8.64 mmol, 2.5 eq.) was added to a solution of Compound **H7-1** (1.8 g, 3.46 mmol, 1.0 eq.) in 1,2-dichloroethane (30 mL). Then Compound **H7-2** (0.99 g, 6.91 mmol, 2.0 eq.) was added at 0°C, and the mixture was stirred at room temperature for 1 hour under nitrogen protection. The reaction mixture was then spin dried and dissolved in methanol (30 mL). The mixture was stirred at 70°C for 1 hour under nitrogen protection. The reaction mixture was spin dried, dichloromethane/ethyl acetate (10 mL/10 mL) was added, and the mixture was stirred overnight at room temperature. Filtration was performed, and the filter cake was washed with dichloromethane, and dried to obtain a crude product of Compound **H7-3** (1.64 g, a yellow solid). LC-MS (ESI): m/z 431.0 [M+1]$^+$, $^1$H NMR (400 MHz, DMSO-$d_6$) δ: 9.35-9.15 (m, 2H), 8.23 (d, 1H), 8.00-7.90 (m, 1H), 7.60-7.50 (m, 1H), 7.50-7.40 (m, 1H), 7.00 (d, 1H), 4.29 (q, 2H), 3.83 (s, 2H), 1.30 (t, 3H).

8. Preparation of Compound **H7-4**

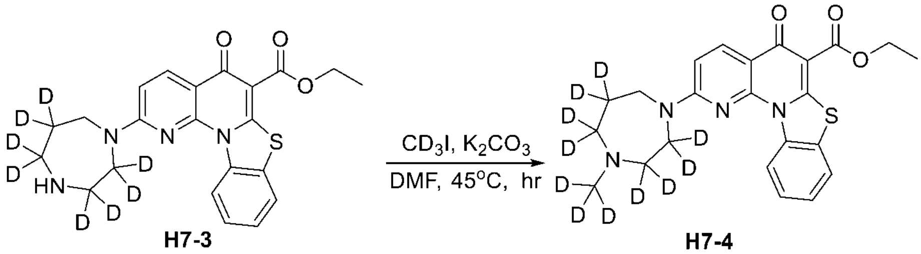


H7-3 H7-4

Potassium carbonate (352.0 mg, 2.6 mmol, 5.0 eq.) and iodomethane-$d_3$ (81.0 mg, 0.6 mmol, 1.1 eq.) were added to a solution of Compound **H7-3** (200.0 mg, 0.5 mmol, 1.0 eq.) in acetonitrile (10 mL). The mixture was stirred at 50°C for 2 hours. The reaction mixture was cooled to room temperature, and the aqueous layer was extracted with dichloromethane, and concentrated to obtain Compound **H7-4** (60 mg, a yellow solid, with a yield of 23.0%). MS-ESI: m/z 448 $[M+1]^+$, $^1H$ NMR (400 MHz, DMSO-$d_6$) δ: 9.40-9.30 (m, 1H), 8.37 (d, 1H), 7.90-7.80 (m, 1H), 7.60-7.40 (m, 2H), 6.94 (d, 1H), 4.28 (q, 2H), 3.85-3.55 (m, 2H), 1.30 (t, 3H).

9. Preparation of Compound **H7-5**

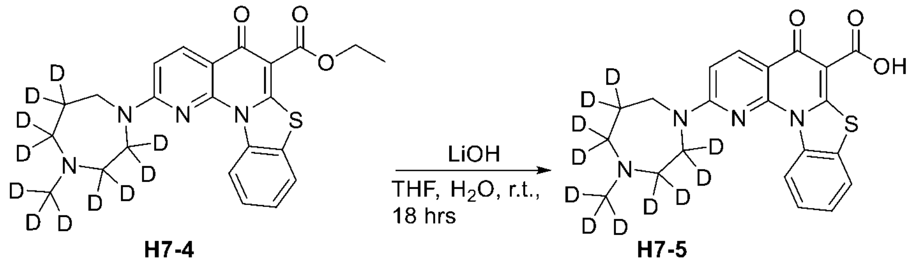


H7-4 H7-5

Compound **H7-4** (60.0 mg, 0.1 mmol, 1.0 eq.) was dissolved in tetrahydrofuran/water (1:1, v/v, 4 mL), and lithium hydroxide (10.0 mg, 0.4 mmol, 3.0 eq.) was added. The mixture was stirred at room temperature for 18 hours. After concentration, Compound **H7-5** was obtained, and this crude product was directly used in the next reaction (70 mg, a yellow solid, with a yield of 100.0%). MS-ESI: m/z 420 $[M+1]^+$, $^1H$ NMR (400 MHz, DMSO-$d_6$) δ: 9.40-9.30 (m, 1H), 8.23 (d, 1H), 8.00-7.90 (m, 1H), 7.55-7.45 (m, 1H), 7.45-7.35 (m, 1H), 6.99 (d, 1H), 3.90-3.60 (m, 2H).

10. Preparation of Compound **H-7**

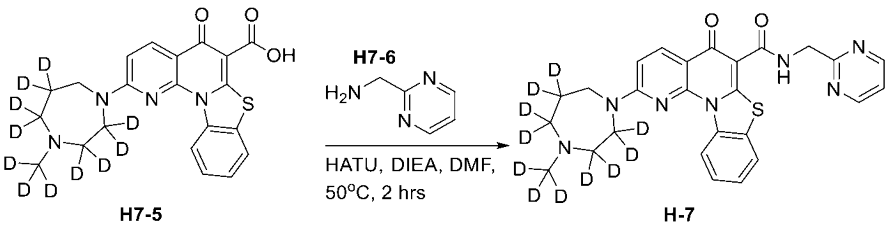


H7-5 H-7

2-(7-Azabenzotriazol-1-yl)-N,N,N',N'-tetramethyluronium hexafluorophosphate (41 mg, 0.107 mmol, 1.5 eq.) and N,N-diisopropylethylamine (28 mg, 0.215 mmol, 3.0 eq.) were added to a solution of compound **H7-5** (30 mg, 0.072 mmol, 1.0 eq.) in N,N-dimethylformamide (2 mL). The mixture was stirred at 50°C for 0.5 hour under nitrogen protection. Then, 2-methylaminopyrimidine compound **H7-6** (12 mg, 0.107 mmol, 1.5 eq.) dissolved in N,N-dimethylformamide (1 mL) was added, and the mixture was stirred at 50°C for 16 hours under nitrogen protection. The reaction mixture was spin dried to obtain a residue, which was purified by preparative HPLC. The eluate was lyophilized to obtain Compound **H-7** (11 mg, a yellow solid, with a yield of 30%). LC-MS (ESI): m/z 511.2 $[M+1]^+$, $^1H$ NMR (400 MHz, DMSO-$d_6$) δ: 11.25-11.10 (m, 1H), 9.50-9.35 (m, 1H), 8.88 (s, 2H), 8.40-8.30 (m, 1H), 8.05-7.90 (m, 1H), 7.60-7.35 (m, 2H), 7.10-6.95 (m, 1H), 4.85-4.70 (m, 2H), 3.95-3.70 (m, 2H).

Example 7: Preparation of Compound H-8

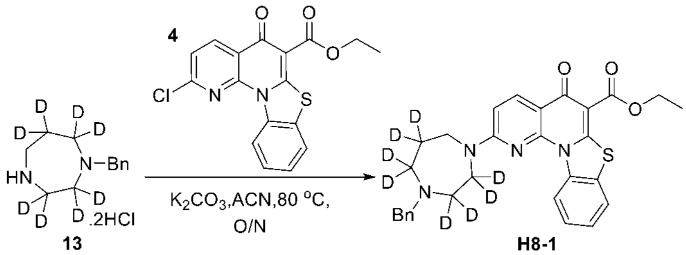


13 H8-1

Potassium carbonate (4.3 g, 31.3 mmol, 5.0 eq.) was added to a solution of Compound **4** (1.7 g, 6.27 mmol, 1.0 eq.) in acetonitrile (50 mL). Then Compound **13** (2.5 g, 6.89 mmol, 1.1 eq.) was added, and the mixture was stirred at 80°C for 16 hours under nitrogen protection. Then, the reaction mixture was cooled to room temperature, and washed and extracted with dichloromethane/methanol (10/1, v/v), followed by drying and spin drying to obtain a residue. The residue was purified by silica gel column chromatography to obtain Compound **H8-1** (2.8 g, a light yellow solid, with a yield of 72%). LC-MS (ESI): m/z 521.2 $[M+1]^+$, 1H NMR (400 MHz, DMSO-d6) δ: 9.50 - 9.25 (m, 1H), 8.23 (d, 1H), 8.00-7.90 (m, 1H), 7.60-7.35 (m, 2H), 7.30-7.10 (m, 5H), 7.00-6.90 (m, 1H), 4.29 (q, 2H), 4.00 -3.50 (m, 4H), 1.29 (t, 3H).

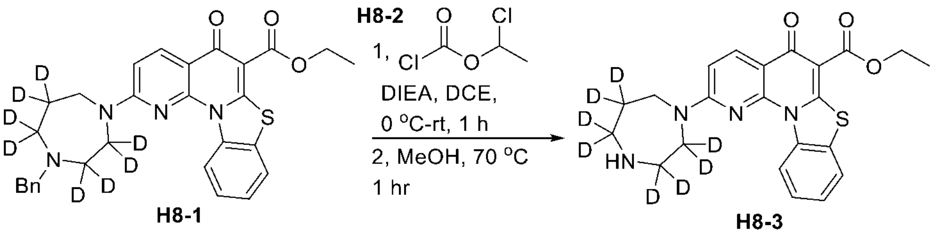


H8-1 H8-3

N,N-diisopropylethylamine (1.1 g, 8.64 mmol, 2.5 eq.) was added to a solution of Compound **H8-1** (1.8 g, 3.46 mmol, 1.0 eq.) in 1,2-dichloroethane (30 mL). Then Compound **H8-2** (0.99 g, 6.91 mmol, 2.0 eq.) was added at 0°C, and the mixture was stirred at room temperature for 1 hour under nitrogen protection. The reaction mixture was spin dried and dissolved in methanol (30 mL). The mixture was stirred at 70°C for 1 hour under nitrogen protection. The reaction mixture was spin dried, dichloromethane/ethyl acetate (10 mL/10 mL) was added, and the mixture was stirred at room temperature overnight. Then filtration was performed, and the filter cake was washed with dichloromethane, and dried to obtain a crude product of Compound **H8-3** (1.64 g, a yellow solid). LC-MS (ESI): m/z 431.0 $[M+1]^+$.

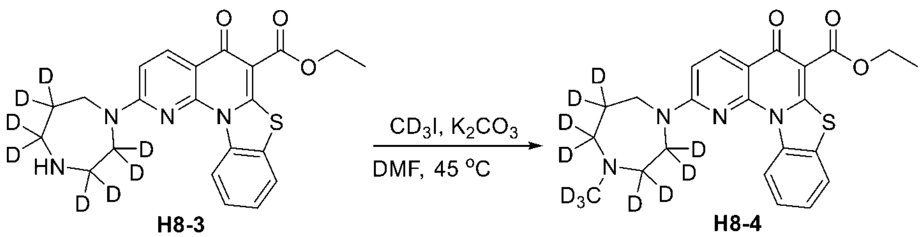


H8-3 H8-4

Potassium carbonate (0.58 g, 4.17 mmol, 3.0 eq.) was added to a solution of Compound **H8-3** (0.60 g, 1.39 mmol, 1.0 eq.) in N,N-dimethylformamide (10 mL). The mixture was stirred at 45°C for 0.5 hour. Then iodomethane-d3 (0.2 g, 1.39 mmol, 1.0 eq.) was added, and the mixture was stirred at 45°C for 0.5 hour under nitrogen protection. The reaction was then quenched by adding water, and the aqueous phase was extracted with dichloromethane/methanol (5/1, v/v, 150 mL × 5). The combined organic layers were concentrated to obtain a residue. The residue was purified by silica gel column chromatography to obtain Compound **H8-4** (the material was recovered and re-subjected to the reaction, and two rounds of reaction were conducted in total) (0.30 g, a light yellow solid, with a two-step yield of 40%). LC-MS (ESI): m/z 448.2 $[M+1]^+$, $^1H$ NMR (400 MHz, DMSO-$d_6$) δ: 9.50-9.35 (m, 1H), 8.23 (d, 1H), 7.95-7.85 (m, 1H), 7.60-7.35 (m, 2H), 7.00-6.90 (m, 1H), 4.28 (q, 2H), 4.00-3.60 (m, 2H), 1.29 (t, 3H).

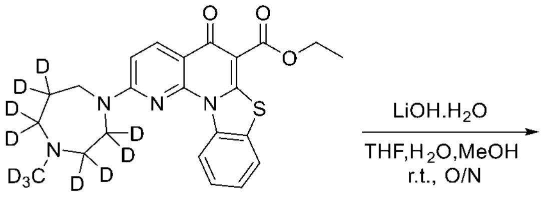

H8-4 H8-5

LiOH·$H_2O$ (0.17 g, 4.02 mmol, 2.5 eq.) was added to a mixed solution of Compound **H8-4** (0.72 g, 1.61 mmol, 1.0 eq.) in tetrahydrofuran (8 mL), methanol (4 mL), and water (8 mL). The reaction mixture was stirred at room temperature for 16 hours under nitrogen protection. The reaction mixture was then concentrated to obtain a residue, which was washed with water, and filtered. The filter cake was dried to obtain Compound **H8-5** (0.78 g, a light yellow solid), and this crude product was directly used in the next step. LC-MS (ESI): m/z 419.8 $[M+1]^+$, $^1H$ NMR (400 MHz, DMSO-$d_6$) $\delta$: 9.50-9.35 (m, 1H), 8.38 (d, 1H), 7.95-7.85 (m, 1H), 7.60-7.35 (m, 2H), 7.10-6.95 (m, 1H), 4.00-3.60 (m, 2H).

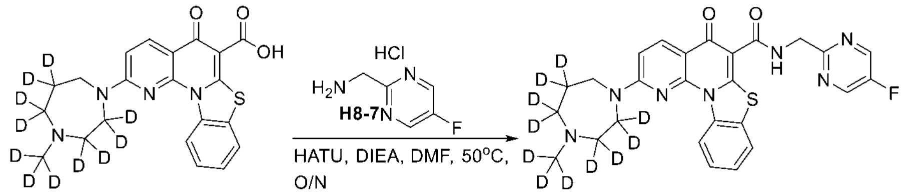

H8-6 H-8

2-(7-Azabenzotriazol-1-yl)-N,N,N',N'-tetramethyluronium hexafluorophosphate (0.13 g, 0.34 mmol, 1.5 eq.) and N,N-diisopropylethylamine (0.12 mL, 0.68 mmol, 3.0 eq.) were added to a solution of Compound **H8-6** (0.095 g, 0.27 mmol, 1.0 eq.) in N,N-dimethylformamide (2 mL). The mixture was stirred at 50°C for 0.5 hours under nitrogen protection. Then Compound **H8-7** (0.056 g, 0.34 mmol, 1.5 eq.) dissolved in N,N-dimethylformamide (1 mL) was added, and the mixture was stirred at 50°C for 16 hours under nitrogen protection. The reaction mixture was spin dried to obtain a residue. The residue was purified by preparative HPLC, and the eluate was lyophilized to obtain Compound **H-8** (0.012 g, a light yellow solid, with a yield of 10%). LC-MS (ESI): m/z 529.2 $[M+1]^+$, $^1H$ NMR (400 MHz, DMSO-$d_6$) $\delta$: 11.25-11.10 (m, 1H), 9.50-9.35 (m, 1H), 8.88 (s, 2H), 8.40-8.30 (m, 1H), 8.05-7.90 (m, 1H), 7.60-7.35 (m, 2H), 7.10-6.95 (m, 1H), 4.85-4.70 (m, 2H), 3.95-3.70 (m, 2H).

Example 8: Preparation of Compound H-11

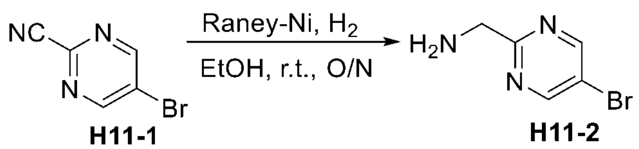

**H11-1** **H11-2**

Raney-Ni (0.08 g) and $NH_3$. $H_2O$ (0.2 mL) were added to a solution of Compound **H11-1** (0.5 g, 2.72 mmol) in EtOH (20 mL). The mixture was stirred at room temperature for 2 hours under a hydrogen atmosphere (3.5 MPa). Filtration was performed, and the filtrate was spin dried to obtain a residue. This residue was directly used in the next step without further purification. LC-MS (ESI): m/z 188.0 $[M+1]^+$.

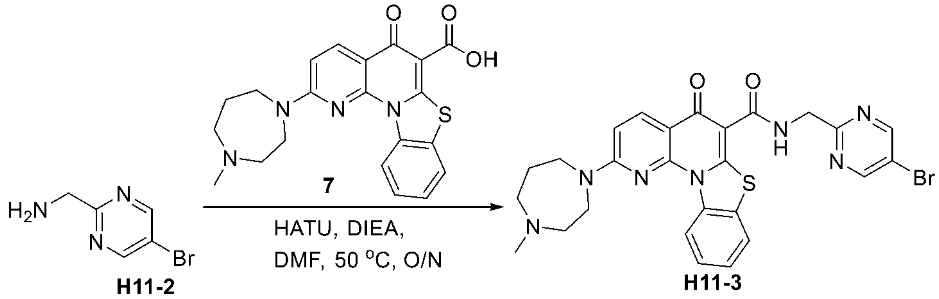

**H11-2** **H11-3**

2-(7-Azabenzotriazol-1-yl)-N,N,N',N'-tetramethyluronium hexafluorophosphate (0.23 g, 0.6 mmol, 1.5 eq.) and N,N-diisopropylethylamine (0.16 g, 1.2 mmol, 3.0 eq.) were added to a solution of Compound 7 (0.16 g, 0.4 mmol, 1.0 eq.) in N,N-dimethylformamide (5 mL). The mixture was stirred at 50°C for 0.5 hour under nitrogen protection. Then, Compound **H11-2** (0.3 g, 1.6 mmol, 4.0 eq.) dissolved in N,N-dimethylformamide (2 mL) was added, and the mixture was stirred at 50°C for 16 hours under nitrogen protection. The reaction mixture was spin dried to obtain a residue. The residue was slurried with methanol, followed by filtration, and the filter cake was washed and dried to obtain Compound **H11-3** (0.2 g, a white solid, with a yield of 56%). LC-MS: m/z 578.00, 580.00 $[M+1]^+$, $^1H$ NMR (400 MHz, DMSO-$d_6$) δ: 11.15-11.00 (m, 1H), 9.35-9.15 (m, 1H), 8.98 (s, 2H), 8.45-8.30 (m, 1H), 8.05-7.90 (m, 1H), 7.65-7.30 (m, 2H), 7.10-6.95 (m, 1H), 4.85-4.65 (m, 2H), 4.20-3.70 (m, 4H), 3.60-3.40 (m, 2H), 3.10 -2.60 (m, 5H), 2.30-2.15 (m, 2H).

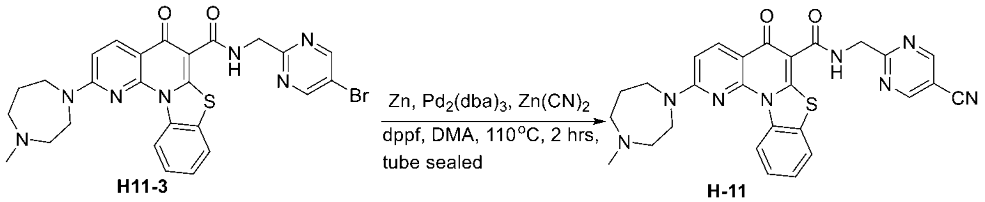


H11-3 H-11

Zinc cyanide (0.037 g, 0.31 mmol, 1.5 eq.), zinc (0.007 g, 0.104 mmol, 0.5 eq.), tris(dibenzylideneacetone) dipalladium (0.02 g, 0.021 mmol, 0.1 eq.), and 1,1'-bis(diphenylphosphino)ferrocene (0.023 g, 0.04 mmol, 0.2 eq.) were added to a solution of Compound **H11-3** (0.12 g, 0.21 mmol, 1.0 eq.) in DMA (6 mL). The mixture reacted in a sealed tube at 110°C for 2 hours under nitrogen protection. The reaction mixture was concentrated, and the residue was slurried with methanol. The filter residue was purified by preparative HPLC, and the eluate was lyophilized to obtain Compound **H-11** (0.0199 g, a light yellow solid, with a yield of 19%). LC-MS (ESI): m/z 525.2 $[M+1]^+$, $^1H$ NMR (400 MHz, DMSO-$d_6$) δ: 11.15-11.05 (m, 1H), 9.29 (s, 2H), 9.25-9.15 (m, 1H), 8.27 (d, 1H), 7.95-7.85 (m, 1H), 7.55-7.30 (m, 2H), 7.05-6.85 (m, 1H), 4.87 (d, 2H), 4.05-3.85 (m, 2H), 3.85-3.60 (m, 2H), 3.15-2.85 (m, 4H), 2.37 (s, 3H), 2.15-2.05 (m, 2H).

Example 9: Preparation of Compound H-12

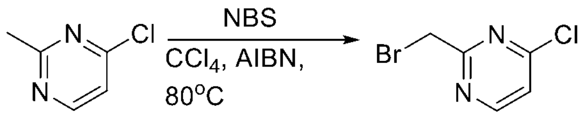


**H12-1** **H12-2**

Compound **H12-1** (2 g, 15.557 mmol, 1.0 eq.) was dissolved in carbon tetrachloride (16 mL), NBS (3.1 g, 17.113 mmol, 1.1 eq.) and AIBN (0.51 g, 3.111 mmol, 0.2 eq.) were added, and the resulting mixture was stirred at 80°C overnight. Ethyl acetate and water were added for washing and extraction, and the organic phase was dried and concentrated, and the resulting residue was purified by column chromatography to obtain Compound **H12-2** (500 mg, a yellow solid, with a yield of 15%). $^1H$ NMR (400 MHz, DMSO-$d_6$) δ: 8.79 (d, 1H), 7.64 (d, 1H), 4.64 (s, 2H).

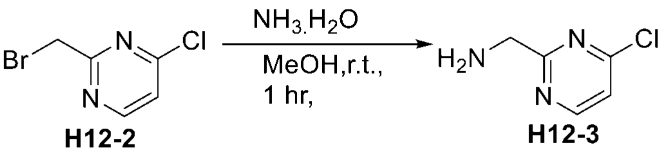


**H12-2** **H12-3**

$NH_3 \cdot H_2O$ (14 mL) was added to a solution of Compound **H12-2** (500 mg, 2.410 mmol, 1.0 eq.) in methanol (7 mL), and the resulting mixture was stirred at room temperature for 1 hour. Dichloromethane/methanol (10/1, v/v) was added for washing and extraction, and the organic phase was dried and concentrated, and the resulting residue was purified by column chromatography to obtain Compound **H12-3** (450 mg, a yellow solid, with a yield of 90%). $^1H$ NMR (400 MHz, DMSO-$d_6$) δ: 8.85 (d, 1H), 8.42 (s, 2H), 7.75 (d, 1H), 4.30 (s, 2H).

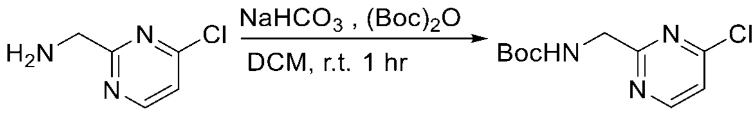


H12-3 H12-4

A saturated aqueous solution of sodium bicarbonate (3.2 mL) and di-tert-butyl dicarbonate (396 mg, 1.811 mmol, 1.3 eq.) were added to a solution of Compound **H12-3** (200 mg, 1.393 mmol, 1.0 eq.) in dichloromethane (4 mL). The reaction mixture was stirred at room temperature for 1 hour under a nitrogen atmosphere. Ethyl acetate and water were added for extraction, and the organic phase was dried and concentrated, and the resulting residue was purified by column chromatography to obtain Compound H12-4 (150 mg, a white solid, with a yield of 44%).

LC-MS (ESI): m/z 244.7 $[M+1]^+$.

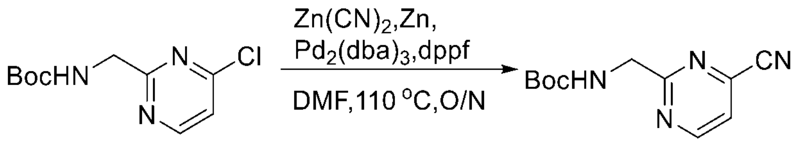


H12-4 H12-5

Zinc cyanide (51 mg, 0.431 mmol, 0.7 eq.) and zinc (16 mg, 0.246 mmol, 0.4 eq.) were added to a solution of Compound **H12-4** (150 mg, 0.615 mmol, 1.0 eq.) in N,N-dimethylformamide (5 mL), then 1,1'-bis(diphenylphosphino) ferrocene (21 mg, 0.036 mmol, 0.06 eq.) was added, and finally tris(dibenzylideneacetone)dipalladium (17 mg, 0.018 mmol, 0.03 eq.) was added. The mixture was stirred at 110°C overnight under nitrogen protection. Ethyl acetate and water were added for washing and extraction, and the organic phase was dried and concentrated, and the resulting residue was purified by column chromatography to obtain Compound **H12-5** (70 mg, a light yellow solid, with a yield of 49%). LC-MS (ESI):135.1 $[M+1]^+$ (without Boc group), $^1H$ NMR (400 MHz, DMSO-$d_6$) δ: 9.07 (d1H), 7.98 (d, 1H), 7.38 (s, 1H), 4.34 (d, 2H), 1.36 (s, 9H).

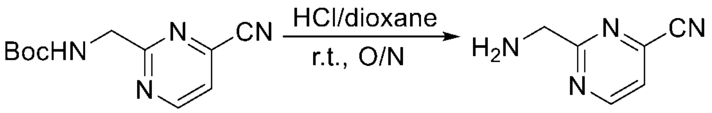


H12-5 H12-6

Compound **H12-5** (70 mg, 0.299 mmol, 1.0 eq.) was dissolved in HCl/dioxane (3 mL), and the solution was stirred at room temperature overnight. Concentration was performed to remove the solvent and obtain a crude product of Compound **H12-6** (50 mg, a yellow solid).

LC-MS (ESI): m/z 135.1 $[M+1]^+$,$^1H$ NMR (400 MHz, DMSO-$d_6$) δ: 9.07 (d, 1H), 8.62 (s, 2H), 7.93 (d, 1H), 4.37-4.34 (m, 2H).

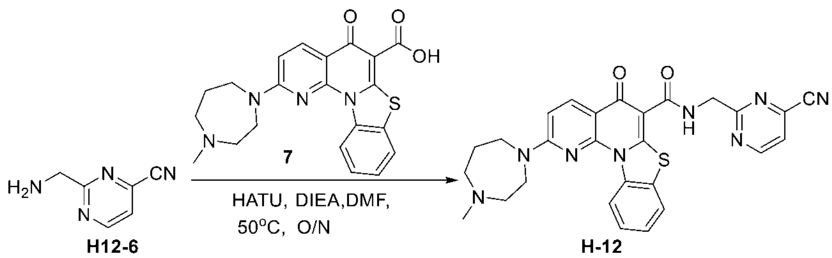


H12-6 H-12

2-(7-Azabenzotriazol-1-yl)-N,N,N',N'-tetramethyluronium hexafluorophosphate (155 mg, 0.410 mmol, 1.5 eq.) and N,N-diisopropylethylamine (105 mg, 0.808 mmol, 3.0 eq.) were added to a solution of Compound **H12-6** (110 mg, 0.273 mmol, 1.0 eq.) in N,N-dimethylformamide (3 mL). The mixture was stirred at 50°C for 0.5 hour under nitrogen protection. Then, Compound 7 (55 mg, 0.410 mmol, 1.5 eq.) dissolved in N,N-dimethylformamide (1 mL) was added to the mixture, and the resulting mixture was stirred at 50°C for 16 hours under nitrogen protection. The reaction mixture was spin dried to obtain a residue. The residue was purified by preparative HPLC to obtain Compound H-12 (12.9 mg, a light yellow solid, with a two-step yield of 10%). LC-MS (ESI): m/z 525.2 $[M+1]^+$, $^1H$ NMR (400 MHz, DMSO-$d_6$) δ: 11.20-11.10 (m, 1H), 9.45-9.35 (m, 1H), 9.13 (d, 1H), 8.45-8.35 (m, 1H), 8.30-8.20 (m, 1H), 8.10-7.95 (m, 2H), 7.60-7.40 (m, 2H), 7.10-7.00 (m, 1H), 4.90-4.80 (m, 2H), 3.80-3.40 (m, 4H), 2.80-2.65 (m, 1H), 2.60-2.50 (m, 2H), 2.25 (s, 3H), 2.05-1.90 (m, 2H).

Example 10: Preparation of Compound H-13

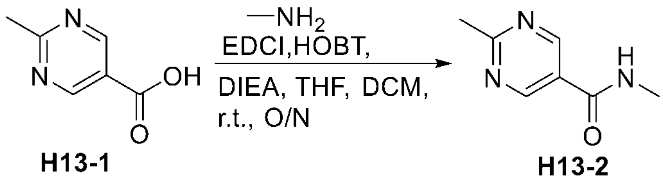


**H13-1** **H13-2**

Compound **H13-1** (4.67 g, 33.81 mmol, 1.0 eq.) was dissolved in a mixed solution of tetrahydrofuran (70 mL) and dichloromethane (70 mL). EDCI (9.72 g, 50.713 mmol, 1.5 eq.), HOBT (6.85 g, 50.713 mmol, 1.5 eq.), and N,N-diisopropylethylamine (13.10 g, 101.426 mmol, 3.0 eq.) were added to the solution, and finally methylamine hydrochloride (2.1 g, 67.617 mmol, 2.0 eq.) was added. The mixture was stirred at room temperature overnight. The reaction mixture was concentrated, and purified by column chromatography to obtain Compound **H13-2** (1.31 g, a light yellow solid, with a yield of 25%).

LC-MS (ESI): m/z 152.2 $[M+1]^+$, $^1$H NMR (400 MHz, DMSO-$d_6$) δ: 9.00 (s, 2H), 8.70 (d, 1H), 2.77 (d, 3H), 2.63 (s, 3H).

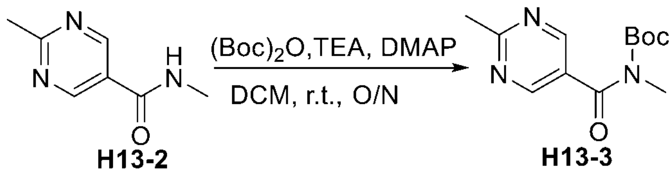


**H13-2** **H13-3**

4-Dimethylaminopyridine (0.529 g, 4.333 mmol, 0.5 eq.) and di-tert-butyl dicarbonate (3.782 g, 17.331 mmol, 2.0 eq.) were added to a solution of Compound **H13-2** (1.31 g, 8.666 mmol, 1.0 eq.) in dichloromethane (35 mL), and then triethylamine (4.384 g, 43.329 mmol, 5.0 eq.) was added. The mixture was stirred at room temperature overnight. Dichloromethane and water were added for washing and extraction. The organic phase was dried and concentrated, and the resulting residue was purified by column chromatography to obtain Compound **H13-3** (1.77 g, a light yellow solid, with a yield of 81%).

LC-MS (ESI): m/z 252.3 $[M+1]^+$, $^1$H NMR (400 MHz, DMSO-$d_6$) δ: 8.76 (s, 2H), 3.17 (s, 3H), 2.64 (s, 3H), 1.17 (s, 9H).

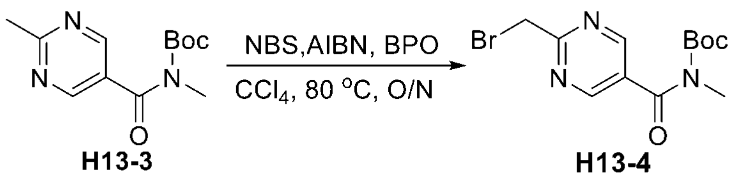


**H13-3** **H13-4**

N-bromosuccinimide (1.38 g, 7.748 mmol, 1.1 eq.), azobisisobutyronitrile (0.116 g, 0.7043 mmol, 0.1 eq.) and benzoyl peroxide (0.171 g, 0.7043 mmol, 0.1 eq.) were added to a solution of Compound **H13-3** (1.77 g, 7.043 mmol, 1.0 eq.) in carbon tetrachloride (50 mL). The reaction mixture was stirred at 80°C overnight under a nitrogen atmosphere. Dichloromethane/methanol (10/1, v/v) and water were added for washing and extraction. The organic phase was dried and concentrated, and the resulting residue was purified by column chromatography to obtain Compound **H13-4** (690 mg, a white oily substance, with a yield of 30%). LC-MS(ESI): m/z 331.2 $[M+1]^+$, $^1$H NMR (400 MHz, DMSO-$d_6$) δ: 8.91 (s, 1H), 4.71 (s, 2H), 3.18 (s, 3H), 1.16 (s, 9H).

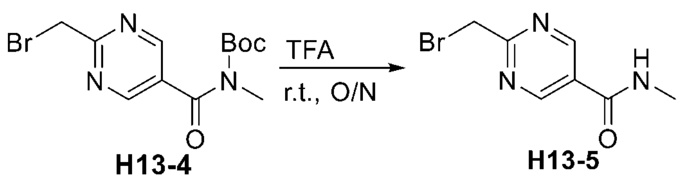


**H13-4** **H13-5**

Compound **H13-4** (690 mg, 2.09 mmol, 1.0 eq.) was dissolved in dichloromethane (5 mL), and trifluoroacetic acid

(2.38 g, 20.89 mmol, 10 eq.) was added. The mixture was stirred at room temperature overnight. The solvent was removed to obtain Compound **H13-5** (740 mg, a dark yellow solid), and the crude product was directly used in the next step. LC-MS (ESI): m/z 252.3 $[M+1]^+$, 1H NMR (400 MHz, DMSO-d6) δ: 9.11 (s, 2H), 4.70 (s, 2H), 2.78 (d, 3H).

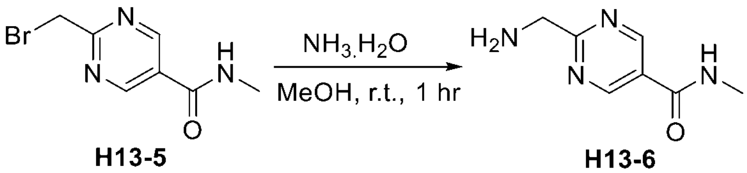


**H13-5** **H13-6**

$NH_3.H_2O$ (15 mL) was added to a solution of Compound **H13-5** (740 mg, 3.216 mmol, 1.0 eq.) in methanol (10 mL). The mixture was stirred at room temperature overnight. The solvent was removed, and the residue was lyophilized to obtain Compound **H13-6** (754 mg). The crude product was directly used in the next step. LC-MS (ESI): m/z 167.2 [M+1]+, $^1H$ NMR (400 MHz, DMSO-d6) δ: 9.18 (s, 2H), 8.87 (d, 1H), 4.34 (s, 2H), 2.80 (d, 3H).

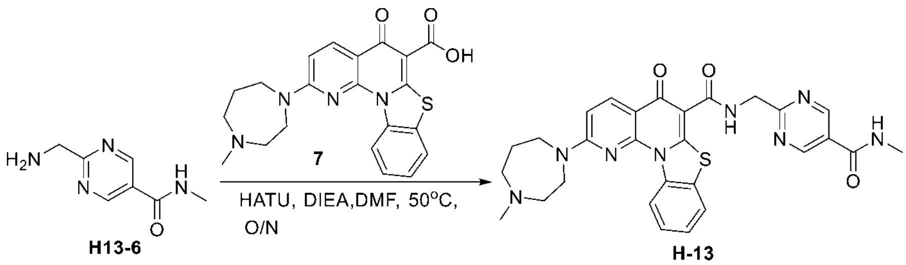


**H13-6** **H-13**

2-(7-Azabenzotriazol-1-yl)-N,N,N',N'-tetramethyluronium hexafluorophosphate (1.5 eq.) and N,N-diisopropylethylamine (4.0 eq.) were added to a solution of Compound 7 (754 mg, 1.0 eq.) in N,N-dimethylformamide (15 mL). The mixture was stirred at 50°C for 0.5 hour under nitrogen protection. Then, Compound **H13-6** (2.5 eq.) dissolved in N,N-dimethylformamide (5 mL) was added. The resulting mixture was stirred at 50°C overnight under nitrogen protection. The reaction mixture was spin dried to obtain a residue. The residue was slurried with methanol (8 mL) overnight, and filtered. The filter residue was collected and dried to obtain Compound **H-13** (212.3 mg, a light yellow solid, with a three-step yield of 21%). LC-MS (ESI): m/z 557.2 $[M+1]^+$, $^1H$ NMR (400 MHz, DMSO-$d_6$) δ: 11.25-11.15 (m, 1H), 9.45-9.35 (m, 1H), 9.11 (s, 2H), 8.80-8.75 (m, 1H), 8.33 (d, 1H), 8.00-7.90 (m, 1H), 7.60-7.35 (m, 2H), 7.44 (d, J = 7.6 Hz, 1H), 7.05-6.95 (m, 1H), 4.84 (d, 2H), 4.05-3.60 (m, 4H), 2.80 (d, 3H), 2.75-2.65 (m, 2H), 2.25 (s, 3H), 2.05-1.90 (m, 2H).

Example 11: Preparation of Compound H-15

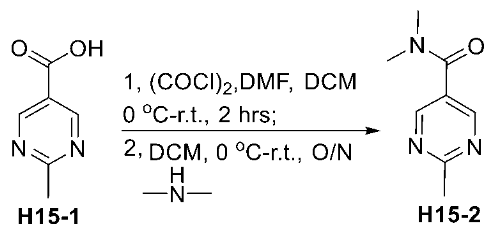


**H15-1** **H15-2**

Compound **H15-1** (2 g, 14.48 mmol, 1.0 eq.) was dissolved in a mixed solution of N,N-dimethylformamide (0.01 mL) and dichloromethane (80 mL). Oxalyl chloride (2.76 g, 21.72 mmol, 1.5 eq.) was added to the solution at 0°C. After the addition was completed, the resulting mixture was warmed to room temperature and stirred overnight. The reaction mixture was concentrated to obtain a crude product of Compound **H15-2** (1.5 g, a light yellow solid, with a yield of 60%), which was directly used in the next step.

LC-MS (ESI): m/z 166.2 $[M+1]^+$

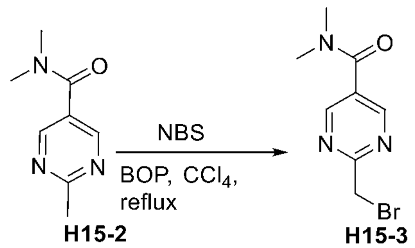


H15-2 H15-3

N-bromosuccinimide (1.75 g, 9.85 mmol, 1.1 eq.) and azobisisobutyronitrile (441 mg, 2.69 mmol, 0.3 eq.) were added to a solution of Compound **H15-2** (1.48 g, 8.96 mmol, 1.0 eq.) in carbon tetrachloride (10 mL). The mixture was stirred at 80°C overnight. Dichloromethane and water were added for washing and extraction. The organic phase was dried and concentrated, and the resulting residue was purified by column chromatography to obtain Compound **H15-3** (240 mg, a light yellow oily substance, with a yield of 10%).

LC-MS (ESI): m/z 245.1 $[M+1]^+$, $^1H$ NMR (400 MHz, DMSO-$d_6$) δ: 8.88 (s, 2H), 4.69 (s, 2H), 2.98 (s, 3H), 2.93 (s, 3H).

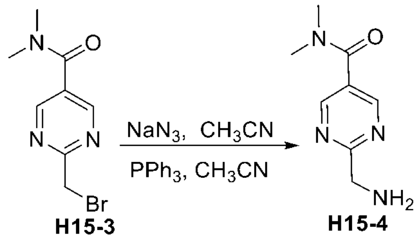


H15-3 H15-4

$NH_3.H_2O$ (6 mL) was added to a solution of Compound **H15-3** (200 mg, 0.82 mmol, 1.0 eq.) in methanol (3 mL). The mixture was stirred at room temperature for 2 hours. The reaction solvent was removed, and the residue was lyophilized to obtain Compound **H15-4** (180 mg, a yellow solid). The crude product was directly used in the next step. LC-MS (ESI): m/z 181.2 $[M+1]^+$, $^1H$ NMR (400 MHz, DMSO-$d_6$) δ: 8.96 (s, 2H), 4.34 (s, 2H), 2.99 (s, 3H), 2.93 (s, 3H).

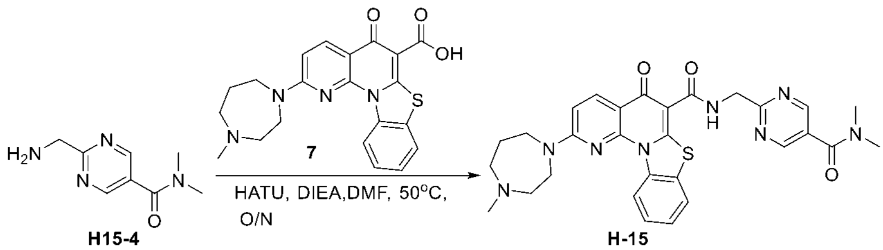


H15-4 H-15

2-(7-Azabenzotriazol-1-yl)-N,N,N',N'-tetramethyluronium hexafluorophosphate (148 mg, 0.39 mmol, 1.5 eq.) and N,N-diisopropylethylamine (101 mg, 0.78 mmol, 3.0 eq.) were added to a solution of Compound 7 (106 mg, 0.26 mmol, 1.0 eq.) in N,N-dimethylformamide (2 mL). The mixture was stirred at 50°C for 0.5 hour under nitrogen protection. Then, Compound **H15-4** (70 mg, 0.39 mmol, 1.5 eq.) dissolved in N,N-dimethylformamide (1 mL) was added, and the mixture was stirred at 50°C overnight under nitrogen protection. The reaction solvent was removed by concentration to obtain a residue. The residue was slurried with methanol (3 mL) overnight, and filtered, and the filter residue was collected and dried to obtain Compound **H-15** (19.9 mg, a white solid, with a two-step yield of 19%). LC-MS (ESI): m/z 571.4 $[M+1]^+$, $^1H$ NMR (400 MHz, DMSO-$d_6$) δ: 11.25-11.10 (m, 1H), 9.40-9.25 (m, 1H), 8.88 (s, 2H), 8.45-8.35 (m, 1H), 8.05-7.95 (m, 1H), 7.65-7.35 (m, 2H), 7.10-7.00 (m, 1H), 4.86 (d, 2H), 4.05-3.60 (m, 4H), 3.50-3.40 (m, 4H), 2.99 (s, 3H), 2.96 (s, 3H), 2.65-2.50 (m, 3H), 2.20-2.05 (m, 2H).

Example 12: Preparation of Compound H-16

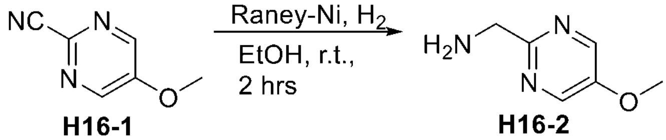


H16-1 H16-2

Raney-Ni (0.02 g) and $NH_3 \cdot H_2O$ (0.1 mL) were added to a solution of Compound **H16-1** (0.15 g, 1.11 mmol) in ethanol (6 mL). The mixture was stirred at room temperature for 2 hours under a hydrogen atmosphere (3.5 MPa). After filtration, the filtrate was spin dried to obtain a residue, which was directly used in the next step without further purification. LC-MS (ESI): m/z 140.0 $[M+1]^+$.

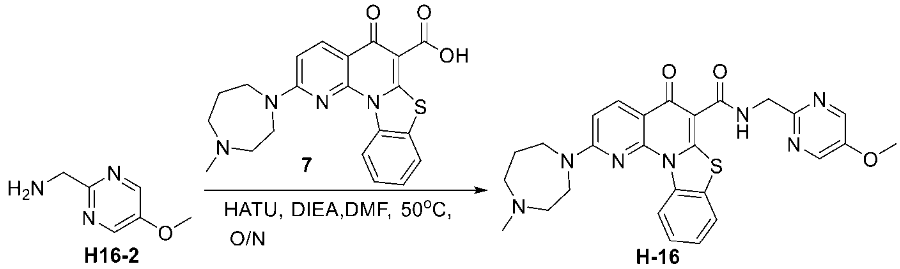


H16-2 H-16

2-(7-Azabenzotriazol-1-yl)-N,N,N',N'-tetramethyluronium hexafluorophosphate (103 mg, 0.27 mmol, 1.5 eq.) and N,N-diisopropylethylamine (0.1 mL, 0.54 mmol, 3.0 eq.) were added to a solution of Compound 7 (74 mg, 0.18 mmol, 1.0 eq.) in N,N-dimethylformamide (3 mL). The mixture was stirred at 50 °C for 0.5 hour under nitrogen protection. Then, a crude compound **H16-2** (100 mg, 0.72 mmol, 4.0 eq.) dissolved in N,N-dimethylformamide (1 mL) was added, and the mixture was stirred at 50 °C for 16 hours under nitrogen protection. The reaction mixture was spin dried to obtain a residue, which was purified by preparative HPLC to obtain Compound **H-16** (125 mg, a light yellow solid, with a two-step yield of 20%). LC-MS (ESI): m/z 530.2 $[M+1]^+$, $^1H$ NMR (400 MHz, DMSO-$d_6$) δ: 11.15-11.05 (m, 1H), 9.45-9.30 (m, 1H), 8.54 (s, 2H), 8.35-8.20 (m, 1H), 8.00-7.85 (m, 1H), 7.60-7.30 (m, 2H), 7.05-6.85 (m, 1H), 4.80-4.60 (m, 2H), 4.00-3.55 (m, 7H), 2.80-2.60 (m, 2H), 2.60-2.50 (m, 2H), 2.24 (s, 3H), 2.05-1.90 (m, 2H).

Example 13: Preparation of Compound H-17

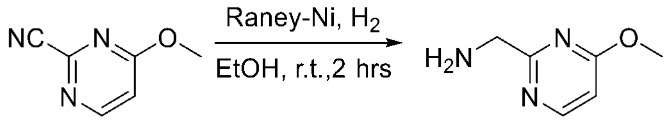


H17-1 H17-2

Compound **H17-1** (100 mg, 0.74 mmol, 1.0 eq.) was dissolved in ethanol (5 mL), and Raney-Ni (40 mg) and ammonia water (47%, 0.1 mL) were added. The mixture was stirred at room temperature for 2 hours under a hydrogen atmosphere (3 MPa). After filtration, lyophilization was performed to obtain Compound **H17-2** (40 mg, a red oily substance, with a yield of 38%). LC-MS (ESI): m/z 140.2 $[M+1]^+$, $^1H$ NMR (400 MHz, DMSO-$d_6$) δ: 8.42 (s, 1H), 6.74 (s, 1H), 3.87 (s, 3H), 3.72 (s, 2H).

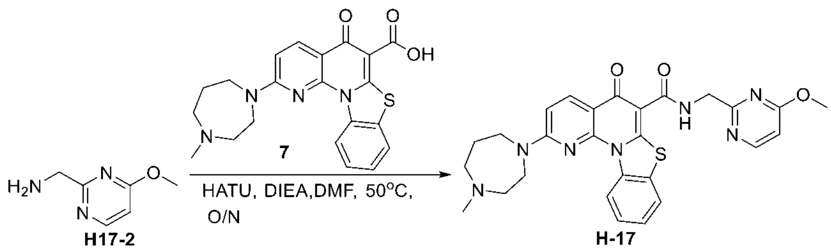


H17-2 H-17

Compound 7 (59 mg, 0.14 mmol, 1.0 eq.) was dissolved in N,N-dimethylformamide (2 mL), and 2-(7-azabenzotriazol-1-yl)-N,N,N',N'- tetramethyluronium hexafluorophosphate (84 mg, 0.22 mmol, 1.5 eq.) and N,N-diisopropylethylamine (57 mg, 0.44 mmol, 3.0 eq.) were added therein. The reaction mixture reacted at 50 °C for 0.5 hour under nitrogen

protection, then compound **H17-2** (40 mg, 0.28 mmol, 2.0 eq.) was added, and the reaction mixture was allowed to continue reacting at 50 °C for 16 hours. The reaction mixture was concentrated to remove N,N-dimethylformamide, and then methanol (2 mL) was added. The reaction mixture was stirred at room temperature overnight. After filtration, the filter cake was washed with methanol, and purified by preparative HPLC to obtain Compound **H-17** (3.1 mg, a light yellow solid, with a yield of 3.9%). LC-MS (ESI): m/z 530.2 $[M+1]^+$, $^1$H NMR (400 MHz, DMSO-$d_6$) δ: 11.25-11.15 (m, 1H), 9.50-9.40 (m, 1H), 8.49 (d, 1H), 8.40-8.30 (m, 2H), 7.98 (d, 1H), 7.60-7.40 (m, 2H), 7.10-6.96 (m, 1H), 6.83 (d, 1H), 4.67 (d, 2H), 4.05-3.60 (m, 7H), 2.80-2.65 (m, 2H), 2.55-2.50 (m, 2H), 2.26 (s, 3H), 2.05-1.90 (m, 2H).

Example 14: Preparation of Compound H-18

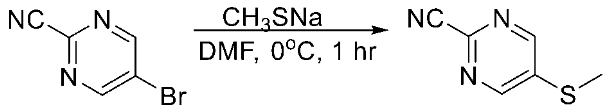


**H18-1** **H18-2**

Compound **H18-1** (100 mg, 0.544 mmol, 1.0 eq.) was dissolved in N,N-dimethylformamide (2 mL), and $CH_3SNa$ (229 mg, 0.652 mmol, 1.2 eq.) was added. The mixture was stirred at 0 °C for 1 hour. Ethyl acetate and water were added for extraction, and the organic phase was dried, concentrated, and then purified by column chromatography to obtain Compound **H18-2** (77 mg, a light yellow solid, with a yield of 93%). $^1$H NMR (400 MHz, DMSO-d6) δ: 8.86 (s, 2H), 2.63 (s, 3H).

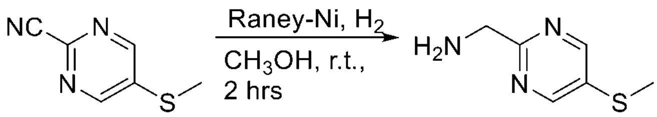


**H18-2** **H18-3**

$NH_3.H_2O$ (194 mg, 5.534 mmol, 11 eq.) and Raney-Ni (18 mg, 0.307 mmol, 0.6 eq.) were added to a solution of Compound **H18-2** (77 mg, 0.509 mmol, 1.0 eq.) in ethanol (3 mL). The mixture was stirred overnight at room temperature under a hydrogen atmosphere. Filtration was performed with diatomaceous earth, the filtrate was concentrated, and then water was added for lyophilization to obtain Compound **H18-3** (61 mg, a light yellow solid, with a yield of 34%). LC-MS (ESI): m/z 156.0 $[M+1]^+$.

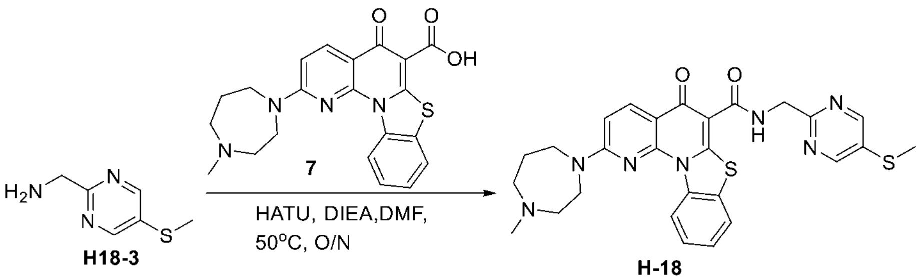


**H18-3** **7** **H-18**

2-(7-Azabenzotriazol-1-yl)-N,N,N',N'-tetramethyluronium hexafluorophosphate (185 mg, 0.485 mmol, 1.5 eq.) and N,N-diisopropylethylamine (126 mg, 0.966 mmol, 3.0 eq.) were added to a solution of Compound 7 (132 mg, 0.322 mmol, 1.0 eq.) in N,N-dimethylformamide (4 mL). The mixture was stirred at 50 °C for 0.5 hour under nitrogen protection. Then, Compound **H18-3** (150 mg, 0.966 mmol, 3.0 eq.) dissolved in N,N-dimethylformamide (1 mL) was added, and the mixture was stirred at 50 °C for 16 hours under nitrogen protection. The reaction mixture was spin dried to obtain a residue. Extraction was performed with ethyl acetate and water, and the organic phase was dried, concentrated, and then purified by preparative HPLC to finally obtain Compound **H-18** (21 mg, a light yellow solid, with a yield of 12%). LC-MS (ESI): m/z 546.1 $[M+1]^+$, $^1$H NMR (400 MHz, DMSO-$d_6$) δ: 11.15-11.05 (m, 1H), 9.40-9.30 (m, 1H), 8.75-8.65 (m, 2H), 8.45-8.35 (m, 1H), 8.05-7.90 (m, 1H), 7.65-7.40 (m, 2H), 7.10-7.00 (m, 1H), 4.80-4.70 (m, 2H), 4.25-3.95 (m, 2H), 3.90-3.70 (m, 2H), 3.60-3.45 (m, 4H), 2.79 (s, 3H), 2.58 (s, 3H), 2.40-2.25 (m, 2H).

Example 15: Preparation of Compound H-19

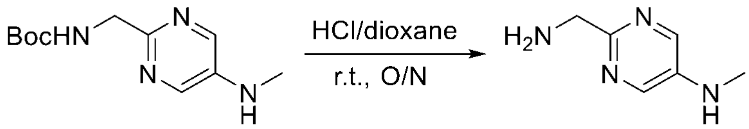


H19-1 H19-2

Compound **H19-1** (50 mg, 0.209 mmol, 1.0 eq.) was dissolved in HCl/dioxane (2 mL), and the mixture was stirred overnight at room temperature. The solvent was removed by concentration to obtain a crude product of Compound **H19-2** (35 mg, a light yellow solid). LC-MS (ESI): m/z 139.2 $[M+1]^+$, $^1H$ NMR (400 MHz, DMSO-$d_6$) δ: 8.37 (d, 3H), 8.11 (s, 2H), 4.03 (d, 2H), 2.70 (s, 3H).

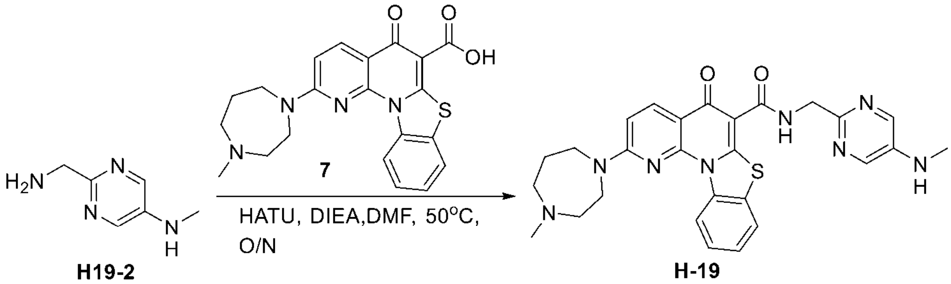


H19-2 H-19

2-(7-Azabenzotriazol-1-yl)-N,N,N',N'-tetramethyluronium hexafluorophosphate (98 mg, 0.253 mmol, 1.5 eq.) and N,N-diisopropylethylamine (67 mg, 0.514 mmol, 3.0 eq.) were added to a solution of Compound 7 (70 mg, 0.169 mmol, 1.0 eq.) in N,N-dimethylformamide (2 mL). The mixture was stirred at 50 °C for 0.5 hour under nitrogen protection. Then, compound **H19-2** (35 mg, 0.253 mmol, 1.5 eq.) dissolved in N,N-dimethylformamide (1 mL) was added, and the mixture was stirred at 50 °C for 16 hours under nitrogen protection. The solvent was removed by concentration, and purification were performed by preparative HPLC to finally obtain Compound **H-19** (6.1 mg, a light yellow solid, with a two-step yield of 7%). LC-MS (ESI): m/z 529.2 $[M+1]^+$, $^1H$ NMR (400 MHz, DMSO-$d_6$) δ: 11.00-10.90 (m, 1H), 9.25-9.10 (m, 1H), 8.30 -8.20 (m, 1H), 8.11 (s, 2H), 7.95-7.85 (m, 1H), 7.55-7.35 (m, 2H), 7.00-6.90 (m, 1H), 6.10-6.00 (m, 1H), 4.65-4.55 (m, 2H), 4.10-3.95 (m, 2H), 3.80-3.40 (m, 4H), 3.20-3.05 (m, 2H), 2.75-2.60 (m, 6H), 2.30-2.20 (m, 2H).

Example 16: Preparation of Compound H-20

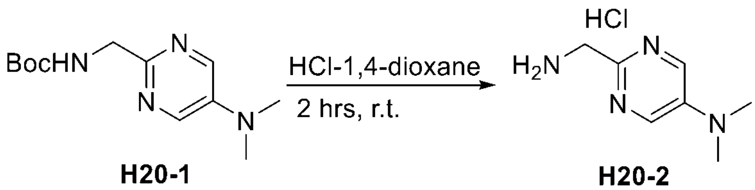


H20-1 H20-2

HCl-dioxane (2 mL) was added to Compound **H20-1** (0.03 g, 0.12 mmol). The mixture was stirred at room temperature for 2 hours under nitrogen protection. The solvent was removed by concentration to obtain Compound **H20-2** (25 mg, a yellow solid, with a yield of 97%). LC-MS (ESI): m/z 153.2 $[M+1]^+$.

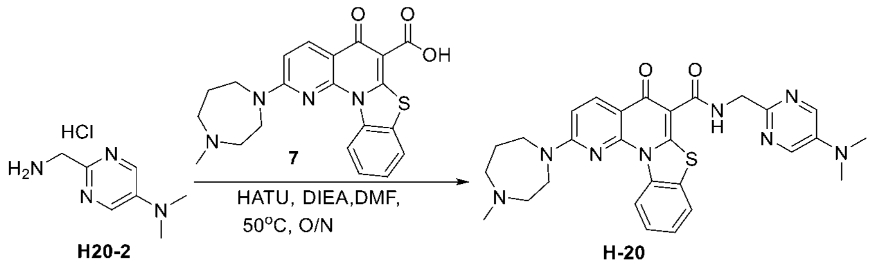


2-(7-Azabenzotriazol-1-yl)-N,N,N',N'-tetramethyluronium hexafluorophosphate (0.06 g, 0.16 mmol, 1.5 eq.) and N,N-diisopropylethylamine (0.075 mL, 0.42 mmol, 4.0 eq.) were added to a solution of Compound 7 (0.044 g, 0.11 mmol, 1.0 eq.) in N,N-dimethylformamide (2 mL). The mixture was stirred at 50 °C for 0.5 hour under nitrogen protection, then Compound **H20-2** (0.03 g, 0.16 mmol, 1.5 eq.) dissolved in N,N-dimethylformamide (1 mL) was added, and the mixture was stirred at 50 °C for 16 hours under nitrogen protection. The reaction mixture was concentrated to obtain a residue. The residue was purified by preparative HPLC, and the eluate was lyophilized to obtain Compound **H-20** (0.029 g, a light yellow solid, with a yield of 50%). LC-MS (ESI): m/z 543.2 $[M+1]^+$, $^1H$ NMR (400 MHz, DMSO-$d_6$) δ: 11.10-11.00 (m, 1H), 9.45-9.35 (m, 1H), 8.35-8.30 (m, 1H), 8.28 (s, 2H), 8.00-7.90 (m, 1H), 7.60-7.35 (m, 2H), 7.05-6.95 (m, 1H), 4.63 (d, 2H), 4.05-3.60 (m, 4H), 2.92 (s, 6H), 2.80-2.70 (m, 2H), 2.55-2.45 (m, 2H), 2.25 (s, 3H), 2.05-1.90 (m, 2H).

Example 17: Preparation of Compound H-21

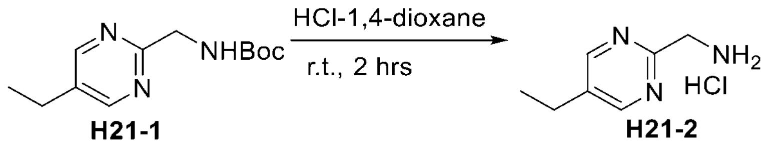


HCl-dioxane (4M, 3 mL) was added to Compound **H21-1** (0.05 g, 0.25 mmol). The mixture was stirred at room temperature for 2 hours under nitrogen protection. The solvent was removed by concentration to obtain Compound **H21-2** (30 mg, a gray solid, with a yield of 97%). LC-MS (ESI): m/z 138.4 $[M+1]^+$, $^1H$ NMR (400 MHz, DMSO-$d_6$) δ: 8.73 (s, 2H), 8.49 (s, 3H), 4.21 (q, 2H), 2.63 (q, 2H), 1.18 (t, 3H)

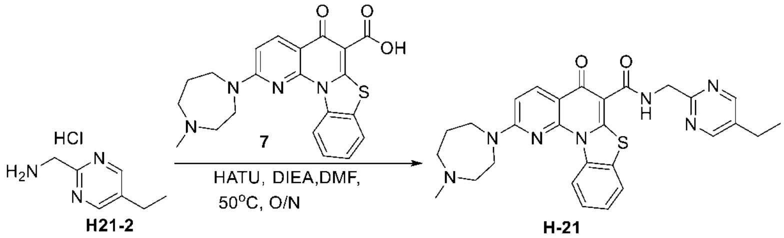


2-(7-Azabenzotriazol-1-yl)-N,N,N',N'-tetramethyluronium hexafluorophosphate (0.11 g, 0.29 mmol, 1.5 eq.) and N,N-diisopropylethylamine (0.14 mL, 0.77 mmol, 4.0 eq.) were added to a solution of Compound 7 (0.079 g, 0.19 mmol, 1.0 eq.) in N,N-dimethylformamide (3 mL). The mixture was stirred at 50 °C for 0.5 hour under nitrogen protection. Then, Compound **H21-2** (0.05 g, 0.29 mmol, 1.5 eq.) dissolved in N,N-dimethylformamide (1 mL) was added, and the mixture was stirred at 50 °C for 16 hours under nitrogen protection. The reaction mixture was concentrated to obtain a residue. The residue was extracted with dichloromethane/methanol (10:1, v/v) and water. The combined organic phases were dried, concentrated, then slurried with methanol, and filtered. The filter cake was dried to obtain Compound **H-21** (0.048 g, a light yellow solid, with a yield of 47%). LC-MS (ESI): m/z 528.2 $[M+1]^+$, $^1H$ NMR (400 MHz, DMSO-$d_6$) δ: 11.20-11.10 (m, 1H), 9.40-9.25 (m, 1H), 8.68 (s, 2H), 8.50-8.40 (m, 1H), 8.05-7.95 (m, 1H), 7.65-7.40 (m, 2H), 7.15-7.05 (m, 1H), 4.75 (d, 2H), 4.10-3.40 (m, 6H), 2.81 (s, 3H), 2.61 (q, 2H), 2.50-2.45 (m, 2H), 2.30-2.15 (m, 2H), 1.19 (t, 3H).

Example 18: Preparation of Compound H-22

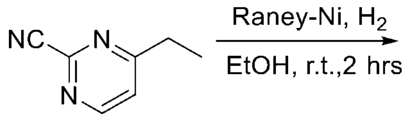


**H22-1** **H22-2**

Raney-Ni (0.08 g) and $NH_3 \cdot H_2O$ (0.2 mL) were added to a solution of Compound **H22-1** (0.56 g, 4.21 mmol) in ethanol (25 mL). The mixture was stirred at room temperature for 2 hours under a hydrogen atmosphere (3.5 MPa). After filtration, the filtrate was concentrated to obtain a residue, which was directly used in the next step without further purification. LC-MS (ESI): m/z 138.1 $[M+1]^+$.

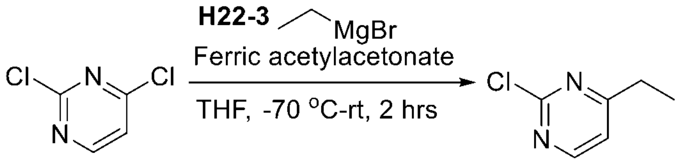


**H22-2** **H22-4**

Compound **H22-3** (2.1 g, 16.2 mmol, 1.2 eq.) was added dropwise to a mixture of Compound **H22-2** (2.0 g, 13.4 mmol, 1.0 eq.) and iron(III) acetylacetonate (0.95 g, 2.7 mmol, 0.2 eq.) maintained at -78 °C. The reaction mixture was stirred at -78 °C for 0.5 hour, then gradually warmed to room temperature and reacted at room temperature for 1 hour. The reaction mixture was quenched with a saturated ammonium chloride solution, and then extracted with ethyl acetate and water. The combined organic phases were dried and concentrated to obtain a residue. The residue was purified by column chromatography to obtain Compound **H22-4** (0.7 g, a light yellow oil, with a yield of 40%). LC-MS (ESI): m/z 143.1 $[M+1]^+$, $^1H$ NMR (400 MHz, $CDCl_3$) δ: 8.47 (d, 1H), 7.11 (d, 1H), 2.78 (q, 2H), 1.29 (t, 3H).

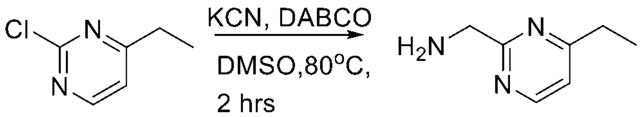


**H22-4** **H22-5**

Potassium cyanide (0.73 g, 11.22 mmol, 2.0 eq.) and 1,4-diazabicyclo[2.2.2]octane (1.26 g, 11.22 mmol, 2.0 eq.) were added to a solution of Compound **H22-4** (0.8 g, 5.61 mmol, 1.0 eq.) in dimethyl sulfoxide (48 mL) and water (10 mL). The reaction was carried out at 80 °C for 2 hours under nitrogen protection. Extraction was performed with ethyl acetate and water. The combined organic phases were dried and concentrated to obtain a residue. The residue was purified by column chromatography to obtain Compound **H22-5** (0.56 g, a light yellow oil, with a yield of 75%). LC-MS (ESI): m/z 134.1 $[M+1]^+$.

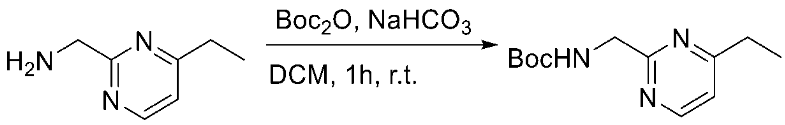


**H22-5** **H22-6**

Di-tert-butyl dicarbonate (0.1 g, 0.44 mmol, 1.2 eq.) and a saturated aqueous solution of sodium bicarbonate (1 mL) were added to a solution of Compound **H22-5** (0.15 g, 0.36 mmol, 1.0 eq.) in dichloromethane (2 mL). The mixture was stirred at room temperature for 1 hour under nitrogen protection. The reaction mixture was extracted with dichloromethane and water, and the organic phases were combined, dried, and concentrated to obtain a residue. The residue was purified by column chromatography to obtain Compound **H22-6** (0.03 g, a white solid, with a yield of 30%). LC-MS (ESI): m/z 238.2 $[M+1]^+$, $^1H$ NMR (400 MHz, $CDCl_3$) δ 8.51 (d, 1H), 7.01 (d, 1H), 5.80-5.65 (m, 1H), 4.51 (d, 2H), 2.74 (q, 2H), 1.43 (s, 9H), 1.26 (t, 3H).

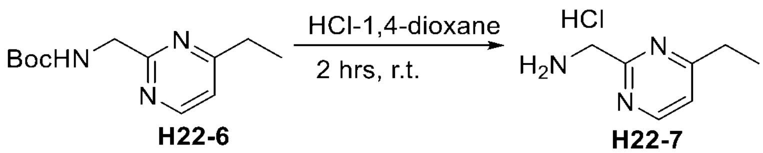


**H22-6** **H22-7**

HCl-dioxane (4M, 2 mL) was added to Compound **H22-6** (0.03 g, 0.13 mmol). The mixture was stirred at room temperature for 2 hours under nitrogen protection. The reaction solvent was removed by concentration to obtain Compound **H22-7** (25 mg, a white solid, with a yield of 98%). LC-MS (ESI): m/z 138.1 [M+1]$^+$.

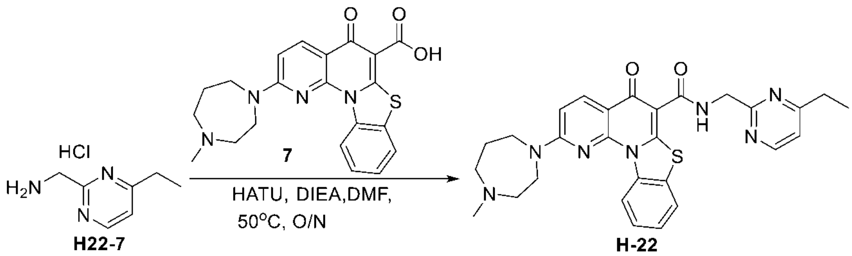


H22-7 H-22

2-(7-Azabenzotriazol-1-yl)-N,N,N',N'-tetramethyluronium hexafluorophosphate (0.055 g, 0.14 mmol, 1.5 eq.) and N,N-diisopropylethylamine (0.07 mL, 0.38 mmol, 4.0 eq.) were added to a solution of Compound 7 (0.04 g, 0.1 mmol, 1.0 eq.) in N,N-dimethylformamide (1 mL). The mixture was stirred at 50 °C for 0.5 hour under nitrogen protection. Compound **H22-7** (0.025 g, 0.14 mmol, 1.5 eq.) dissolved in N,N-dimethylformamide (2 mL) was added, and the mixture was stirred at 50 °C for 16 hours under nitrogen protection. The reaction mixture was concentrated to obtain a residue. The residue was purified by preparative HPLC, and the eluate was lyophilized to obtain Compound **H-22** (0.03 g, a light yellow solid, with a yield of 59%). LC-MS (ESI): m/z 528.2 [M+1]$^+$, $^1$H NMR (400 MHz, DMSO-$d_6$) δ: 11.20-11.10 (m, 1H), 9.45-9.30 (m, 1H), 8.70-8.60 (m, 1H), 8.35-8.25 (m, 1H), 8.00-7.90 (m, 1H), 7.55-7.35 (m, 2H), 7.29 (d, 1H), 7.05-6.90 (m, 1H), 4.73 (d, 2H), 4.05-3.60 (m, 6H), 2.80-2.70 (m, 2H), 2.55-2.45 (m, 2H), 2.24 (s, 3H), 2.55-1.90 (m, 2H), 1.24 (t, 3H).

Example 19: Preparation of Compound H-26

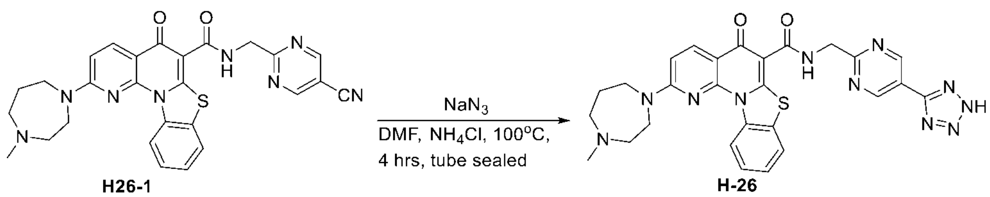


H26-1 H-26

Compound **H26-1** (0.11 g, 0.21 mmol, 1.0 eq.) was dissolved in N,N-dimethylformamide (6 mL), and sodium azide (0.082 g, 1.26 mmol, 6.0 eq.) and ammonium chloride (0.22 g, 4.2 mmol, 20.0 eq.) were added to the reaction mixture. The resulting mixture reacted at 100 °C for 4 hours in a sealed tube. Purification was performed by preparative HPLC, and the eluate was lyophilized to obtain Compound **H-26** (0.0076 g, a white solid, with a yield of 7%). LC-MS (ESI): m/z 568.2 [M+1]$^+$, $^1$H NMR (400 MHz, DMSO-$d_6$) δ: 11.15-11.05 (m, 1H), 9.40-9.30 (m, 1H), 9.23 (s, 2H), 8.45-8.35 (m, 1H), 8.00-7.90 (m, 1H), 7.60-7.40 (m, 2H), 7.10-7.00 (m, 1H), 4.81 (d, 2H), 4.05-3.60 (m, 4H), 3.05-2.95 (m, 2H), 2.65-2.45 (m, 5H), 2.20-2.05 (m, 2H).

Example 20: Preparation of Compound H-27

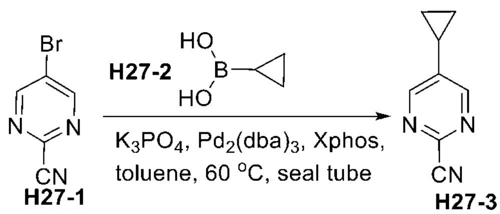


H27-1 H27-3

Compound **H27-1** (600 mg, 3.3 mmol, 1.0 eq.) was dissolved in toluene (12 mL), and tris(dibenzylideneacetone) dipalladium (589 mg, 0.65 mmol, 0.2 eq.), XPhos (620 mg, 1.3 mmol, 0.4 eq.), $K_3PO_4.3H_2O$ (2.6 g, 9.8 mmol, 3.0 eq.) and Compound **H27-2** (560 mg, 6.5 mmol, 2.0 eq.) were added. The mixture reacted in a sealed tube at 60 °C overnight.

Filtration was performed, and the filtrate was concentrated, and purified by column chromatography to obtain Compound **H27-3** (500 mg, a light yellow oil, with a purity of approximately 80%), which was directly used in the next step. LC-MS (ESI): m/z 146.2 $[M+1]^+$, $^1H$ NMR (400 MHz, DMSO-$d_6$) δ 8.75 (s, 2H), 2.03 (s, 1H), 1.15-1.11 (m, 2H), 0.97 (m, 2H).

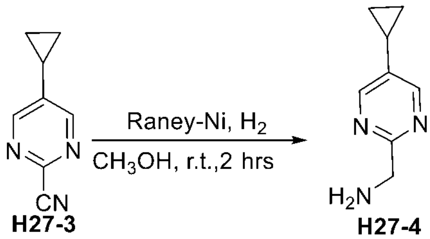


**H27-3** **H27-4**

Compound **H27-3** (200 mg, 1.4 mmol, 1.0 eq.) was dissolved in methanol (3 mL), and Raney-Ni (60 mg) and concentrated hydrochloric acid (0.2 mL) were added. The mixture was stirred overnight at room temperature under a hydrogen atmosphere, followed by filtration, and the resulting residue was purified by thin-layer chromatography to obtain Compound **H27-4** (35 mg, a light yellow oil, with a two-step yield of 18.0%). LC-MS (ESI): m/z 150.3 $[M+1]^+$, $^1H$ NMR (400 MHz, DMSO-$d_6$) δ 8.50 (s, 2H), 3.86 (s, 2H), 1.90 (m, 1H), 0.99 (m, 2H), 0.79-0.76 (m, 2H).

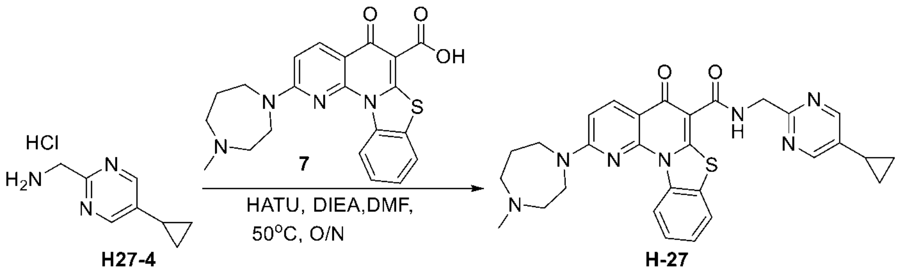


**H27-4** **H-27**

Compound 7 (48 mg, 0.12 mmol, 1.0 eq.) was dissolved in N,N-dimethylformamide (2 mL), and 2-(7-azabenzotriazol-1-yl)-N,N,N',N'- tetramethyluronium hexafluorophosphate (88 mg, 0.23 mmol, 2.0 eq.) and N,N-diisopropylethylamine (47 mg, 0.36 mmol, 3.0 eq.) were added therein. The reaction mixture reacted at 50 °C for 0.5 hour under nitrogen protection, then Compound **H27-4** (35 mg, 0.23 mmol, 2.0 eq.) was added, and the reaction mixture was allowed to continue reacting at 50 °C for 16 hours. The reaction mixture was concentrated to remove N,N-dimethylformamide, and methanol (2 mL) was added. The mixture was stirred at room temperature overnight. After filtration, the filter cake was washed with methanol and dried to obtain Compound **H-27** (5.0 mg, a light yellow solid, with a yield of 7.9%). LC-MS (ESI): m/z 540.1 $[M+1]^+$, $^1H$ NMR (400 MHz, DMSO-$d_6$) δ: 11.20-11.10 (m, 1H), 9.45-9.35 (m, 1H), 8.38 (s, 2H), 8.35-8.25 (m, 1H), 8.00-7.90 (m, 1H), 7.60-7.35 (m, 2H), 7.05-6.90 (m, 1H), 4.74 (d, 2H), 4.00-3.60 (m, 4H), 2.80-2.70 (m, 2H), 2.60-2.50 (m, 2H), 2.27 (s, 3H), 2.05-1.90 (m, 2H), 1.10-0.95 (m, 2H), 0.85-0.75 (m, 2H).

Example 21: Preparation of Compound H-28

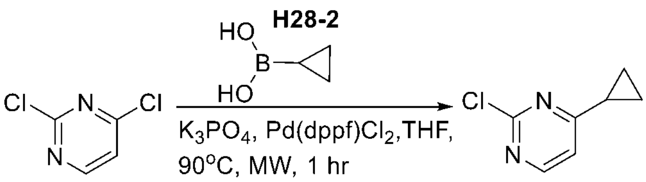


**H28-1** **H28-3**

Compound **H28-1** (500 mg, 3.4 mmol, 1.0 eq.) was dissolved in tetrahydrofuran (10 mL), and Pd(dppf)$Cl_2$ (246 mg, 0.34 mmol, 0.1 eq.), $K_3PO_4.3H_2O$ (2.2 g, 8.4 mmol, 2.5 eq.) and Compound **H28-2** (289 mg, 3.4 mmol, 1.0 eq.) were added. The mixture reacted under microwave at 90 °C for 1 hour. After filtration, the filtrate was concentrated and purified by column chromatography to obtain Compound **H28-3** (130 mg, a light yellow oily substance, with a yield of 25%). LC-MS (ESI): m/z 155.2 $[M+1]^+$, $^1H$ NMR (400 MHz, $CDCl_3$) δ: 8.33 (d, 1H), 7.06 (d, 1H), 1.96 (m, 1H), 1.22-1.11 (m, 4H).

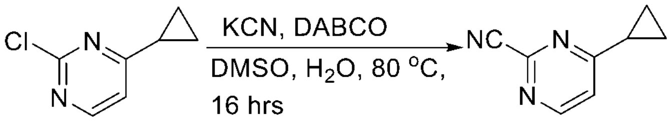


**H28-3** **H28-4**

Compound **H28-3** (130 mg, 0.84 mmol, 1.0 eq.) was dissolved in dimethyl sulfoxide (7 mL) and water (1 mL), and potassium cyanide (109 mg, 1.68 mmol, 2.0 eq.) and 1,4-diazabicyclo[2.2.2]octane (188 mg, 1.68 mmol, 2.0 eq.) were added. The reaction mixture reacted at 80 °C overnight. Water and ethyl acetate were added for extraction, and the organic phases were combined, dried, then concentrated, and purified by column chromatography to obtain Compound **H28-4** (80 mg, a light-yellow oily substance, with a yield of 65.6%). LC-MS (ESI): m/z 146.2 [M+1]+, $^1$H NMR (400 MHz, DMSO-d6) δ: 8.71 (d, 1H), 7.74 (d, 1H), 2.21 (s, 1H), 1.20-1.15 (m, 2H), 1.06 (m, 2H).

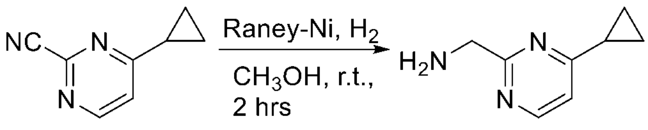


4 5

Compound **H28-4** (80 mg, 0.55 mmol, 1.0 eq.) was dissolved in methanol (3 mL), and Raney-Ni (20 mg) and ammonia water (47%) (0.1 mL) were added. The mixture was stirred at room temperature for 2 hours under a hydrogen atmosphere (3 MPa), followed by filtration and lyophilization to obtain Compound **H28-5** (43 mg, a light yellow oily substance, with a yield of 52.4%). LC-MS (ESI): m/z 150.3 [M+1]$^+$.

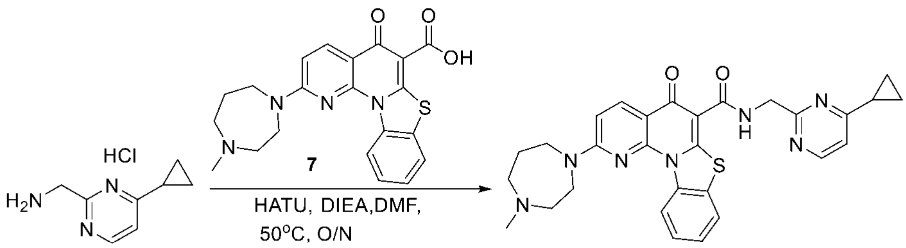


**H28-5** **H-28**

Compound 7 (36 mg, 0.09 mmol, 1.0 eq.) was dissolved in N,N-dimethylformamide (2 mL), and 2-(7-azabenzotriazol-1-yl)-N,N,N',N'- tetramethyluronium hexafluorophosphate (52 mg, 0.14 mmol, 1.5 eq.) and N,N-diisopropylethylamine (35 mg, 0.27 mmol, 3.0 eq.) were added therein. The reaction mixture reacted at 50 °C for 0.5 hour under nitrogen protection, then Compound **H28-5** (40 mg, 0.27 mmol, 3.0 eq.) was added, and the reaction mixture was allowed to continue reacting at 50 °C for 16 hours. The reaction mixture was concentrated to remove N,N-dimethylformamide. Methanol (2 mL) was added to the residue, and the resulting mixture was stirred at room temperature overnight. After filtration, the filter cake was washed with methanol and then purified by preparative HPLC to obtain Compound **H-28** (19.6 mg, a light yellow solid, with a yield of 40.8%). LC-MS (ESI): m/z 540.2 [M+1]$^+$, $^1$H NMR (400 MHz, DMSO-$d_6$) δ: 11.15-11.05 (m, 1H), 9.30-9.20 (m, 1H), 8.55-8.50 (m, 1H), 8.35-8.25 (m, 1H), 7.95-7.85 (m, 1H), 7.55-7.35 (m, 2H), 7.35-7.25 (m, 1H), 7.0-6.90 (m, 1H), 4.66 (d, 2H), 4.00-3.60 (m, 4H), 3.15-2.80 (m, 4H), 2.55 (s, 3H), 2.20-2.00 (m, 3H), 1.20-1.00 (m, 4H).

Example 22: Preparation of Compound H-31

1. Preparation of Compound **H31-2**

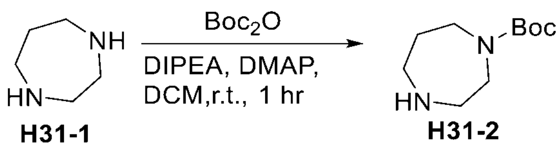


**H31-1** **H31-2**

Compound **H31-1** (1 g, 9.98 mmol, 1.0 eq.) was dissolved in dichloromethane (20 mL), N,N-diisopropylethylamine (1.29 g, 9.98 mmol, 1.0 eq.) and 4-dimethylaminopyridine (1.22 g, 9.98 mmol, 1.0 eq.) were added, and then di-tert-butyl dicarbonate (2.39 g, 10.98 mmol, 1.1 eq.) was added. The mixture was stirred at room temperature for 1 hour. The reaction mixture was washed and extracted with dichloromethane/methanol (10:1, v/v), and the organic phases were combined, dried, concentrated, and purified by column chromatography to obtain Compound **H31-2** (600 mg, a light yellow solid, with a yield of 30%). LC-MS (ESI): m/z 201.3 $[M+1]^+$, $^1$H NMR (400 MHz, DMSO-$d_6$) δ 3.33-3.28 (m, 4H), 2.67 (m, 4H), 1.66-1.54 (m, 2H), 1.35 (s, 9H).

2. Preparation of Compound **H31-3**

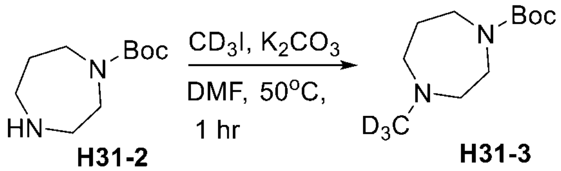


**H31-2** **H31-3**

Potassium carbonate (6.2 g, 45.0 mmol, 1.0 eq.) was added to a solution of Compound **H31-2** (3 g, 15.0 mmol, 1.0 eq.) in N,N-dimethylformamide (45 mL). The mixture was stirred at 50 °C for 10 minutes, and then iodomethane-d3 (1.74 g, 12.0 mmol, 0.8 eq.) was added. An additional 0.2 eq. of iodomethane-d3 was added every 10 minutes, and the addition was completed within 30 minutes. The reaction mixture reacted at 50 °C for 0.5 hour under a nitrogen atmosphere. Water was added to quench the reaction, and extraction was performed with dichloromethane/methanol (8:1, v/v, 80 mL × 5). The organic phases were combined, dried, concentrated, and purified by column chromatography to finally obtain Compound **H31-3** (1.72 g, a light yellow oily substance, with a yield of 53%). LC-MS (ESI): m/z 218.0 $[M+1]^+$.

3. Preparation of Compound **H31-4**

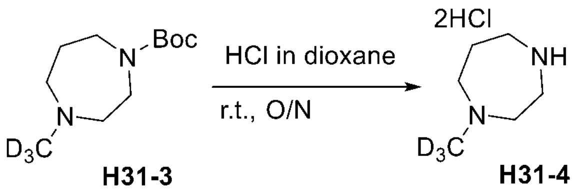


**H31-3** **H31-4**

Compound **H31-3** (1.95 g, 9.0 mmol, 1.0 eq.) was dissolved in HCl/dioxane (4M, 20 mL), and the mixture reacted overnight at room temperature. The reaction solvent was removed, and dichloromethane was added to help remove the residual dioxane, finally obtaining Compound **H31-4** (1.7 g, a light yellow oily substance, with a yield of 100%). LC-MS (ESI): m/z 118.1 [M+1]+, 1H NMR (400 MHz, DMSO-d6) δ 11.38 (s, 1H), 9.70 (s, 1H), 9.42 (s, 1H), 3.68-3.57 (m, 2H), 3.51-3.24 (m, 4H), 3.25-2.10 (m, 2H), 2.16-2.03 (m, 2H).

4. Preparation of Compound **H31-5**

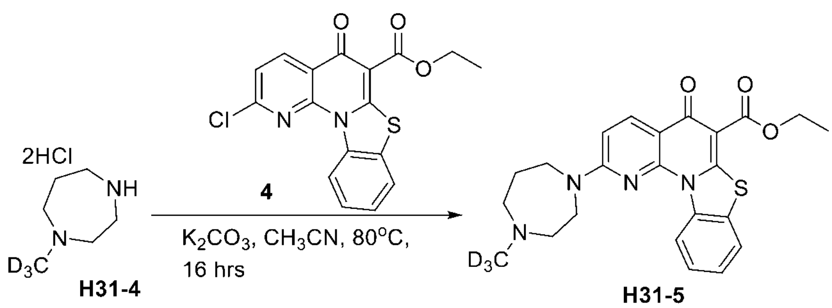


**H31-4** **H31-5**

Potassium carbonate (2.47 g, 17.88 mmol, 3.0 eq.) and Compound **H31-4** (1.7 g, 8.94 mmol, 1.5 eq.) were added to a solution of Compound **4** (2.14 g, 5.96 mmol, 1.0 eq.) in acetonitrile (20 mL). The reaction mixture was stirred at 80 °C for 16 hours under a nitrogen atmosphere. The reaction solvent was removed by concentration, water was added to the residue, and the mixture was washed and extracted with dichloromethane/methanol (10:1, v/v). The organic phases were combined, and concentrated. The crude product was slurried with ethyl acetate overnight, and filtered, and the filter cake was dried to obtain a product **H31-5** (1.6 g, a light yellow solid, with a yield of 61%). LC-MS (ESI): m/z 440.6 $[M+1]^+$, $^1H$ NMR (400 MHz, DMSO-$d_6$) δ: 9.34 (d, 1H), 8.25-8.15 (m, 1H), 8.25-7.90 (m, 1H), 7.55-7.40 (m, 2H), 6.93 (d, 1H), 4.28 (q, 2H), 4.05-3.60 (m, 4H), 2.90-2.65 (m, 2H), 2.60-2.50 (m, 2H), 2.02-1.85 (m, 2H), 1.29 (t, 3H).

5. Preparation of Compound **H31-6**

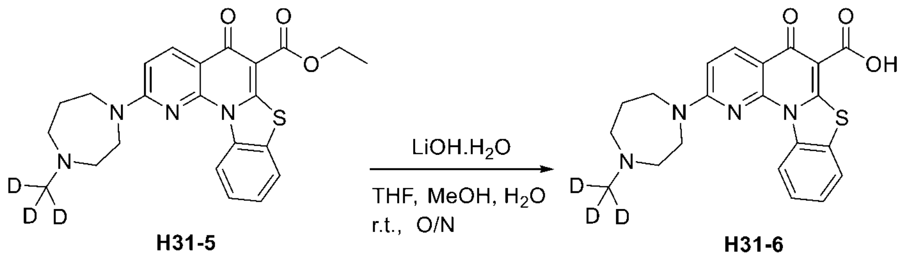


**H31-5** **H31-6**

LiOH·$H_2O$ (133 mg, 3.18 mmol, 2.0 eq.) was added to a solution of Compound **H31-5** (700 mg, 1.59 mmol, 1.0 eq.) in tetrahydrofuran/methanol/water (6 mL/2 mL/6 mL). The mixture was stirred overnight at room temperature under nitrogen protection. The reaction solvent was removed by concentration, and lyophilization was performed to obtain Compound H31-6 (1.1 g, containing lithium hydroxide) as a crude product, which was directly used in the next step. LC-MS (ESI): m/z 412.5 $[M+1]^+$, $^1H$ NMR (400 MHz, DMSO-$d_6$) δ 9.42 (d, 1H), 8.37 (d, 1H), 7.95-7.80 (m, 1H), 7.60-7.30 (m, 2H), 7.00 (d, 1H), 4.05-3.50 (m, 4H), 2.90-2.65 (m, 2H), 2.60-2.50 (m, 2H), 2.02-1.85 (m, 2H).

6. Preparation of Compound H-31

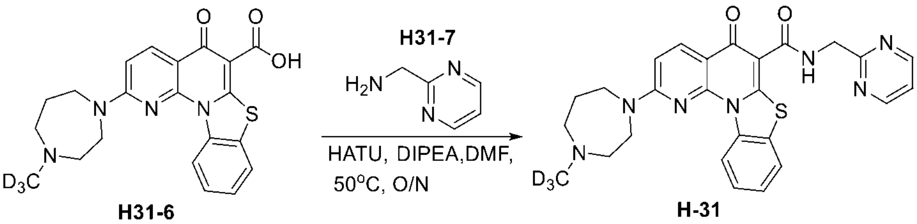


**H31-6** **H-31**

2-(7-Azabenzotriazol-1-yl)-N, N, N', N'-tetramethyluronium hexafluorophosphate (690 mg, 1.8 mmol, 1.5 eq.) and N, N-diisopropylethylamine (470 mg, 3.6 mmol, 3.0 eq.) were added to a solution of Compound **H31-6** (500 mg, 1.2 mmol, 1.0 eq.) in N, N-dimethylformamide (6 mL). The mixture was stirred at 50 °C for 0.5 hour under nitrogen protection. Compound H31-7 (200 mg, 1.8 mmol, 1.5 eq.) dissolved in N, N-dimethylformamide (2 mL) was added, and the resulting mixture was stirred at 50 °C for 16 hours under nitrogen protection. The reaction mixture was concentrated to obtain a residue. The residue was dissolved clear with dichloromethane/methanol (10/1, v/v), and washed and extracted with water. The organic phases were combined, dried, and concentrated. The crude product was slurried with methanol (5 mL) overnight, filtered, and dried to obtain Compound H-31. (269.7 mg, a light yellow solid, with a yield of 44%)。LC-MS (ESI): m/z 503.2 [M+1]+, 1H NMR (400 MHz, DMSO-d6) δ : 11.20-11.10 (m, 1H), 9.31 (s, 1H), 8.81 (d, 2H), 8.29 (d, 1H), 7.99-7.87 (m, 1H), 7.51-7.36 (m, 3H), 7.05-6.90 (m, 1H), 4.77 (d, 2H), 4.05-3.50 (m, 4H), 2.90-2.65 (m, 2H), 2.60-2.50 (m, 2H), 2.02-1.85 (m, 2H).

Other compounds in Compound H-1 to Compound H-31 listed in Table 1 were prepared using methods similar to those of the above examples.

Table 1: Compounds H-1 to H-31

| NO. | Compound | Molecular formula | MS(M+1) |
|---|---|---|---|
| H-1 | 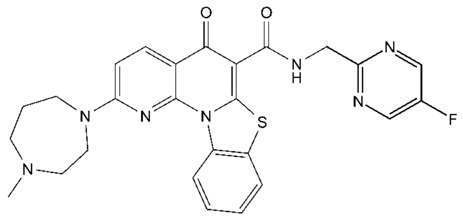 | $C_{26}H_{24}N_7O_2SF$ | 518.2 |
| H-2 | 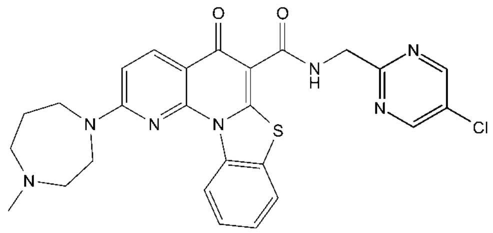 | $C_{26}H_{24}N_7O_2SCl$ | 534.1 |
| H-3 | 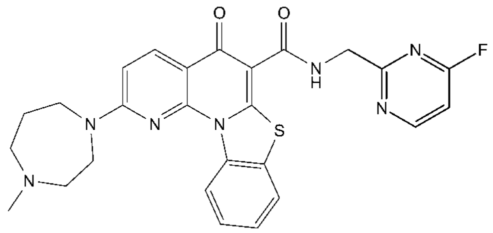 | $C_{26}H_{24}N_7O_2SF$ | 518.2 |
| H-4 | 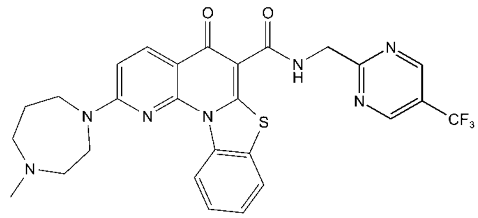 | $C_{27}H_{24}N_7O_2SF_3$ | 568.2 |
| H-5 | 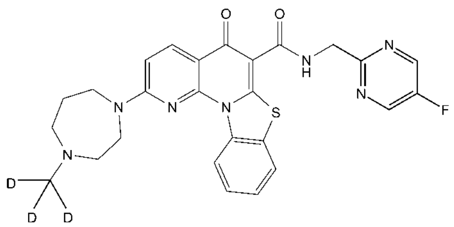 | $C_{26}H_{21}N_7O_2SFD_3$ | 521.2 |
| H-6 | 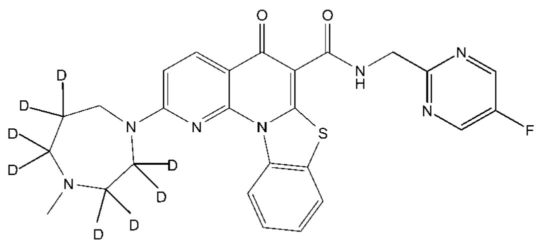 | $C_{26}H_{16}N_7O_2SFD_8$ | 526.2 |

(continued)

| NO. | Compound | Molecular formula | MS(M+1) |
|---|---|---|---|
| H-7 | 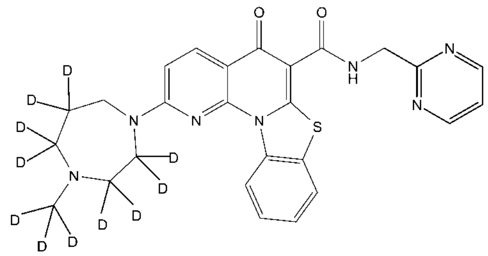 | $C_{26}H_{14}N_7O_2SD_{11}$ | 511 |
| H-8 | 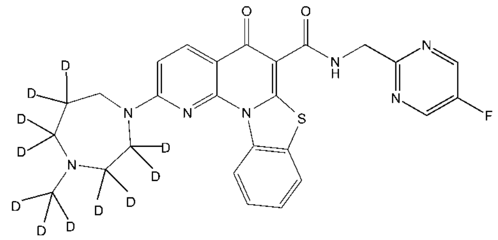 | $C_{26}H_{13}N_7O_2SFD_{11}$ | 529.2 |
| H-9 | 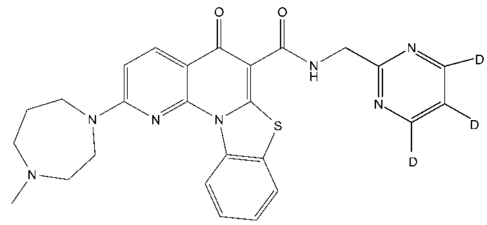 | $C_{26}H_{22}N_7O_2SD_3$ | 503.2 |
| H-10 | 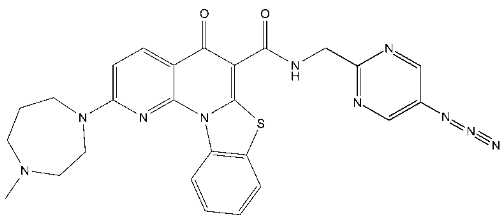 | $C_{26}H_{24}N_{10}O_2S$ | 541.2 |
| H-11 | 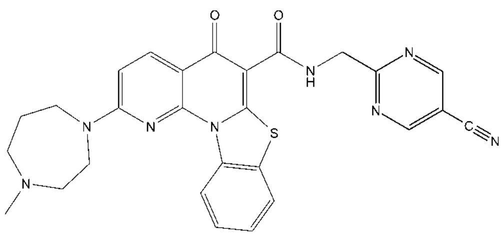 | $C_{27}H_{24}N_8O_2S$ | 525.2 |
| H-12 | 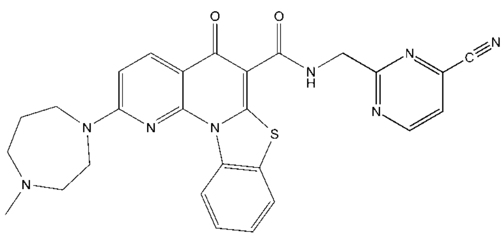 | $C_{27}H_{24}N_8O_2S$ | 525.2 |

(continued)

| NO. | Compound | Molecular formula | MS(M+1) |
|---|---|---|---|
| H-13 | 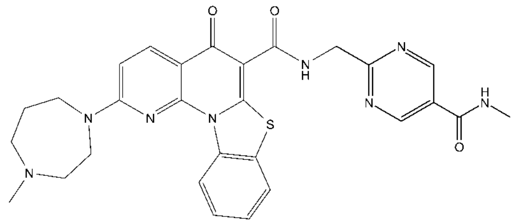 | $C_{28}H_{28}N_8O_3S$ | 557.2 |
| H-14 | 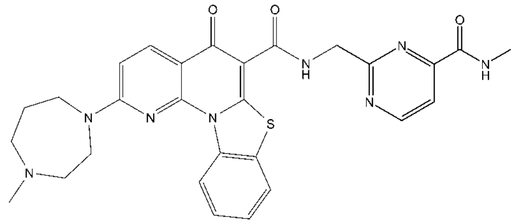 | $C_{28}H_{28}N_8O_3S$ | 557.2 |
| H-15 | 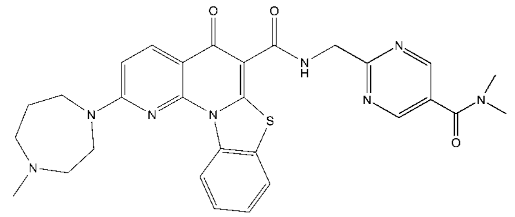 | $C_{29}H_{30}N_8O_3S$ | 571.2 |
| H-16 | 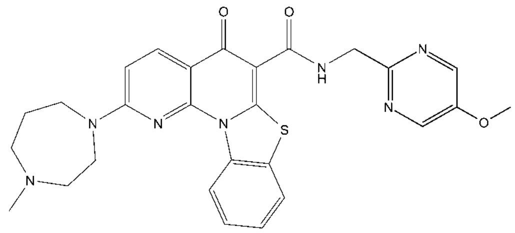 | $C_{27}H_{27}N_7O_3S$ | 530.2 |
| H-17 | 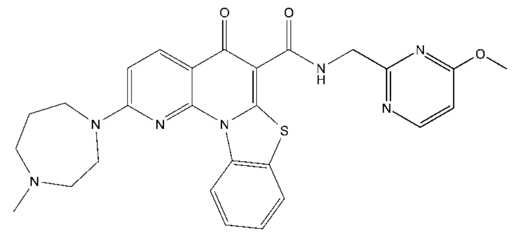 | $C_{27}H_{27}N_7O_3S$ | 530.2 |
| H-18 | 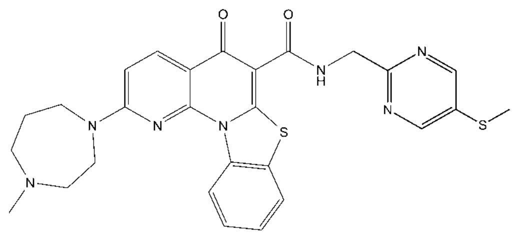 | $C_{27}H_{27}N_7O_2S_2$ | 546.2 |
| H-19 | 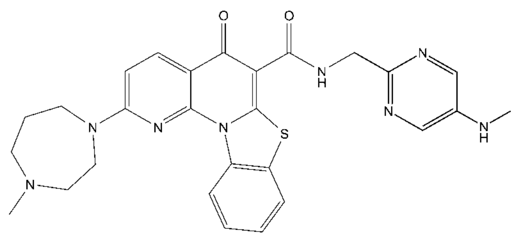 | $C_{27}H_{28}N_8O_2S$ | 529.2 |

(continued)

| NO. | Compound | Molecular formula | MS(M+1) |
|---|---|---|---|
| H-20 | 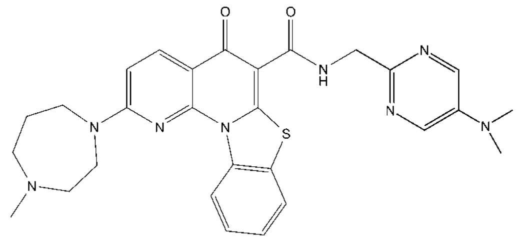 | $C_{28}H_{30}N_8O_2S$ | 543.2 |
| H-21 | 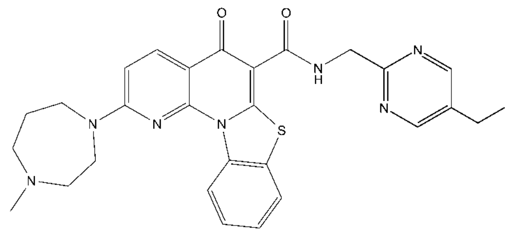 | $C_{28}H_{29}N_7O_2S$ | 528.2 |
| H-22 | 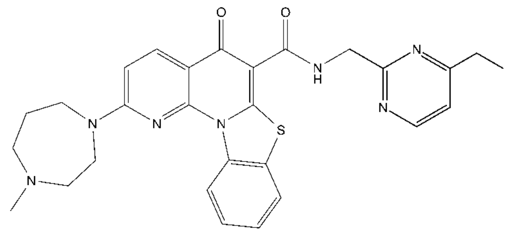 | $C_{28}H_{29}N_7O_2S$ | 528.2 |
| H-23 | 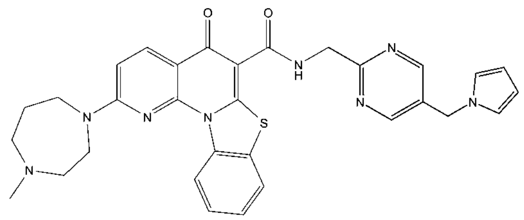 | $C_{31}H_{30}N_8O_2S$ | 579.2 |
| H-24 | 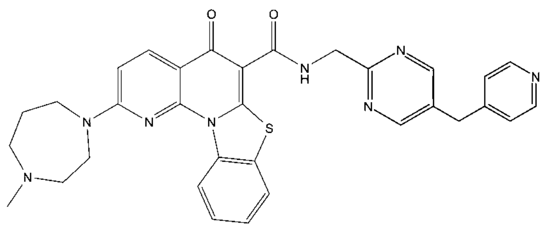 | $C_{32}H_{30}N_8O_2S$ | 591.2 |
| H-25 | 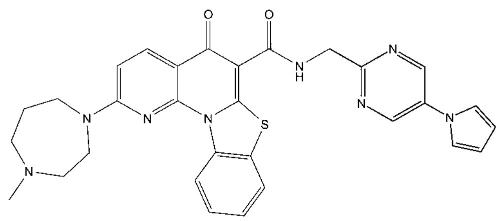 | $C_{30}H_{28}N_8O_2S$ | 565.2 |
| H-26 | 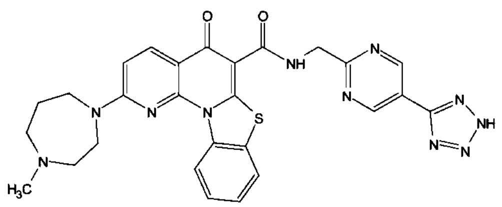 | $C_{27}H_{25}N_{11}O_2S$ | 568.2 |

(continued)

| NO. | Compound | Molecular formula | MS(M+1) |
|---|---|---|---|
| H-27 | 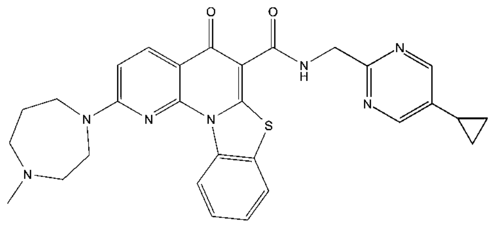 | $C_{29}H_{29}N_7O_2S$ | 540.2 |
| H-28 | 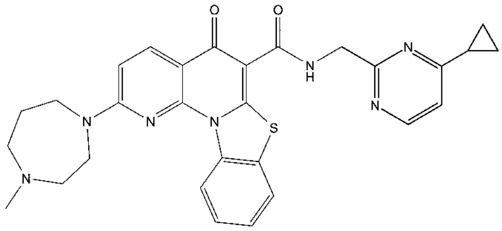 | $C_{29}H_{29}N_7O_2S$ | 540.2 |
| H-29 | 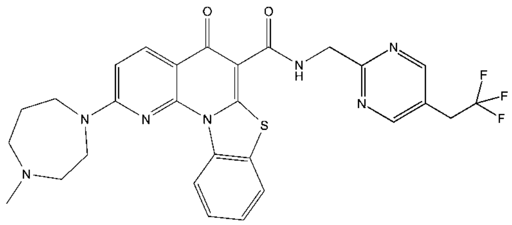 | $C_{28}H_{26}N_7O_2SF_3$ | 582.2 |
| H-30 | 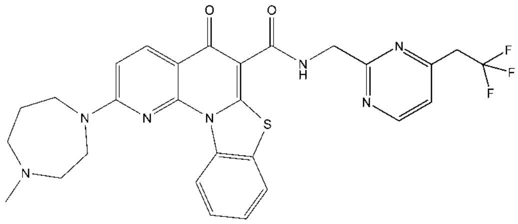 | $C_{28}H_{26}N_7O_2SF_3$ | 582.2 |
| H-31 | 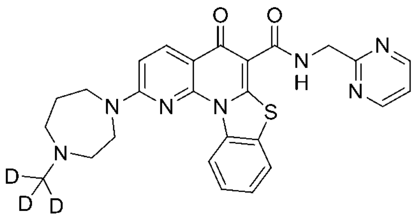 | $C_{26}H_{22}N_7O_2SD_3$ | 502.2 |

Example 23: Biological Evaluation

The following test examples further describe and explain the present application, but these examples are not intended to limit the scope of the present application.

Experimental methods not specified with particular conditions in the test examples of the present application are generally performed under conventional conditions, or under the conditions recommended by the commodity manufacturer. Reagents not specified with particular sources are commercially available conventional reagents.

Test Example 1. Inhibition of Compounds on Cancer Cells

Testing Method for Detecting Cell Activity

(1) Compound Treatment

a) Preparation of Stock Solutions of Compounds
Test compounds were dissolved in DMSO at a concentration of 10 mM or 5 mM, and Olaparib was dissolved in DMSO at a concentration of 10 mM.
b) Compound Storage
All compounds dissolved in DMSO were stored in a desiccator at room temperature for short-term storage and transferred to a -20°C refrigerator for long-term storage.
c) Preparation of Working Concentrations of Compounds

Test compounds were serially diluted in DMSO at a 3-fold gradient to prepare 10 concentration gradients, starting from 10 μM.
Olaparib was serially diluted in DMSO at a 3-fold gradient to prepare 10 concentration gradients, starting from 10 μM.
The 384-well plate loaded with the compounds was centrifuged at 1000 rpm for 1 minute.

(2) Activity Assay

a) Cells were added to the culture medium and incubated in a cell culture incubator at 37°C with 5% $CO_2$.
b) 40 μL of BRCA1- or BRCA2-deficient cells were added to a 384-well plate and incubated overnight at 37°C with 5% $CO_2$.
c) The test compounds were prepared at different concentration gradients on the master plate, and 80 nL of the test compounds and Olaparib were taken from the master plate and added to the 384-well plate containing cells, and incubated at 37°C with 5% $CO_2$ for 6 days.
d) CellTiterGlo (30 μL per well) was added to the 384-well plate, and after incubation at room temperature in the dark for 30 minutes, measurement was performed using an Envision plate reader.

(3) Data Processing

a) GraphPad Prism software was used to calculate the ICso and plot the activity curves as a function of concentration.
b) The inhibition of cell activity by the compounds was calculated using the following formula:

$$\% \text{ Inhibition} = [1 - (\text{Cmpd-Blank}) / (\text{DMSO-Blank})] * 100$$

Test Results:

Table 2. Study on the Activity Inhibition of Quinolone Analogs Against BRCA2 Gene Knockout DLD1 Cancer Cells

| ID# | Structure | $IC_{50}$(nM) DLD1 BRCA2$^{-/-}$ |
|---|---|---|
| **Olaparib** | 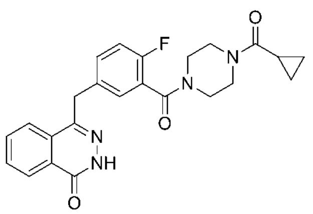 | 489.8 |
| **CX-5461** | 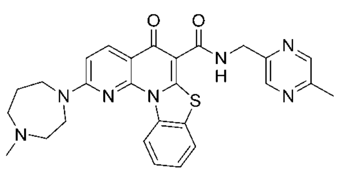 | 43.9 |

(continued)

| ID# | Structure | IC50(nM) DLD1 BRCA2-/- |
|---|---|---|
| Compound H-1 | 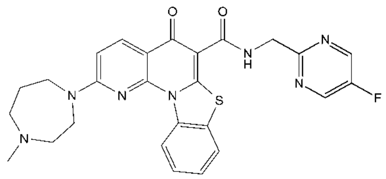 | 37.7 |
| Compound H-2 | 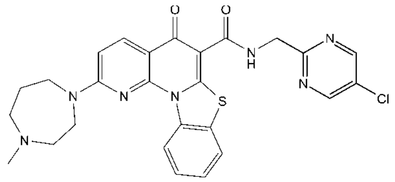 | 33.8 |
| Compound H-4 | 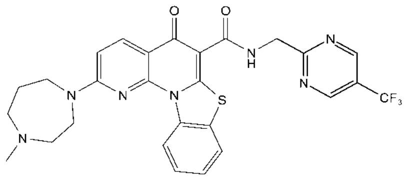 | 36.0 |
| Compound H-6 | 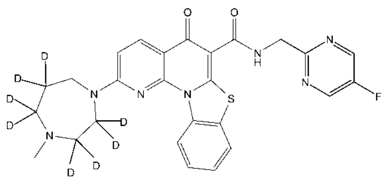 | 23.8 |
| Compound H-7 | 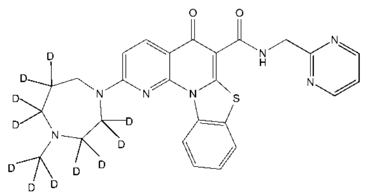 | 16.2 |
| Compound H-8 | 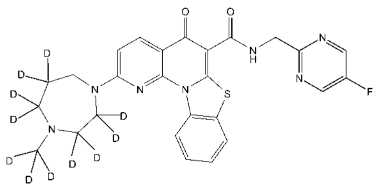 | 15.9 |
| Compound H-11 | 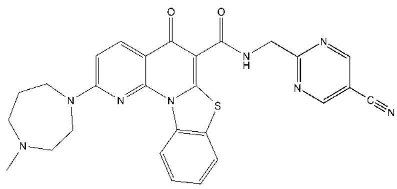 | 16.2 |
| Compound H-12 | 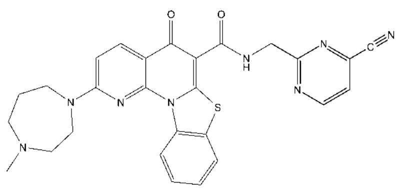 | 13.7 |

(continued)

| ID# | Structure | $IC_{50}$(nM) DLD1 BRCA2$^{-/-}$ |
|---|---|---|
| **Compound H-16** | 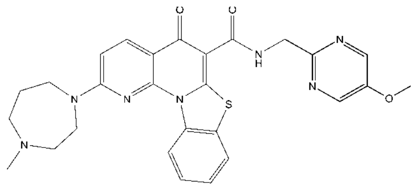 | 27.5 |
| **Compound H-19** | 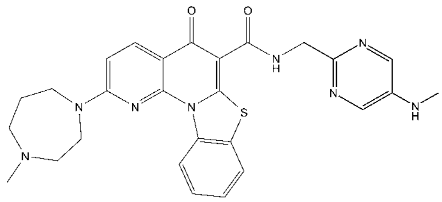 | 30.3 |
| **Compound H-22** | 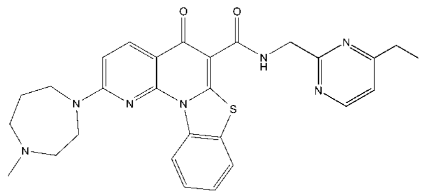 | 36.7 |
| **Compound H-31** | 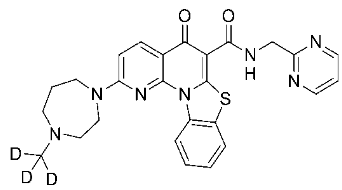 | 12.8 |

The compounds shown in the above table exhibit significantly higher inhibition of the activity of BRCA2 gene knockout DLD1 cancer cells compared to the PARP inhibitor Olaparib and CX-5461.

Table 3. Study on the Activity Inhibition of Quinolone Analogs Against **BRCA1 Gene-Deficient Ovarian Cancer Cells UWB1.289**

| ID# | Structure | $IC_{50}$ (nM) UWB1.289 Cells |
|---|---|---|
| **Olaparib** | 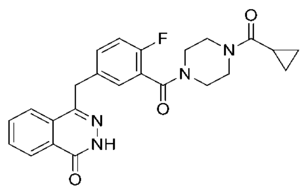 | 716.4 |
| **Compound** (CX-5461) | 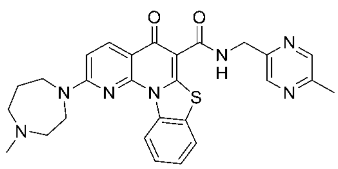 | 91.6 |
| **Compound H-31** | 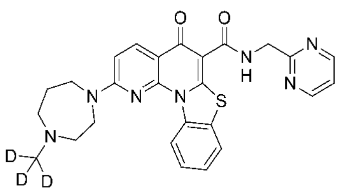 | 28.8 |

The studies on the activity of BRCA1 gene-deficient ovarian cancer cells UWB1.289 revealed that Compound H-31 exhibits a stronger ability to kill BRCA1-deficient cancer cells than the control compounds Olaparib and CX-5461, with a significantly lower $IC_{50}$ than Olaparib and CX-5461.

Test Example 2. *In-Vivo* Pharmacokinetic Study of Compounds in Mice

Test compounds were administered to mice via oral (PO) and intravenous (IV) routes. The test compounds were dissolved at room temperature. The mice used in the study were Balb/c nude mice, all female, aged 6-8 weeks, with a body weight of 20-30 grams. Before administration, the mice had free access to food and water.

Blood samples (approximately 0.03 mL per sample) were collected from the vein at appropriate time points. The samples were placed in tubes containing an anticoagulant and stored on ice until centrifugation. Blood samples were collected at 0.083, 0.25, 0.5, 1, 2, 4, 8, and 24 hours after intravenous administration, and at 0.25, 0.5, 1, 2, 4, 8, and 24 hours after oral administration. The blood samples were centrifuged at approximately 4000 rpm at 2-8 °C for 5 minutes. Plasma samples were then collected and stored frozen at approximately -80 °C until analysis. The LC-MS/MS system was used for bioanalytical work.

Table 4. Bioavailability Testing

| **Bioavailability** | **Mean** |
|---|---|
| CX-5461 | 33.8% |
| Compound H-1 | 60.7% |
| Compound H-2 | 47.2% |
| Compound H-12 | 49.1% |
| Compound H-17 | 74.6% |
| Compound H-21 | 66.8% |
| Compound H-22 | 40.6% |
| Compound H-27 | 59.2% |
| Compound H-28 | 41.6% |
| Compound H-31 | 43.1% |

Based on the *in vivo* pharmacokinetic study in mice, the compounds in Table 4 have significantly improved oral bioavailability compared with CX-5461, which is beneficial for the development of oral pharmaceutical formulations and provides a more convenient administration method for patients.

Test Example 3. hERG Cardiotoxicity Assay of Compounds

Assay Method:

1) The hERG stably expressed HEK 293 cell line (Cat# K1236) was purchased from Invitrogen. Cells were cultured in a medium consisting of 85% DMEM, 10% FBS, 0.1 mM NEAA, 25 mM HEPES, 100 U/mL penicillin-streptomycin, 5 μg/mL Blasticidin, and 400 μg/mL Geneticin. Cells were passaged approximately three times a week using TrypLE™ Express and maintained at ~40% to ~80% confluency. Before assay, cells were placed on coverslips with $5\times10^5$ cells per 6-cm cell culture dish and induced with 1 μg/mL doxycycline for 48 hours.

2) The coverslip was removed from the cell culture dish and placed on the microscope stage. A suitable cell was located using a 10× objective. The tip of the electrode was located under the microscope using the 10× objective, and the focus was adjusted to the plane of the cell. Once the tip was in focus, the coarse control of the manipulator was used to advance the electrode downward toward the cell while moving the objective to keep the tip in focus. When directly above the cell, the objective was switched to 40× objective, and the fine control of the manipulator was used to approach the cell surface in small steps.

3) A blank control was first performed to establish a baseline. Once the hERG current was found to be stable for 5 minutes, the working solution was applied. In the presence of the test compounds, the hERG current was recorded for approximately 5 minutes to reach a steady state, followed by capturing 5 scans. For dose-response testing, 5 doses of the test compounds were cumulatively applied to the cells from low to high concentrations. The positive control, dofetilide at a concentration of 150 nM, was applied to each cell, and the hERG current was measured in the test compounds at the highest concentration as a negative control to normalize the percentage of inhibition. To ensure good performance of the

cultured cells and operations, cells from the same batch were also tested with 5 doses of dofetilide.

Table 5. hERG Toxicity Assay Results

| Test Compounds | hERG $IC_{50}$ [μM] |
|---|---|
| CX-5461 | 5.1 |
| Compound H-13 | >30.0 |
| Compound H-15 | >30.0 |
| Compound H-18 | 10.7 |

Test Results: The $IC_{50}$ values of the compounds listed in Table 5 in the hERG toxicity assay are higher than that of CX-5461, indicating an improvement in cardiotoxicity compared to CX-5461.

Test Example 4. Compound Toxicity Assay in Mouse Models

a. Effects of Repeated Dosing at 50 mg/kg of CX-5461, Compound H-31, and Vehicle on Mouse Body Weight

Test compounds were administered to mice via the oral (PO) route. The test compounds were dissolved at room temperature. The mice used in the study were all female, and aged 6-8 weeks. Before administration, the mice had free access to food and water.

Vehicle control: 50 mM $NaH_2PO_4$, pH 4.0, administered once every 3 days. CX-5461 and Compound H-31 were administered once every 3 days at a dose of 50 mg/kg. If the body weight of the mice decreased by more than 15%, administration was suspended until the body weight recovered, after which administration was resumed. The effects of the compounds on mouse body weight are shown in FIG. 1.

The above experiment showed that after 21 days of repeated administration at a dose of 50 mg/kg, CX-5461 significantly reduced the body weight of the mice (P<0.01, t-test). In addition to the body weight loss, the entire group of animals in the CX-5461 administration group showed poor overall condition, sluggish movement, and pallor in the tails and toes due to blood loss. Furthermore, as the average body weight loss exceeded 10%, administration was halted for the entire group. In contrast, mice administered Compound H-31 exhibited weight trends similar to the control group, and no abnormal clinical symptoms were observed. This indicates that the toxic and side effects of Compound H-31 are less than those of CX-5461.

b. Effects of Repeated Administration Dose of 55 mg/kg of CX-5461, Compounds of the H Series, and Vehicle on Mouse Body Weight

Test compounds were administered to mice via the oral (PO) route. The test compounds were dissolved at room temperature. The mice (Balb/c) used in the study were all female, and aged 6-8 weeks. Before administration, the mice had free access to food and water.

Vehicle control: 50 mM $NaH_2PO_4$, pH 4.0. CX-5461 and the H series compounds were administered once every 3 days at a dose of 55 mg/kg, with four doses administered in total. The effects of these compounds on mouse body weight are shown in FIG. 2-1 and FIG. 2-2.

The above experiments showed that, at a dose of 55 mg/kg and after 4 repeated administrations, CX-5461 significantly reduced the body weights of the mice, and all mice in this group died by day 13. In contrast, after administration of Compounds H-4 and H-17, the body weights of the mice showed a slight decrease followed by recovery, with no deaths observed. After administration of Compounds H-12, H-21, H-27, and H-28, the body weights of the mice remained unchanged. These results indicate that the toxic side effects of Compounds H-4, H-12, H-17, H-21, H-27, and H-28 are significantly improved compared to CX-5461.

Test Example 5. Inhibition of Human-Derived Cancer by Compounds in Mouse Models

The anti-cancer efficacy of the compounds of the present application was evaluated in NOD SCID mice. Female mice aged 6-8 weeks were subcutaneously inoculated with MDA-MB-436 cancer cells to form tumors. When the tumors reached a size of 100-150 $mm^3$, the mice were randomly divided into groups, with 6 mice in each group. The administration route was oral administration. Olaparib was administered once a day at a dose of 100 mg/kg for a total of 28 days. The vehicle control group and Compound H-31 were administered once every 3 days at a dose of 25 mg/kg.

As shown in FIG. 3, compared with the control group, both the Olaparib treatment group and the Compound H-31

treatment group significantly inhibited tumor growth ($p<0.001$). The cancer inhibition rate of the Olaparib treatment group was 63.4%, and the cancer inhibition rate of the Compound H-31 treatment group was 82.2%. The cancer inhibition rate of the Compound H-31 treatment group was significantly higher than that of the Olaparib treatment group ($p<0.001$), and the administration dose of Compound H-31 (25 mg/kg, Q3D) was much lower than that of Olaparib (100 mg/kg, Q1D).

Test Example 6. Inhibition of Viral DNA Replication by Compounds

A segment of viral DNA containing a quadruplex DNA sequence was used as a template, and the primer was fluorescently labeled and annealed to the 3'-end of the template DNA. Taq DNA polymerase was added to the system to synthesize a complementary strand using the fluorescently labeled primer. If DNA synthesis was not inhibited, a full-length copy of the template would be synthesized. If the added compound bound non-specifically to the doublestranded DNA, the full-length product would decrease, and PCR products of different short lengths would be formed. If the compound specifically bound to the quadruplex DNA in the viral DNA sequence, DNA replication would only stop at the quadruplex DNA region. PCR products were detected by DNA electrophoresis to determine whether the compound inhibits viral DNA replication compared to the vehicle control.

The experimental results showed that the $IC_{50}$ of the compound of Formula (I) for inhibiting viral DNA replication was 0.01-1 μM. Therefore, the compound of Formula (I) has strong inhibitory effects on the replication of viral DNAs containing quadruplex DNA structures.

Test Example 7 Determination of Antiviral Cell-Protective Activity of Compounds

MRC5 cells were seeded in microplates at a specific density and cultured overnight in an incubator at 37 °C with 5% $CO_2$. Serially diluted compounds and GFP-labeled human herpesvirus HCMV-AD169 were added to the microplates. A cell control group (cells without compound treatment or viral infection) and a virus control group (cells infected with the virus but without compound treatment) were set up. The test compounds and control compounds were tested at 8 concentrations, with 3-fold serial dilutions and triplicate wells for each concentration. Meanwhile, the cytotoxicity of the test compounds and control compounds on cells was determined. The cells were cultured in an incubator at 37 °C with 5% $CO_2$ for 7 days. Acumen Cellista was used to detect the GFP fluorescence intensity inside the cells in each well. Cell viability detection reagents CCK-8 or Cell Titer Glo were used to measure cell viability, and the original data were used for calculating the cytotoxicity of the test compounds. GraphPad Prism software was applied to analyze the dose-response curves of the test compounds, calculate the virus inhibition $EC_{50}$ and cytotoxicity $CC_{50}$ values, and further calculate the therapeutic index (SI), using the formula $SI = CC_{50}/EC_{50}$, which was used to evaluate the therapeutic potential of the drug; a higher SI value indicates stronger efficacy of the drug against viruses.

The experiments showed that when the $IC_{50}$ concentrations of the compounds of Formula (I) were approximately 0.01-1.0 μM, the compounds exerted a protective effect against the decrease in cell activity caused by viral infection. Among these compounds, Compound H-31 exhibited higher inhibitory efficacy against HCMV than Ganciclovir.

Table 6: Effective Inhibition of Human Herpesvirus HCMV by H-31

| ID | Virus inhibition $EC_{50}$ (μM) | **SI** ($CC_{50}/EC_{50}$) |
|---|---|---|
| H-31 | 0.03 | >101.7 |
| Ganciclovir | 1.60 | >60.7 |

Test Example 8. Inhibition of Multiple Sclerosis by Compounds of Formula (I)

The autoimmune encephalomyelitis (EAE) model was established as follows: C57BL/6 male mice, aged 6-8 weeks, were divided into groups with 6 mice per group:

Group 1: EAE + vehicle;
Group 2: EAE + PRN2246;
Group 3: EAE + test compound.

The administration frequency was once a day, and the administration cycle lasted for 21 days. MOG35-55 was diluted with PBS, and the diluted solution was mixed with the complete Freund's adjuvant. The mixture was emulsified into a water-in-oil state using a glass syringe to form an antigen emulsion for EAE induction. On the day of immunization and the second day, the mice were intraperitoneally injected with pertussis toxin to induce the establishment of the mouse autoimmune encephalomyelitis model.

Neurological function scoring criteria:
The commonly used 0-5 grade scoring scale was adopted, where:

Grade 0: No disease onset;
Grade 1: Decreased tail tension or tail paralysis in mice;
Grade 2: Tail paralysis and hindlimb weakness in mice;
Grade 3: Hindlimb palsy in mice;
Grade 4: Quadriplegia in mice, with inability to reset when manually turned over;
Grade 5: Near-death state or death after disease onset.

Scoring frequency: Starting from day 11, scoring was performed once a day for a total of 10 days. Body weight measurement: twice per week.
The experiment showed that when the concentrations of the compounds of Formula (I) were 0.1-10 μM, the compounds exerted a delaying effect on the progression of multiple sclerosis.
The specific examples of the present application have been described above; however, it should be understood that the present application is not limited to the above-specified embodiments. Those skilled in the art can make various variations and modifications without departing from the concept of the present application, and all such variations and modifications fall within the protection scope of the present application.

## Claims

1. A compound of Formula (I), or a pharmaceutically acceptable salt, stereoisomer, racemate, deuterated compound, or solvate thereof:

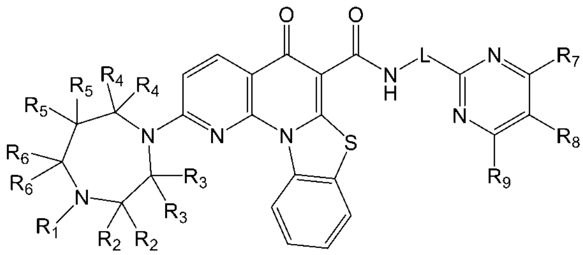

Formula (I)

wherein:

$R_1$, $R_2$, $R_3$, $R_4$, $R_5$, and $R_6$ are selected from H, deuterium, halogen, C1-C6 alkyl, or C1-C4 alkyl substituted with one or more deuterium atoms;
$R_7$, $R_8$, and $R_9$ are selected from H, deuterium, or C1-C4 alkyl substituted with one or more deuterium atoms; alternatively, $R_7$, $R_8$, and $R_9$ may simultaneously or separately be D, halogen, azido, -CN, $-CF_3$, $-CONR_{10}R_{11}$, $-NR_{10}R_{11}$, $-SR_{11}$, $-OR_{11}$, $-R_{12}$, -W, -M-W, -V, or $-L-N(R_{13})-V$;
L is $-(CH_2)_n-$, where n = 0-6;
$R_{10}$ is selected from H or C1-C6 alkyl, optionally substituted with one or more halogen atoms or =O;
$R_{11}$ is selected from H, C1-C10 alkyl, C1-C10 heteroalkyl, C2-C10 alkenyl, or C2-C10 heteroalkenyl, each of which may optionally be substituted with one or more halogen atoms, =O, or optionally substituted 3- to 7-membered carbocycle or heterocycle;
$R_{12}$ is selected from optionally substituted C1-C10 alkyl, C2-C10 alkenyl, C5-C10 aryl, or C6-C12 arylalkyl or heterocycle, each of which may optionally be substituted with one or more halogen atoms, =O, or optionally substituted 3- to 6-membered carbocycle or heterocycle;
$R_{13}$ is selected from H, C1-C10 alkyl, C1-C10 heteroalkyl, C2-C10 alkenyl, or C2-C10 heteroalkenyl, each of which may optionally be substituted with one or more halogen atoms, =O, or optionally substituted 3- to 7-membered carbocycle or heterocycle;
M is a linker selected from C1-C10 alkylene, C1-C10 heteroalkylene, C2-C10 alkenylene, or C2-C10 heteroalkenylene, each of which may optionally be substituted with one or more substituents selected from halogen, =O, or C1-C6 alkyl;
W is selected from optionally substituted 4- to 7-membered nitrogen-containing heterocycle, which optionally contains a heteroatom selected from N, O, or S as a ring-forming member; and

V is selected from optionally substituted 3- to 4-membered carbocycle, or C1-C6 alkyl substituted with 1 to 4 fluorine atoms.

**2.** The compound, or the pharmaceutically acceptable salt, stereoisomer, racemate, deuterated compound, or solvate thereof according to claim 1, wherein:

$R_1$, $R_2$, $R_3$, $R_4$, $R_5$, and $R_6$ are selected from H, deuterium, halogen, C1-C6 alkyl, or C1-C4 alkyl substituted with one or more deuterium atoms;
$R_7$, $R_8$, and $R_9$ are selected from H, deuterium, or C1-C4 alkyl substituted with one or more deuterium atoms; alternatively, $R_7$, $R_8$, and $R_9$ may simultaneously or separately be D, halogen, azido, -CN, $-CF_3$, - $CONR_{10}R_{11}$, $-NR_{10}R_{11}$, $-SR_{11}$, $-OR_{11}$, $-R_{12}$, -W, -M-W, or -V;
L is $-(CH_2)_n-$, where n = 0-6;
$R_{10}$ is selected from H or C1-C6 alkyl, optionally substituted with one or more halogen atoms or =O;
$R_{11}$ is selected from H, C1-C10 alkyl, C1-C10 heteroalkyl, C2-C10 alkenyl, or C2-C10 heteroalkenyl, each of which may optionally be substituted with one or more halogen atoms, =O, or optionally substituted 3- to 7-membered carbocycle or heterocycle;
$R_{12}$ is selected from optionally substituted C1-C10 alkyl, C2-C10 alkenyl, C5-C10 aryl, or C6-C12 arylalkyl or heterocycle, each of which may optionally be substituted with one or more halogen atoms, =O, or optionally substituted 3- to 6-membered carbocycle or heterocycle;
M is a linker selected from C1-C10 alkylene, C1-C10 heteroalkylene, C2-C10 alkenylene, or C2-C10 heteroalkenylene, each of which may optionally be substituted with one or more substituents selected from halogen, =O, or C1-C6 alkyl;
W is selected from optionally substituted 4- to 7-membered nitrogen-containing heterocycle, which optionally contains a heteroatom selected from N, O, or S as a ring-forming member; and
V is selected from optionally substituted 3- to 4-membered carbocycle, or C1-C6 alkyl substituted with 1 to 4 fluorine atoms.

**3.** The compound, or the pharmaceutically acceptable salt, stereoisomer, racemate, deuterated compound, or solvate thereof according to claim 1 or 2, wherein:

$R_1$, $R_2$, $R_3$, $R_4$, $R_5$, and $R_6$ are each independently selected from H, deuterium, halogen, C1-C6 alkyl, or C1-C4 alkyl substituted with one or more deuterium atoms;
$R_7$, $R_8$, and $R_9$ are each independently selected from H, deuterium, halogen, azido, -CN, C1-C6 alkyl, C1-C6 haloalkyl, -O(C1-C6 alkyl), -S(C1-C6 alkyl), $-CONR_{10}R_{11}$, $-NR_{10}R_{11}$, 3- to 4-membered cycloalkyl, 4- to 7-membered nitrogen-containing heterocycle, or $-(CH_2)_m$-(4- to 7-membered nitrogen-containing heterocycle), wherein $R_{10}$ and $R_{11}$ are each independently selected from H or C1-C6 alkyl; the 4- to 7-membered nitrogen-containing heterocycle contains 1, 2, 3, or 4 nitrogen atoms; and m = 1-3;
L is $-(CH_2)_n-$, where n = 1-6;
$R_{10}$ is selected from H or C1-C6 alkyl, wherein the C1-C6 alkyl is optionally substituted with one or more halogen atoms; and
$R_{11}$ is selected from H or C1-C6 alkyl, wherein the C1-C6 alkyl is optionally substituted with one or more halogen atoms.

**4.** The compound, or the pharmaceutically acceptable salt, stereoisomer, racemate, deuterated compound, or solvate thereof according to any one of claims 1 to 3, wherein:

$R_1$, $R_2$, $R_3$, $R_4$, $R_5$, and $R_6$ are each independently selected from H, deuterium, C1-C6 alkyl, or C1-C4 alkyl substituted with one or more deuterium atoms; or
$R_1$ is C1-C6 alkyl, or C1-C4 alkyl substituted with one or more deuterium atoms, and $R_2$ to $R_6$ are each independently H or deuterium; or
$R_1$ is $-CH_3$ or $-CD_3$, and $R_2$ to $R_6$ are all H or all deuterium; or
$R_1$ is $-CH_3$ or $-CD_3$, and $R_2$ to $R_6$ are all H; or, $R_4$ is H and $R_2$, $R_3$, $R_5$, and $R_6$ are all deuterium; or
$R_1$ is $-CH_3$, and $R_2$ to $R_6$ are all H; or
$R_1$ is $-CD_3$, and $R_2$ to $R_6$ are all H.

**5.** The compound, or the pharmaceutically acceptable salt, stereoisomer, racemate, deuterated compound, or solvate thereof according to any one of claims 1 to 4, wherein

$R_7$, $R_8$, and $R_9$ are each independently selected from H, deuterium, halogen, azido, -CN, C1-C6 alkyl, C1-C6 haloalkyl, -O(C1-C6 alkyl), -S(C1-C6 alkyl), $-CONR_{10}R_{11}$, $-NR_{10}R_{11}$, 3- to 4-membered cycloalkyl, 5- to 6-membered nitrogen-containing heterocycle, or $-CH_2$-(5- to 6-membered nitrogen-containing heterocycle), wherein $R_{10}$ and $R_{11}$ are each independently selected from H or C1-6 alkyl, and the 5- to 6-membered nitrogen-containing heterocycle contains 1, 2, 3, or 4 nitrogen atoms; or

one of $R_7$, $R_8$, and $R_9$ is selected from H, halogen, azido, -CN, C1-C6 alkyl, C1-C6 haloalkyl, -O(C1-C6 alkyl), -S(C1-C6 alkyl), $-CONR_{10}R_{11}$, $-NR_{10}R_{11}$, 3- to 4-membered cycloalkyl, 5- to 6-membered nitrogen-containing heterocycle, or $-CH_2$-(5- to 6-membered nitrogen-containing heterocycle), wherein $R_{10}$ and $R_{11}$ are each independently selected from H or C1-6 alkyl, and the 5- to 6-membered nitrogen-containing heterocycle contains 1, 2, 3, or 4 nitrogen atoms; and the other two of $R_7$, $R_8$, and $R_9$ are H. In some embodiments, $R_7$, $R_8$, and $R_9$ are all deuterium; or

$R_8$ is selected from H, halogen, azido, -CN, C1-C6 alkyl, C1-C6 haloalkyl, -O(C1-C6 alkyl), -S(C1-C6 alkyl), $-CONR_{10}R_{11}$, $-NR_{10}R_{11}$, 3- to 4-membered cycloalkyl, 5- to 6-membered nitrogen-containing heterocycle, or $-CH_2$-(5- to 6-membered nitrogen-containing heterocycle), wherein $R_{10}$ and $R_{11}$ are each independently selected from H or C1-6 alkyl, and the 5- to 6-membered nitrogen-containing heterocycle contains 1, 2, 3, or 4 nitrogen atoms; and $R_7$ and $R_9$ are H; or

$R_7$ is selected from H, halogen, azido, -CN, C1-C6 alkyl, C1-C6 haloalkyl, -O(C1-C6 alkyl), -S(C1-C6 alkyl), $-CONR_{10}R_{11}$, $-NR_{10}R_{11}$, 3- to 4-membered cycloalkyl, 5- to 6-membered nitrogen-containing heterocycle, or $-CH_2$-(5- to 6-membered nitrogen-containing heterocycle), wherein $R_{10}$ and $R_{11}$ are each independently selected from H or C1-6 alkyl, and the 5- to 6-membered nitrogen-containing heterocycle contains 1, 2, 3, or 4 nitrogen atoms; and $R_8$ and $R_9$ are H; or

$R_8$ is H, F, Cl, $-CF_3$, -CN, $-N_3$, $-CONHCH_3$, $-CON(CH_3)_2$, $-OCH_3$, $-SCH_3$, $-NHCH_3$, $-N(CH_3)_2$, $-C_2H_5$, - $CH_2$-1-pyrrolyl, $-CH_2$-4-pyridyl, 1-pyrrolyl, 2H-tetrazol-5-yl, cyclopropyl, or $-CH_2CF_3$; and $R_7$ and $R_9$ are H; or

$R_7$ is H, F, Cl, $-CF_3$, -CN, $-N_3$, $-CONHCH_3$, $-CON(CH_3)_2$, $-OCH_3$, $-SCH_3$, $-NHCH_3$, $-N(CH_3)_2$, $-C_2H_5$, - $CH_2$-1-pyrrolyl, $-CH_2$-4-pyridyl, 1-pyrrolyl, 2H-tetrazol-5-yl, cyclopropyl, or $-CH_2CF_3$; and $R_8$ and $R_9$ are H; or

$R_7$ is H, F, Cl, $-CF_3$, -CN, $-N_3$, $-CONHCH_3$, $-CON(CH_3)_2$, $-OCH_3$, $-SCH_3$, $-NHCH_3$, $-N(CH_3)_2$, $-C_2H_5$, cyclopropyl, or $-CH_2CF_3$; and $R_8$ and $R_9$ are H; or

$R_7$ is H, F, -CN, $-CONHCH_3$, $-OCH_3$, $-C_2H_5$, cyclopropyl, or $-CH_2CF_3$; and $R_8$ and $R_9$ are H.

**6.** The compound, or the pharmaceutically acceptable salt, stereoisomer, racemate, deuterated compound, or solvate thereof according to any one of claims 1 to 5, wherein

L is $-(CH_2)_n-$, where n = 1-3; or L is $-CH_2-$; and/or
$R_{10}$ and $R_{11}$ are each independently H or $-CH_3$.

**7.** The compound, or the pharmaceutically acceptable salt, stereoisomer, racemate, deuterated compound, or solvate thereof according to claim 1, wherein the compound is selected from the following structural formulas:

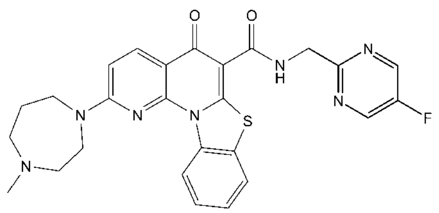
H-1,

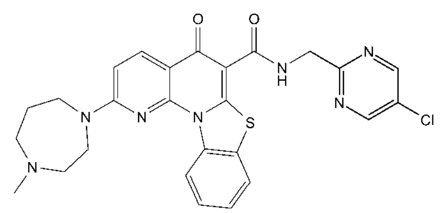
H-2,

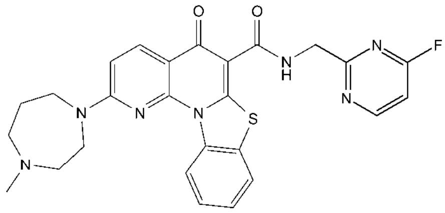
H-3,

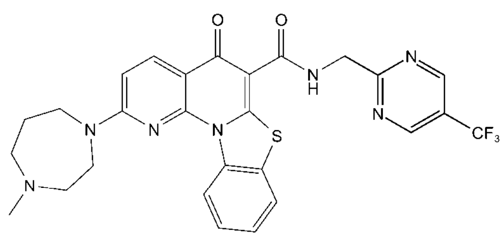
H-4,

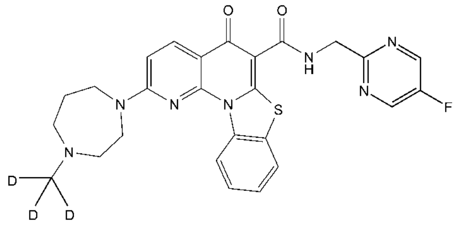
H-5,

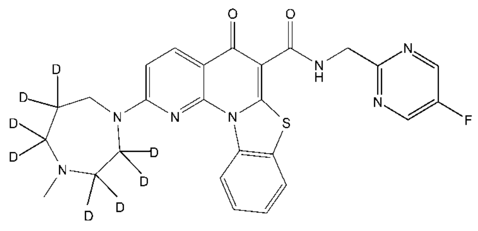
H-6,

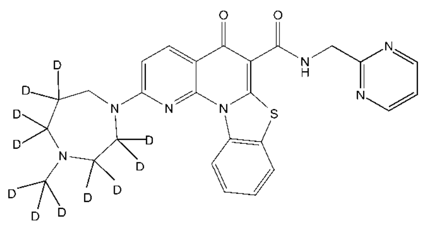
H-7,

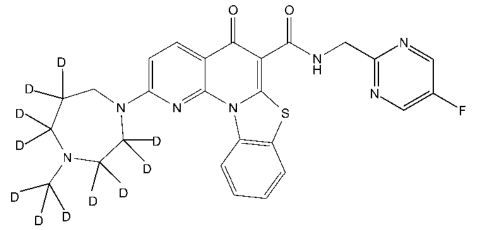
H-8,

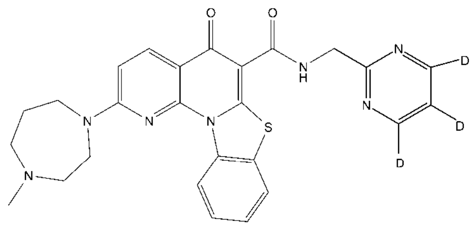
H-9,

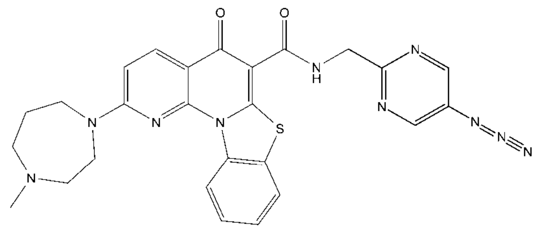
H-10,

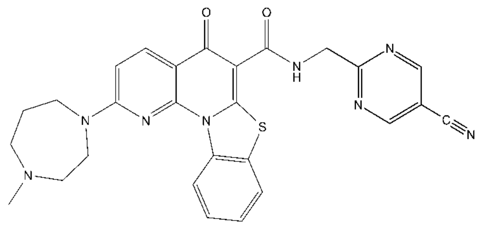
H-11,

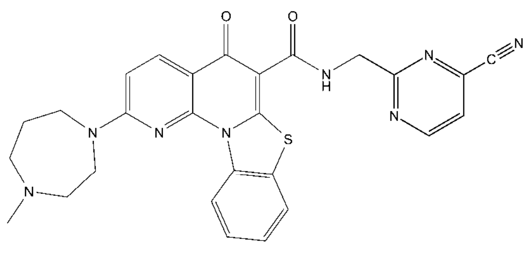
H-12,

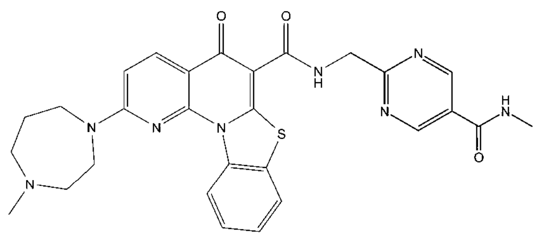
H-13,

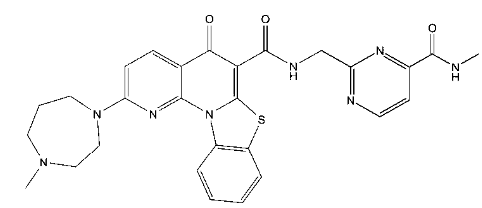
H-14,

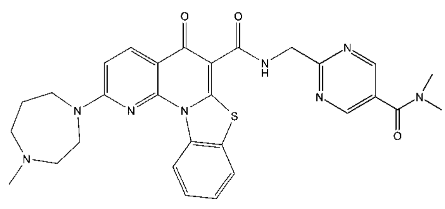
H-15,

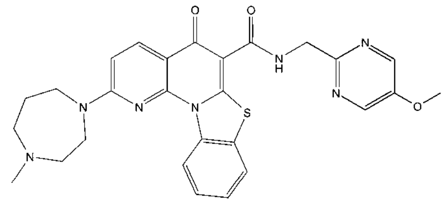
H-16,

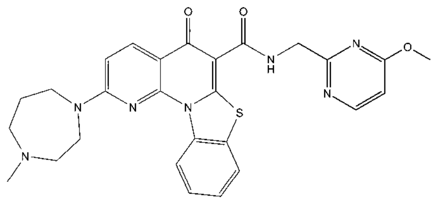
H-17,

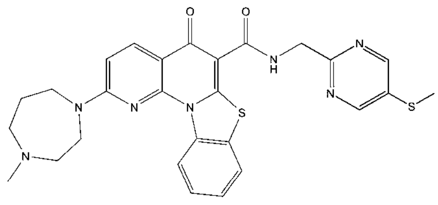
H-18,

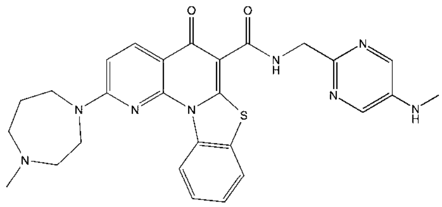
H-19,

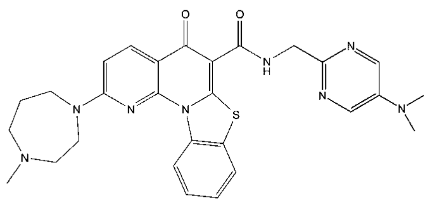
H-20,

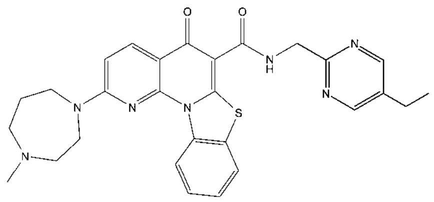
H-21,

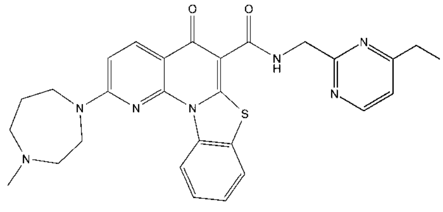
H-22,

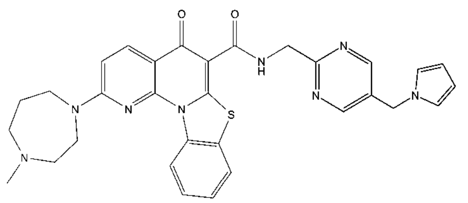
H-23,

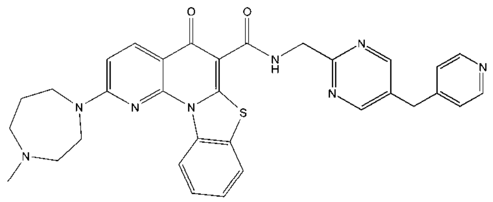
H-24,

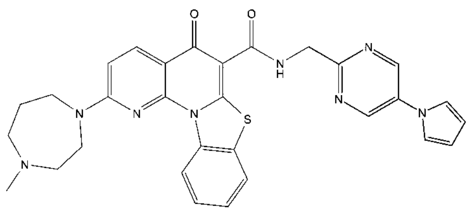
H-25,

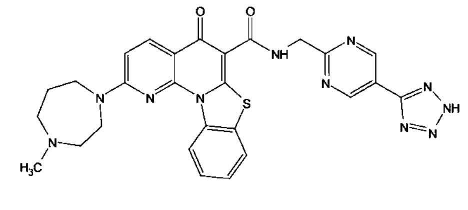
H-26,

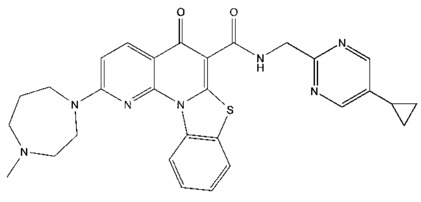
H-27,

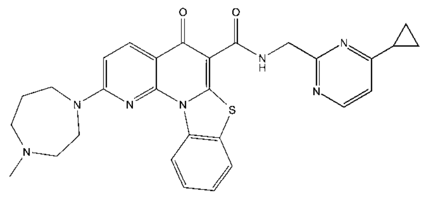
H-28,

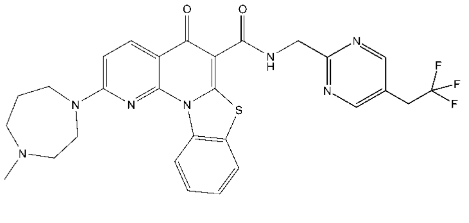
H-29,

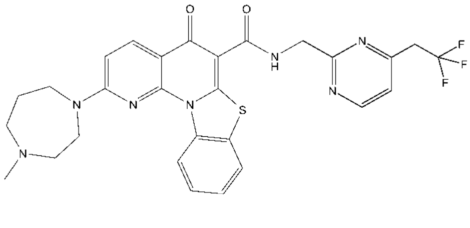
H-30,

or

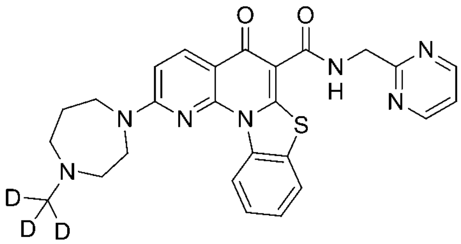

H-31.

8. A pharmaceutical composition comprising an effective amount of the compound of Formula (I), or the pharmaceutically acceptable salt, stereoisomer, racemate, deuterated compound, or solvate thereof according to any one of claims 1 to 7, and a pharmaceutically acceptable excipient.

9. Use of the compound of Formula (I), or the pharmaceutically acceptable salt, stereoisomer, racemate, deuterated compound, or solvate thereof according to any one of claims 1 to 7, or the pharmaceutical composition according to claim 8, in the manufacture of a medicament for the treatment of tumors, autoimmune diseases, or diseases caused by bacteria, fungi or viruses.

10. The use according to claim 9, wherein the tumors are a cancer, wherein the cancer is a cancer with BRCA-1 or BRCA-2 mutation (homozygous or heterozygous mutation), a homologous recombination repair-deficient cancer, a non-homologous end joining (NHEJ) repair-deficient cancer, or other DNA damage repair-deficient cancers, and a cancer with overexpression of c-Myc, N-Myc or L-Myc.

11. The use according to claim 9, wherein the tumors are a cancer, wherein the cancer is breast cancer, ovarian cancer, endometrial cancer, cervical cancer, oral cancer, pancreatic cancer, prostate cancer, lung cancer, liver cancer/hepatocellular carcinoma, leukemia, lymphoma, myeloma, skin cancer, peritoneal cancer, glioblastoma, osteosarcoma, lymph node cancer, gastrointestinal tumor, head and neck cancer, kidney cancer, or cardiac cancer.

12. The use according to claim 9, wherein the autoimmune diseases are multiple sclerosis.

13. The use according to claim 9, wherein the viruses are selected from hepatitis B virus, hepatitis C virus, rhinovirus, varicella-zoster virus, herpes simplex virus, cytomegalovirus, poxvirus, encephalitis virus, hantavirus, arbovirus, human papillomavirus, Sironi virus, human immunodeficiency virus, influenza virus, Epstein-Barr virus, respiratory syncytial virus, or coronavirus.

14. Use of the compound of Formula (I), or the pharmaceutically acceptable salt, stereoisomer, racemate, deuterated compound, or solvate thereof according to any one of claims 1 to 7, or the pharmaceutical composition according to claim 8 in the manufacture of a medicament for treating diseases that are ameliorated or rendered resistant by the use of a PARP inhibitor, or in combination with a PARP inhibitor drug, wherein the PARP inhibitor drug used in combination is selected from Olaparib, Niraparib, Rucaparib, Talazoparib, Fluzoparib, or Pamiparib.

15. Use of the compound of Formula (I), or the pharmaceutically acceptable salt, stereoisomer, racemate, deuterated compound, or solvate thereof according to any one of claims 1 to 7, or the pharmaceutical composition according to claim 8, in combination with a topoisomerase I and topoisomerase II inhibitor, an ATM inhibitor, an ATR inhibitor, or a DNA-pK inhibitor.

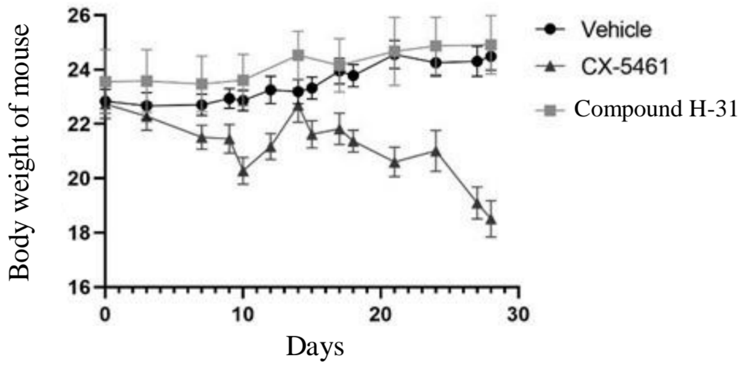


FIG. 1

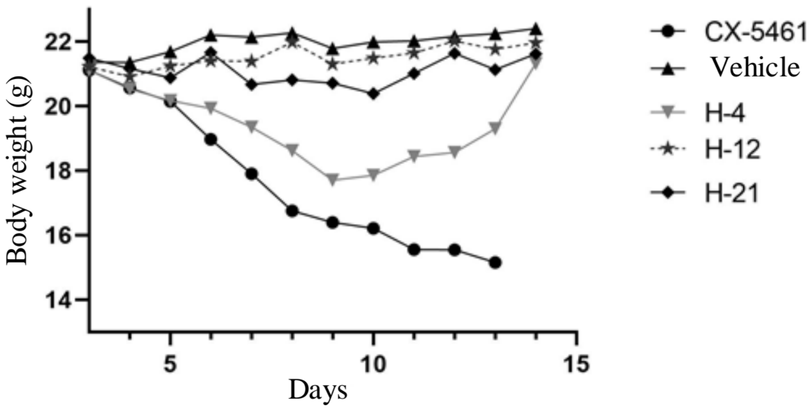


FIG. 2-1

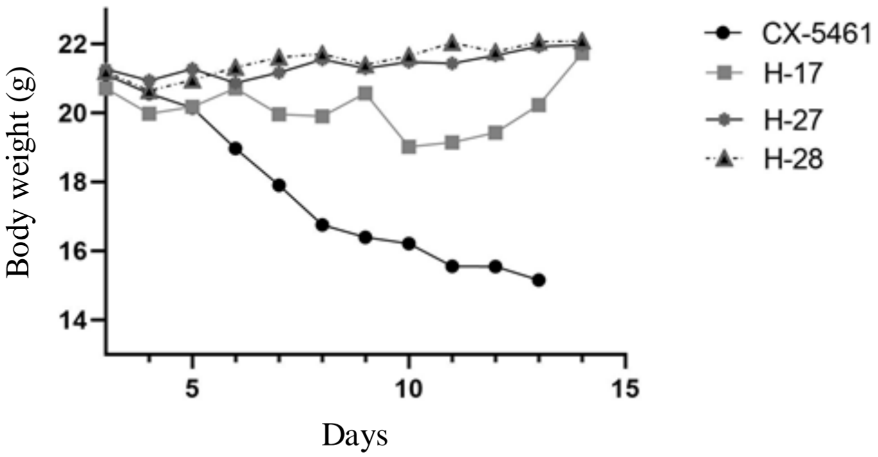


FIG. 2-2

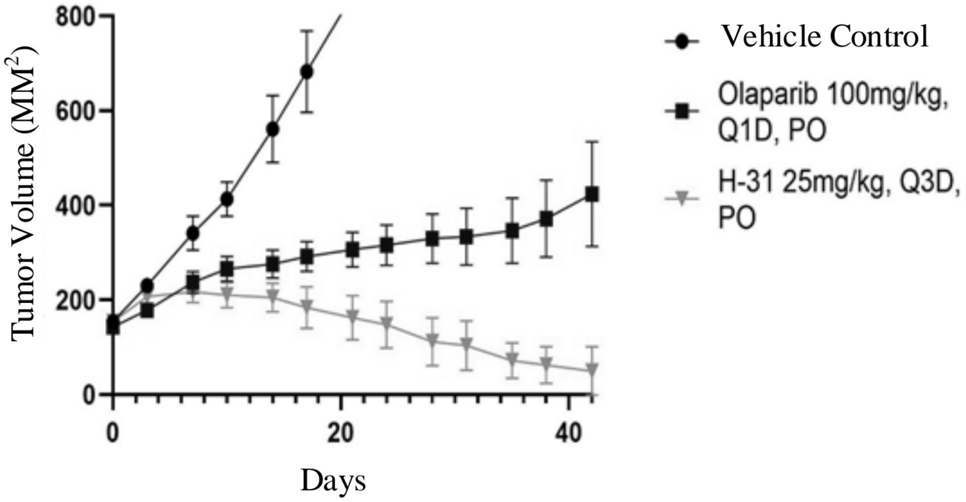
Tumor Volume (MM$^2$)
800
600
400
200
0
0
20
40
Days
Vehicle Control
Olaparib 100mg/kg, Q1D, PO
H-31 25mg/kg, Q3D, PO

FIG. 3

**INTERNATIONAL SEARCH REPORT**

International application No. **PCT/CN2024/076636**

**A. CLASSIFICATION OF SUBJECT MATTER**

C07D513/14(2006.01)i; A61K31/4353(2006.01)i; A61K31/55(2006.01)i; A61P35/00(2006.01)i; A61P37/06(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNTXT, WPABS, ENTXTC, CAPLUS(STN), REGISTRY(STN), CNKI: 信义核新, 徐红, 肿瘤, 癌症, 自身免疫, 细菌, 真菌, 病毒, 氮杂环庚烷, 喹诺酮, cancer, tumor, tumour, autoimmune, bacterial, fungal, viral, azepane, quinolone, 结构式检索, structural formula search.

**C. DOCUMENTS CONSIDERED TO BE RELEVANT**

| Category* | Citation of document, with indication, where appropriate, of the relevant passages | Relevant to claim No. |
|---|---|---|
| X | CN 101888780 A (CYLENE PHARMACEUTICALS INC.) 17 November 2010 (2010-11-17) claims 1-5 and 31-39, the fourth compound in claim 29, and description, paragraphs [0167] and [0189] | 1-15 |
| X | CN 103533934 A (TEL HASHOMER MEDICAL RESEARCH INFRASTRUCTURE AND SERVICES LTD.) 22 January 2014 (2014-01-22) claim 1, and the fourth compound in claim 9 | 1-15 |
| A | CN 104177379 A (SHANGHAI INSTITUTE OF MATERIA MEDICA, CHINESE ACADEMY OF SCIENCES) 03 December 2014 (2014-12-03) claims 1-14 | 1-15 |
| A | CN 108699083 A (SENHWA BIOSCIENCES INC.) 23 October 2018 (2018-10-23) claims 1-88 | 1-15 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **09 April 2024** | **12 April 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/076636**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **14-15**
because they relate to subject matter not required to be searched by this Authority, namely:

The subject matter of claim 14 is the use of the compound of formula (I) or a pharmaceutically acceptable salt, stereoisomer, racemate, deuterated compound or solvent compound thereof of any one of claims 1-7 or the pharmaceutical composition of claim 8 in the treatment of a disease which is ameliorated or to which resistance is generated due to the usage of a PARP inhibitor, and the subject matter of claim 15 is the combined use of the compound of formula (I) or a pharmaceutically acceptable salt, stereoisomer, racemate, deuterated compound or solvent compound thereof of any one of claims 1-7 or the pharmaceutical composition of claim 8 with a topoisomerase I and II inhibitor, an ATM inhibitor, an ATR inhibitor and a DNA-pK inhibitor. Said claims relate to a living human or animal body, and falls within disease treatment methods. The present report is provided on the basis of a pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/076636**

| Patent document cited in search report | Publication date (day/month/year) | Patent family member(s) | Publication date (day/month/year) |
|---|---|---|---|
| CN 101888780 A | 17 November 2010 | RU 2010117225 A | 10 November 2011 |
| | | RU 2549895 C2 | 10 May 2015 |
| | | AU 2008308485 A1 | 09 April 2009 |
| | | AU 2008308485 B2 | 07 August 2014 |
| | | PL 2214491 T3 | 30 November 2016 |
| | | PT 3092901 T | 21 May 2020 |
| | | IL 249906 A0 | 30 March 2017 |
| | | IL 249906 B | 29 October 2020 |
| | | CA 2701630 A1 | 09 April 2009 |
| | | CA 2701630 C | 25 October 2016 |
| | | US 2009093455 A1 | 09 April 2009 |
| | | US 7928100 B2 | 19 April 2011 |
| | | US 2011218184 A1 | 08 September 2011 |
| | | US 8853234 B2 | 07 October 2014 |
| | | NO 20100596 L | 10 June 2010 |
| | | BRPI 0817806 A2 | 07 October 2014 |
| | | PT 2214491 T | 25 October 2016 |
| | | PL 3092901 T3 | 19 October 2020 |
| | | DK 3092901 T3 | 18 May 2020 |
| | | WO 2009046383 A1 | 09 April 2009 |
| | | NZ 584892 A | 29 June 2012 |
| | | MX 2010003685 A | 02 June 2010 |
| | | KR 20160020578 A | 23 February 2016 |
| | | KR 101682867 B1 | 05 December 2016 |
| | | EP 3092901 A1 | 16 November 2016 |
| | | EP 3092901 B1 | 29 April 2020 |
| | | JP 2014159474 A | 04 September 2014 |
| | | JP 2010540663 A | 24 December 2010 |
| | | JP 5824213 B2 | 25 November 2015 |
| | | IL 204844 A0 | 30 November 2010 |
| | | IL 204844 A | 31 January 2017 |
| | | HK 1150728 A1 | 13 January 2012 |
| | | ES 2791305 T3 | 03 November 2020 |
| | | KR 20100064392 A | 14 June 2010 |
| | | KR 101601332 B1 | 08 March 2016 |
| | | ES 2582662 T3 | 14 September 2016 |
| | | EP 2214491 A1 | 11 August 2010 |
| | | EP 2214491 A4 | 10 November 2010 |
| | | EP 2214491 B1 | 13 July 2016 |
| CN 103533934 A | 22 January 2014 | US 2014086839 A1 | 27 March 2014 |
| | | EP 2685976 A1 | 22 January 2014 |
| | | EP 2685976 B1 | 27 December 2017 |
| | | WO 2012123938 A1 | 20 September 2012 |
| CN 104177379 A | 03 December 2014 | None | |
| CN 108699083 A | 23 October 2018 | CA 3006502 A1 | 22 June 2017 |
| | | AU 2015417382 A1 | 14 June 2018 |
| | | EP 4249072 A2 | 27 September 2023 |
| | | EP 4249072 A3 | 25 October 2023 |
| | | JP 2018537486 A | 20 December 2018 |
| | | KR 20180111797 A | 11 October 2018 |

Form PCT/ISA/210 (patent family annex) (July 2022)

**INTERNATIONAL SEARCH REPORT**
**Information on patent family members**

International application No.
**PCT/CN2024/076636**

| Patent document cited in search report | Publication date (day/month/year) | Patent family member(s) | | | Publication date (day/month/year) |
|---|---|---|---|---|---|
| | | AU | 2021203085 | A1 | 10 June 2021 |
| | | AU | 2021203085 | B2 | 08 June 2023 |
| | | EP | 3394070 | A1 | 31 October 2018 |
| | | EP | 3394070 | A4 | 08 May 2019 |
| | | EP | 3394070 | C0 | 02 August 2023 |
| | | ES | 2959536 | T3 | 26 February 2024 |
| | | RU | 2018124599 | A3 | 16 January 2020 |
| | | IL | 260004 | A | 31 July 2018 |
| | | WO | 2017105382 | A1 | 22 June 2017 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310438329